(19) Europäisches Patentamt
European Patent Office
Office européen des brevets

(11) EP 4 083 044 A1

(12) EUROPEAN PATENT APPLICATION
published in accordance with Art. 153(4) EPC

(43) Date of publication:
02.11.2022 Bulletin 2022/44

(21) Application number: 20908293.2

(22) Date of filing: 25.12.2020

(51) International Patent Classification (IPC):
*C07D 487/04* (2006.01) *A61K 31/53* (2006.01)
*A61P 35/00* (2006.01) *A61P 25/16* (2006.01)

(52) Cooperative Patent Classification (CPC):
A61K 31/53; A61P 25/16; A61P 35/00;
C07D 487/04

(86) International application number:
PCT/CN2020/139690

(87) International publication number:
WO 2021/129843 (01.07.2021 Gazette 2021/26)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 26.12.2019 CN 201911364028

(71) Applicant: Zhejiang Vimgreen Pharmaceuticals, Ltd
Hangzhou, Zhejiang 311122 (CN)

(72) Inventors:
- SUN, Sanxing
  Hangzhou, Zhejiang 311122 (CN)
- CHEN, Zhengshu
  Hangzhou, Zhejiang 311122 (CN)
- YE, Jinqi
  Hangzhou, Zhejiang 311122 (CN)
- ZHAO, Long
  Hangzhou, Zhejiang 311122 (CN)
- HU, Chongbo
  Hangzhou, Zhejiang 311122 (CN)
- YANG, Yong
  Hangzhou, Zhejiang 311122 (CN)
- GUAN, Feng
  Hangzhou, Zhejiang 311122 (CN)
- PAN, Shuhua
  Hangzhou, Zhejiang 311122 (CN)
- HU, Ning
  Hangzhou, Zhejiang 311122 (CN)
- PAN, Tingting
  Hangzhou, Zhejiang 311122 (CN)
- SONG, Guowei
  Hangzhou, Zhejiang 311122 (CN)
- HOU, Fangjie
  Hangzhou, Zhejiang 311122 (CN)

(74) Representative: Böhm, Brigitte
Weickmann & Weickmann
Patent- und Rechtsanwälte PartmbB
Postfach 860 820
81635 München (DE)

# (54) USE OF TRIAZOLOTRIAZINE DERIVATIVE IN TREATMENT OF DISEASES

(57) The present application relates to use of a triazolotriazine derivative or a pharmaceutically acceptable solvate or salt thereof in the preparation of a drug for treating diseases, and a method for treating diseases.



Processed by Luminess, 75001 PARIS (FR)

## Description

## TECHNICAL FIELD

The present application relates to the field of biological medicine, and in particular, relates to a triazolotriazine derivative capable of treating diseases.

## BACKGROUND ART

Parkinson's disease occurs as a result of chronic and progressive degeneration of neurons, the reasons for which are not yet fully understood. Its main clinical symptoms are manifested in the forms of static tremor, stiffness, bradykinesia and postural instability. Current therapeutic methods are mainly based on a dopamine replacement therapy, which would interfere with dopaminergic and/or cholinergic signaling cascade. However, in most cases, these clinical symptoms cannot be satisfactorily controlled with existing therapies, and are manifested as non-dopaminergic diseases in the brain, spinal nerves and peripheral autonomic nervous system. Under existing therapies, many patients with Parkinson's disease suffer from disabilities. Therefore, the top priority in the study of Parkinson's disease lies in the development of a new therapy that can slow down, stop or reverse the progression of the disease.

At present, cancer remains a major health burden across the world. Despite advances in the treatment of cancers, there is still an unsatisfied medical need for more effective treatment with lower toxicity, in particular for those patients who suffer from advanced diseases or cancers and show resistance to existing treatment. In recent years, people have deeply recognized the importance of lymphocytes in tumor immunity. The immune response of body to tumor comprises immune monitoring, which, as a cell-mediated mechanism related to immunity, can effectively eliminate newly generated tumor cells once tumor-associated antigens are recognized. Antibody-mediated anti-cancer actions also normally occur, but they are generally much smaller than cellular immune-mediated anti-cancer actions. Therefore, there is an urgent need of developing a promising new anti-cancer agent to meet medical needs.

## SUMMARY OF THE INVENTION

The present application provides use of a triazolotriazine derivative or a pharmaceutically acceptable solvate or salt thereof in the preparation of a drug for treating diseases, and a method for treating diseases, wherein the diseases comprise tumors and Parkinson's disease. A compound of the present application has at least one of the following properties in the treatment of diseases: (1) good pharmacokinetic properties, higher exposure in vivo and higher bioavailability; (2) improving the exercise ability of subjects with Parkinson's disease; and/or (4) inhibiting tumor growth and reducing tumor volume.

In one aspect, the present application provides use of a compound of formula I in the preparation of a drug for treating diseases,

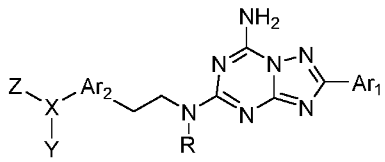

, formula I

wherein:

R is hydrogen or methyl; $Ar_1$ is furanyl optionally with a substituent, phenyl optionally with a substituent, or pyridyl optionally with a substituent; any aromatic ring optionally with a substituent is substituted by halogen or oxo; $Ar_2$ is phenyl optionally with a substituent, pyridyl optionally with a substituent, or pyrimidinyl optionally with a substituent; any aromatic ring optionally with a substituent is substituted by halogen, hydroxyl, cyano, or methoxy; X is oxygen or nitrogen;

and Y and Z are each independently hydrogen, $C_{1-3}$ alkyl optionally with a substituent, $C_{1-5}$ cycloalkyl optionally with a substituent, heterocycloalkyl optionally with a substituent, heterocycloalkylalkyl optionally with a substituent, aryl optionally with a substituent, $C_{1-3}$ alkylcarbonyl optionally with a substituent, $C_{1-5}$ cycloalkylcarbonyl optionally with a substituent, heterocycloalkylcarbonyl optionally with a substituent, heterocycloalkylalkylcarbonyl optionally with a substituent, arylcarbonyl optionally with a substituent, or heteroarylcarbonyl optionally with a substituent; any said group optionally with a substituent is substituted by halogen, hydroxy, methoxy, ethoxy, trifluoromethoxy, trifluoroethoxy, alkylamino, cycloalkylamino, heterocyclyl, aryl, heteroaryl, or $C_{1-3}$ alkyl polyoxyethylene; or, Y and Z

are linked to form heterocycloalkyl optionally with a substituent and having 5 to 7 ring atoms; any said ring optionally with a substituent is substituted by halogen, oxo, methoxy, ethoxy, trifluoromethoxy, or trifluoroethoxy; or Y or Z is not present, or a pharmaceutically acceptable solvate or salt thereof.

In some embodiments, R is hydrogen; $Ar_1$ is 2-furanyl; $Ar_2$ is phenyl optionally with a substituent; any said phenyl optionally with a substituent is substituted by halogen; X is oxygen or nitrogen; Y and Z are each independently hydrogen, $C_{2-3}$ alkyl optionally with a substituent, heterocycloalkyl optionally with a substituent, or heterocycloalkylalkyl optionally with a substituent; any said group optionally substituted is substituted by methoxy, ethoxy, trifluoromethoxy, trifluoroethoxy, or $C_{1-2}$ alkyl polyoxyethylene; or Y and Z are linked to form a morpholinyl ring; or Y or Z is not present, or a pharmaceutically acceptable solvate or salt thereof.

In some embodiments, in said compound of formula I, R is hydrogen; $Ar_1$ is 2-furanyl; $Ar_2$ is phenyl; X is nitrogen; and Y and Z are each independently hydrogen, ethyl optionally with a substituent, or oxetanyl optionally with a substituent; any said group optionally with a substituent is substituted by methoxy, ethoxy, trifluoromethoxy or trifluoroethoxy; or, Y and Z are linked to form a morpholinyl ring, or a pharmaceutically acceptable solvate or salt thereof.

In some embodiments, said compound is selected from the group consisting of:

N-(4-(2-((7-amino-2-(furan-2-yl)-[1,2,4]triazolo[1,5-a][1,3,5]triazin-5-yl)amino)ethyl)phenyl)-3-morpholine propanamide;
N-(4-(2-((7-amino-2-(furan-2-yl)-[1,2,4]triazolo[1,5-a][1,3,5]triazin-5-yl)amino)ethyl)phenyl)-2-morpholine propanamide;
N-(4-(2-((7-amino-2-(furan-2-yl)-[1,2,4]triazolo[1,5-a][1,3,5]triazin-5-yl)amino)ethyl)phenyl)pivalamide;
N-(4-(2-((7-amino-2-(furan-2-yl)-[1,2,4]triazolo[1,5-a][1,3,5]triazin-5-yl)amino)ethyl)phenyl)-3-hydroxy-2,2-dimethylpropionamide;
N-(4-(2-((7-amino-2-(furan-2-yl)-[1,2,4]triazolo[1,5-a][1,3,5]triazin-5-yl)amino)ethyl)phenyl)-2-hydroxy-2-methylpropionamide;
N-(4-(2-((7-amino-2-(furan-2-yl)-[1,2,4]triazolo[1,5-a][1,3,5]triazin-5-yl)amino)ethyl)phenyl)-4-methyltetrahydro-2H-pyran-4-carboxamide;
2-(furan-2-yl)-N5-(2-(pyrimidin-4-yl)ethyl)-[1,2,4]triazolo[1,5-a][1,3,5]triazine-5,7-diamine;
2-(furan-2-yl)-N5-(2-(pyridin-3-yl)ethyl)-[1,2,4]triazolo[1,5-a][1,3,5]triazine-5,7-diamine;
2-(furan-2-yl)-N5-(4-(2-(pyrrolidin-1-yl)ethoxy)phenethyl)-[1,2,4]triazolo[1,5-a][1,3,5]-triazine-5,7-diamine;
N5-(4-(2-(azetidine-1-yl)ethoxy)phenethyl)-2-(furan-2-yl)-[1,2,4]triazolo[1,5-a][1,3,5]triazine-5,7-diamine;
2-(furan-2-yl)-N5-(4-(2-(4-methylpiperazin-1-yl)ethoxy)phenethyl)-[1,2,4]triazolo[1,5-a][1,3,5]triazine-5,7-diamine;
2-(furan-2-yl)-N5-(4-(3-(4-methylpiperazin-1-yl)propoxy)phenethyl)-[1,2,4]triazolo[1,5-a][1,3,5]triazine-5,7-diamine;
N5-(4-(2-(3,3-difluoropyrrolidin-1-yl)ethoxy)phenethyl)-2-(furan-2-yl)-[1,2,4]triazolo[1,5-a][1,3,5]triazine-5,7-diamine;
(R)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine;
(S)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine;
2-(furan-2-yl)-N5-(4-((2-morpholinoethyl)amino)phenethyl)-[1,2,4]triazolo[1,5-a][1,3,5]triazine-5,7-diamine;
2-(furan-2-yl)-N5-(4-((tetrahydro-2H-pyran-4-yl)amino)phenethyl)-[1,2,4]triazolo[1,5-a][1,3,5]triazine-5,7-diamine;
2-(furan-2-yl)-N5-(4-((tetrahydrofuran-3-yl)amino)phenethyl)-[1,2,4]triazolo[1,5-a][1,3,5]triazine-5,7-diamine;
2-(furan-2-yl)-N5-(4-(4-methylpiperazin-1-yl)phenethyl)-[1,2,4]triazolo[1,5-a][1,3,5]triazine-5,7-diamine;
N5-(4-(bis(2-methoxyethyl)amino)phenethyl)-2-(furan-2-yl)-[1,2,4]triazolo[1,5-a][1,3,5]triazine-5,7-diamine;
2-(furan-2-yl)-N5-(4-(oxetane-3-ylamino)phenethyl)-[1,2,4]triazolo[1,5-a][1,3,5]triazine-5,7-diamine;
2-(furan-2-yl)-N5-(4-(oxetan-3-ylmethyl)phenethyl)-[1,2,4]triazolo[1,5-a][1,3,5]triazine-5,7-diamine;
2-(furan-2-yl)-N5-(4-((1-methoxypropane-2-yl)amino)phenethyl)-[1,2,4]triazolo[1,5-a][1,3,5]triazine-5,7-diamine;
2-(furan-2-yl)-N5-(4-((2-methoxyethyl)(methyl)amino)phenethyl)-[1,2,4]triazolo[1,5-a][1,3,5]triazine-5,7-diamine;
N5-(4-(ethyl(2-methoxyethyl)amino)phenethyl)-2-(furan-2-yl)-[1,2,4]triazolo[1,5-a][1,3,5]triazine-5,7-diamine;
2-(furan-2-yl)-N5-(4-((2-methoxyethyl)amino)phenethyl)-[1,2,4]triazolo[1,5-a][1,3,5]triazine-5,7-diamine;
2-(furan-2-yl)-N5-(4-((2-(2-methoxyethoxy)ethyl)amino)phenethyl)-[1,2,4]triazolo[1,5-a][1,3,5]triazine-5,7-diamine;
2-(furan-2-yl)-N5-(4-((2-(2-(2-methoxyethoxy)ethoxy)ethyl)amino)phenethyl)-[1,2,4]triazolo[1,5-a][1,3,5]triazine-5,7-diamine;
N5-(4-((2-ethoxyethyl)amino)phenethyl)-2-(furan-2-yl)-[1,2,4]triazolo[1,5-a][1,3,5]triazine-5,7-diamine;
2-(furan-2-yl)-N5-(4-((2-(2,2,2-trifluoroethoxy)ethyl)amino)phenethyl)-[1,2,4]triazolo[1,5-a][1,3,5]triazine-5,7-diamine;
2-(furan-2-yl)-N5-(4-((3-methoxypropyl)amino)phenethyl)-[1,2,4]triazolo[1,5-a][1,3,5]triazine-5,7-diamine;

2-((4-(2-((7-amino-2-(furan-2-yl)-[1,2,4]triazolo[1,5-a][1,3,5]triazine-5-yl)amino)ethyl)-phenyl)amino)acetonitrile;
N5-(4-(1,3-dimethoxypropane-2-ylamino)phenethyl)-2-(furan-2-yl)-[1,2,4]triazolo[1,5-a][1,3,5]triazine-5,7-diamine;
2-(3-fluorophenyl)-N5-(4-((2-methoxyethyl)amino)phenethyl)-[1,2,4]triazolo[1,5-a][1,3,5]triazine-5,7-diamine; and
2-(3-fluorophenyl)-N5-(4-(oxetane-3-ylamino)phenethyl)-[1,2,4]triazolo[1,5-a][1,3,5]triazine-5,7-diamine;
or a pharmaceutically acceptable solvate or salt thereof.

In another aspect, the present application provides use of a compound of formula II in the preparation of a drug for treating diseases,

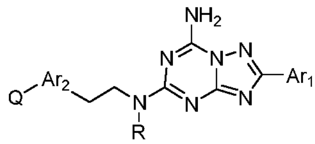
, formula II

wherein R is hydrogen or methyl; $Ar_1$ is furanyl optionally with a substituent, phenyl optionally with a substituent, or pyridyl optionally with a substituent; any aromatic ring optionally with a substituent is substituted by halogen or oxo; $Ar_2$ is phenyl optionally with a substituent, pyridyl optionally with a substituent, or pyrimidinyl optionally with a substituent; any aromatic ring optionally with a substituent is substituted by halogen, hydroxyl, cyano, or methoxy; and Q is a 5-6 membered aromatic ring optionally substituted by X, an aminocarbonyl group optionally substituted by Y and Z on nitrogen, an aminosulfonyl group optionally substituted by Y and Z on nitrogen, a nitro group, or a cyano group; X is halogen or optionally substituted $C_{1-3}$ alkyl; any said optionally substituted alkyl group is substituted by halogen, cyano, methoxy , ethoxy, trifluoromethoxy, trifluoroethoxy, aryl or heteroaryl; Y and Z are each independently hydrogen or optionally substituted $C_{1-3}$ alkyl; any said optionally substituted alkyl group is substituted by halogen, hydroxy, methyl, alkylamino, or cycloalkylamino; or, Y and Z are linked to form an optionally substituted ring having 5 to 7 ring atoms; any said optionally substituted ring is substituted by halogen, methyl, ethyl, trifluoromethyl or trifluoroethyl, or a pharmaceutically acceptable solvate or salt thereof.

In some embodiments, in said compound of formula II, R is hydrogen; $Ar_1$ is 2-furanyl; $Ar_2$ is phenyl or pyridyl; Q is nitro, cyano, or a 5-6 membered aromatic ring optionally substituted by X; X is optionally substituted $C_{1-3}$ alkyl; any said optionally substituted alkyl is halogen, cyano, methoxy, aryl, or heteroaryl, or a pharmaceutically acceptable solvate or salt thereof.

In some embodiments, in said compound of formula II, R is hydrogen; $Ar_1$ is 2-furanyl; $Ar_2$ is phenyl; Q is a tetrazole ring optionally substituted by X; X is an optionally substituted $C_{1-3}$ alkyl; any said optionally substituted alkyl is optionally substituted by halogen, cyano, or methoxy, or a pharmaceutically acceptable solvate or salt thereof.

In some embodiments, said compound is selected from the group consisting of:

4-(2-(7-amino-2-(furan-2-yl)-[1,2,4]triazolo[1,5-a][1,3,5]triazine-5-ylamino)ethyl)-N,N-dimethylbenzamide;
N5-(4-(1-methyl-1H-tetrazol-5-yl)phenethyl)-2-(furan-2-yl)-[1,2,4]triazolo[1,5-a][1,3,5]triazine-5,7-diamine;
N5-(4-(2-methyl-2H-tetrazol-5-yl)phenethyl)-2-(furan-2-yl)-[1,2,4]triazolo[1,5-a][1,3,5]triazine-5,7-diamine;
N5-(4-(2-ethyl-2H-tetrazol-5-yl)phenethyl)-2-(furan-2-yl)-[1,2,4]triazolo[1,5-a][1,3,5]triazine-5,7-diamine;
N5-(4-(2-isopropyl-2H-tetrazol-5-yl)phenethyl)-2-(furan-2-yl)-[1,2,4]triazolo[1,5-a][1,3,5]triazine-5,7-diamine;
N5-(4-(2-propyl-2H-tetrazol-5-yl)phenethyl)-2-(furan-2-yl)-[1,2,4]triazolo[1,5-a][1,3,5]triazine-5,7-diamine;
2-(5-(4-(2-(7-amino-2-(furan-2-yl)-[1,2,4]triazolo[1,5-a][1,3,5]triazine-5-ylamino)ethyl)-phenyl)-2H-tetrazol-2-yl)acetonitrile;
4-(2-(7-amino-2-(furan-2-yl)-[1,2,4]triazolo[1,5-a][1,3,5]triazine-5-ylamino)ethyl)-benzonitrile;
N5-(4-(2-(2,2,2-trifluoroethyl)-2H-tetrazol-5-yl)phenethyl)-2-(furan-2-yl)-[1,2,4]triazolo[1,5-a][1,3,5]triazine-5,7-diamine;
N5-(4-(2-(2-methoxyethyl)-2H-tetrazol-5-yl)phenethyl)-2-(furan-2-yl)-[1,2,4]triazolo[1,5-a][1,3,5]triazine-5,7-diamine;
2-(furan-2-yl)-N5-(4-(pyridin-2-yl)phenethyl)-[1,2,4]triazolo[1,5-a][1,3,5]triazine-5,7-diamine;
2-(furan-2-yl)-N5-(4-(pyridin-3-yl)phenethyl)-[1,2,4]triazolo[1,5-a][1,3,5]triazine-5,7-diamine;
2-(furan-2-yl)-N5-(4-(pyridin-4-yl)phenethyl)-[1,2,4]triazolo[1,5-a][1,3,5]triazine-5,7-diamine;
2-(furan-2-yl)-N5-(4-(pyrimidin-2-yl)phenethyl)-[1,2,4]triazolo[1,5-a][1,3,5]triazine-5,7-diamine;
2-(furan-2-yl)-N5-(4-(1-methyl-1H-1,2,4-triazol-3-yl)phenethyl)-[1,2,4]triazolo[1,5-a][1,3,5]triazine-5,7-diamine;
N5-(4-(2H-tetrazol-5-yl)phenethyl)-2-(furan-2-yl)-[1,2,4]triazolo[1,5-a][1,3,5]triazine-5,7-diamine;
2-(furan-2-yl)-N-(4-(methylsulfonyl)phenethyl)-[1,2,4]triazolo[1,5-a][1,3,5]triazine-5-amine;
2-(3-fluorophenyl)-N5-(4-(2-methyl-2H-tetrazol-5-yl)phenethyl)-[1,2,4]triazolo[1,5-a][1,3,5]triazine-5,7-diamine;

N5-(4-(2-methyl-2H-tetrazol-5-yl)phenethyl)-2-phenyl-[1,2,4]triazolo[1,5-a][1,3,5]triazine-5,7-diamine; and
2-(furan-2-yl)-N5-(2-(6-(2-methyl-2H-tetrazol-5-yl)pyridin-3-yl)ethyl)-[1,2,4]triazolo[1,5-a][1,3,5]triazine-5,7-diamine,
or a pharmaceutically acceptable solvate or salt thereof.

In some embodiments, said compound is selected from the group consisting of:

2-(furan-2-yl)-N5-(4-(oxetane-3-ylamino)phenethyl)-[1,2,4]triazolo[1,5-a][1,3,5]triazine-5,7-diamine;
2-(furan-2-yl)-N5-(4-((2-methoxyethyl)amino)phenethyl)-[1,2,4]triazolo[1,5-a][1,3,5]triazine-5,7-diamine;
N5-(4-(2-methyl-2H-tetrazol-5-yl)phenethyl)-2-(furan-2-yl)-[1,2,4]triazolo[1,5-a][1,3,5]triazine-5,7-diamine; and
N5-(4-(2-ethyl-2H-tetrazol-5-yl)phenethyl)-2-(furan-2-yl)-[1,2,4]triazolo[1,5-a][1,3,5]triazine-5,7-diamine;
and a pharmaceutically acceptable solvate or salt thereof.

In some embodiments, said pharmaceutically acceptable solvate or salt of said compound comprises acetate and/or benzenesulfonate.

In some embodiments, said pharmaceutically acceptable solvate or salt of said compound comprises 2-(furan-2-yl)-N5-(4-(oxetan-3-ylamino)phenethyl)-[1,2,4]triazolo[1,5-a][1,3,5]triazine-5,7-diamine acetate and/or *N*5-(4-(2-methyl-2*H*-tetrazol-5-yl)phenethyl)-2-(furan -2-yl)-[1,2,4]triazolo[1,5-*a*][1,3,5]triazine-5,7-diamine benzenesulfonate.

In some embodiments, said diseases comprise Parkinson's disease.

In some embodiments, said diseases comprise tumors. In some embodiments, said tumors comprise solid tumors and non-solid tumors. In some embodiments, said solid tumors comprise a colon cancer and melanoma. In some embodiments, said non-solid tumors comprise lymphoma, for example, B-cell lymphoma.

In some embodiments, said drug is prepared to adapt to oral administration and/or injection administration.

In some embodiments, said drug further comprises a pharmaceutically acceptable carrier. For example, said pharmaceutically acceptable carrier comprises cyclodextrin.

In another aspect, the present application provides a method for treating diseases, comprising the step of administering said compound of the present application at a therapeutically effective dose to a subject in need thereof.

In some embodiments, said diseases comprise Parkinson's disease.

In some embodiments, said subject exhibits early signs of the Parkinson's disease.

In some embodiments, said subject has previously received or not received a drug for treating the Parkinson's disease.

In some embodiments, said subject receives a drug for treating the Parkinson's disease before, concurrently with or after administration of said compound.

In some embodiments, said subject develops resistance to a drug for treating the Parkinson's disease. In some embodiments, said drug for treating the Parkinson's disease comprises dopamine.

In some embodiments, said treating comprises reducing the rate of progression of the Parkinson's disease in said subject, and/or delaying the clinical progression of the Parkinson's disease in said subject. In some embodiments, said rate of progression of the Parkinson's disease in said subject is assessed by UPDRS.

In some embodiments, said treating comprises delaying the need of said subject for symptomatic treatment against the Parkinson's disease.

In some embodiments, said treating comprises reducing a risk in the need of the subject for the symptomatic treatment against the Parkinsonian disease.

In some embodiments, said diseases comprise tumors. In some embodiments, said tumors comprise solid tumors and non-solid tumors. In some embodiments, said solid tumors comprise a colon cancer and melanoma. In some embodiments, said non-solid tumors comprise lymphoma, for example, B-cell lymphoma.

In some embodiments, a method for administrating said compound comprises oral administration and/or injection administration.

In some embodiments, said compound is administered at a frequency of once a day or twice a day.

In some embodiments, said compound is administered at a dose of from about 0.001 mg/kg to about 500 mg/kg.

In some embodiments, said compound is administered at a dose of from about 1 mg/kg to about 100 mg/kg.

In some embodiments, said subject receives an immunotherapy for treating tumors before, concurrently with, or after administration of said compound.

In some embodiments, said immunotherapy for treating the tumors comprises an immune checkpoint inhibitor.

In some embodiments, said immunotherapy for treating the tumors comprises a PD-1 antibody.

In another aspect, the present application provides a pharmaceutical combination or kit, comprising (1) said compound, and (2) a drug for treating Parkinson's disease.

In some embodiments, said drug for treating the Parkinson's disease comprises dopamine.

In another aspect, the present application provides a pharmaceutical combination or kit, comprising (1) said

compound, and (2) an immunotherapy for treating tumors.

In some embodiments, said immunotherapy for treating the tumors comprises an immune checkpoint inhibitor.

In some embodiments, said immunotherapy for treating the tumors comprises a PD-1 antibody.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

## BRIEF DESCRIPTION OF THE DRAWINGS

The specific features of the present invention involved in the present application are listed in the appended claims. The characteristics and advantages of the present invention involved in the present application can be better understood by referring to the exemplary embodiments and the accompanying drawings described in detail below. A brief description of the drawings is as follows:

FIG. 1 shows the XRPD result of the salt of a compound according to the present application;
FIG. 2 shows the $^{1}$H-NMR spectrum result of the salt of a compound according to the present application; and
FIG. 3 shows the DSC and TGA results of the salt of a compound according to the present application.

## DETAILED DESCRIPTION OF THE INVENTION

The embodiments of the invention of the present application will be illustrated by specific examples below. Those familiar with this technology can easily understand other advantages and effects of the invention of the present application from the content disclosed in the specification.

## DEFINITIONS

In the present application, the term "alkyl" generally refers to straight-chain and branched-chain saturated aliphatic hydrocarbon groups containing a certain number of carbon atoms. For example, C4 alkyl comprises n-butyl, isobutyl, sec-butyl and tert-butyl. The term "cycloalkyl" generally refers to monocyclic and bicyclic saturated aliphatic hydrocarbon groups containing a certain number of carbon atoms.

In the present application, the term "alkenyl" generally refers to straight-chain and branched-chain aliphatic hydrocarbon groups containing at least one carbon-carbon double bond. For example, one carbon-carbon double bond exists.

Unless otherwise specified, the term "aryl" generally refers to monocyclic and bicyclic aromatic rings containing 5 to 14 ring atoms, and in some cases, 6 to 10 ring atoms. The aryl may be optionally substituted by one or more substituents. Unless otherwise specified, the term "aryl" may also comprise monocyclic and bicyclic heteroaryl rings containing 5 to 14 ring atoms, and in some cases, 6 to 10 ring atoms.

In the present application, the term "heterocycloalkyl" generally refers to saturated monocyclic and bicyclic ring systems containing 3 to 14 ring atoms, for example, 4 to 10 ring atoms, wherein one or more of the atoms are non-carbon elements, such as nitrogen, oxygen or sulfur. These heterogeneous elements may exist alone or together with other heterogeneous elements. Examples of heterocycloalkyl comprise, for example, azetidinyl, hexahydroazapyridyl, piperazinyl, piperidinyl, pyrrolidinyl, morpholinyl, tetrahydrofuranyl, thiomorpholinyl and tetrahydrothienyl, and N-oxides thereof.

In the present application, the term "halogen" generally means to comprise fluorine, chlorine, bromine and iodine. The term "trifluoromethyl" generally refers to a "-CF3" group, and the term "hydroxyl" or "hydroxy" generally refers to an "-OH" group.

In the present application, the term "symptomatic treatment against Parkinson's disease" generally comprises any therapy for treating the Parkinson's disease, comprising but not limited to bromocriptine, benztropine, levodopa, ropinirole, pramipexole, rotigotine, cabergoline, entacapone, tolcapone, amantadine, pergolide, apomorphine, lisuride, or selegiline. The term "delaying the need for symptomatic treatment against the Parkinson's disease" refers to delaying, in contrast to patients who have not received the compound of the present application, the need of a subject with the Parkinson's disease, who have received the compound of the present application, for the symptomatic treatment against the Parkinson's disease.

In the present application, the term "reducing the rate of progression of the Parkinson's disease" generally refers to reducing the deterioration experienced by a patient with Parkinson's disease. For example, this deterioration

can be quantified by means of the UPDRS score in contrast to the deterioration experienced by a patient with Parkinson's disease who has not received the compound of the present application for a period of time. The rate of progression is quantified by the total Unified Parkinson's Disease Rating Scale (total UPDRS) scores. An increase in the total UPDRS score represents the progression of the symptoms of Parkinson's disease, and an increment in the increase of the UPDRS score over a period of time represents the development of the symptoms of Parkinson's disease.

In the present application, the term "functional decline" generally refers to the deterioration of the symptoms in a patient with Parkinson's disease over time, as determined by means of the total UPDRS score.

In the present application, the term "delaying the need for symptomatic treatment against the Parkinson's disease" refers to delaying, in contrast to patients who have not received the compound of the present application, the need of a subject with the Parkinson's disease, who have received the compound of the present application, for the symptomatic treatment against the Parkinson's disease.

In the present application, the term "early signs of Parkinson's disease" generally comprises one or more of the following signs:

a) 4-8 Hz pill-rubbing like tremor of a single hand at rest;
b) Maximum tremor at rest, which decreases during movement and disappears during sleep;
c) Stiff and slow movement (bradycardia), movement decline (hypokinesia), and difficulty in starting to move (loss of mobility);
d) Mask-like face with tardiness in opening mouth and blinking, which may be confused with depression;
e) Curved posture;
f) Difficulty in starting to walk, unsteady gait, and small step, with arms bent to the waist without swinging with the steps;
g) Occasionally unintentional acceleration of paces, where a patient would sometimes start trotting suddenly to keep from falling (panic pace);
h) Loss of posture reflex, resulting in leaning forward (advancing) or backward (reverse advancing) when the center of gravity is moved;
i) Weakened pronunciation with monotonous feature, stuttering, and difficulty in vocalization;
j) Decreased motor function and impaired control over terminal muscle tissues, resulting in micrographia and increased difficulty in daily activities;
k) Rare blinking and lack of facial expressions;
l) Reduced movement;
m) Postural reflex disorder; and/or
n) Typical abnormal gait.

In the present application, the stage of a patient of Parkinson's disease refers to the following 5 significant symptom-depending stages described by Hoehn and Yahr (Hoehn MM, Yahr MD, Parkinsonism: onset, progression and mortality. Neurology, 1967, 17: 427-42).

Stage I: (Mild or early disease): symptoms affect one side of the body.

Stage II: both sides of the body are affected, but the posture remains normal.

Stage III: (moderate disease): both sides of the body are affected, showing a slight imbalance during standing and walking; but the patient can remain independent.

Stage IV (advanced disease): both sides of the body are affected, showing impaired instability during standing and walking; and the patient at this stage is in need of a lot of help.

Stage V: a fully-developed serious disease is manifested; and the patient is confined to a bed or chair. In the present application, the "patient at early stage of Parkinson's disease" refers to a patient at stage I or II of Parkinson's disease as defined by Hoehn and Yahr, and he/she does not need symptomatic treatment against Parkinson's disease. For example, such patients do not need the symptomatic treatment against Parkinson's disease at least in the next 9 months. Related tests can be carried out to verify those patients at the early stage of Parkinson's disease.

In the present application, the term "delaying the clinical progression of Parkinson's disease" generally refers to that, in contrast to a subject who has not received the compound of the present application, a lower rate of increase in the total UPDRS score is acquired in a subject, who has received the compound, in a period after the compound has completely exerted a targeted effect, for example 12 weeks after the compound is received. Or, in contrast to a subject who has received treatment with the compound with a delay, the total UPDRS score shows lower deterioration after a sufficient period of time to eliminate changes caused by the delay in starting the treatment with the compound. The sufficient period of time is necessarily more than 52 weeks (e.g., at least more than 72 weeks) after initial treatment with the compound. Or, in contrast to the subject who has received the treatment with the compound with a delay, the total UPDRS score shows a substantially similar deterioration rate (for example, the total UPDRS score per week is within 0.15 units) during a period after a targeted effect exerted by the delayed start of the treatment with the compound.

In the present application, the terms "cancer" and "tumor" are used interchangeably, and generally refer to cells

that have caused malignant transformation or cellular changes with abnormal or unregulated growth or hyperproliferation. Such changes or malignant transformation generally leads to pathogenicity of such cells to a host's organism. Therefore, such cells are also expected to comprise early-stage carcinomatous lesions or precancerous cells that will or may become pathogenic and require or may benefit from intervention. The tumors may comprise solid tumors and non-solid tumors.

## DETAILED DESCRIPTION OF THE INVENTION

In one aspect, the present application provides use of a compound represented by formula I, as well as a hydrate, solvate, pharmaceutically acceptable salt, prodrug thereof and their complexes, in a drug for treating diseases:

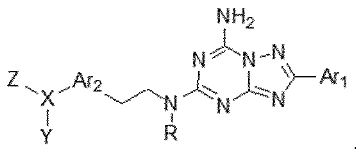

, formula I,

wherein R may be hydrogen or $C_{1-5}$ alkyl optionally with a substituent; any alkyl optionally with a substituent is substituted with halogen, cyano, hydroxy, nitro, amino, alkylamino, cycloalkylamino, methyl, ethyl, methoxy, ethoxy, trifluoromethyl, trifluoroethyl, trifluoromethoxy or trifluoroethoxy; $Ar_1$ may be a 5-6 membered aromatic ring, which is optionally substituted by halogen, oxo, cyano, methyl, methoxy, trifluoromethyl or trifluoromethoxy; $Ar_2$ may be a monocyclic or bicyclic aromatic ring, which is optionally substituted by halogen, hydroxy, cyano, methyl, methoxy, trifluoromethyl or trifluoromethoxy; X is selected from the group consisting of: oxygen, sulfur, carbon, and nitrogen; and Y and Z may each independently be hydrogen, $C_{1-9}$ alkyl optionally with a substituent, monocyclic or bicyclic $C_{1-9}$ cycloalkyl optionally with a substituent, $C_{1-9}$ alkenyl optionally with a substituent, monocyclic or bicyclic $C_{1-9}$ cycloalkenyl optionally with a substituent, aryl optionally with a substituent, heteroaryl optionally with a substituent, aralkyl optionally with a substituent, heteroaralkyl optionally with a substituent, heterocycloalkyl optionally with a substituent, heterocycloalkenyl optionally with a substituent, heterocycloalkylalkyl optionally with a substituent, $C_{1-9}$ alkylcarbonyl optionally with a substituent, $C_{1-9}$ cycloalkylcarbonyl optionally with a substituent, heterocycloalkylcarbonyl optionally with a substituent, heterocycloalkylalkylcarbonyl optionally with a substituent, arylcarbonyl optionally with a substituent, or heteroarylcarbonyl optionally with a substituent; the group optionally with a substituent may be substituted by a substituent selected from the group consisting of: halogen, cyano, hydroxy, nitro, amino, alkylamino, cycloalkylamino, heterocyclyl, aminocarbonyl, sulfonyl, aminosulfonyl, carbonylamino, sulfonylamino, methyl, ethyl, aryl, methoxy, ethoxy, trifluoromethyl, trifluoroethyl, trifluoromethoxy, trifluoroethoxy, polyoxyethylene, polyoxypropylene, $C_{1-3}$ alkyl polyoxyethylene, or $C_{1-3}$ alkyl polyoxypropylene ; or, Y and Z may be linked to form a ring optionally with a substituent and having 3 to 10 ring atoms; a substituent in any ring optionally with a substituent may be substituted by a substituent selected from the group consisting of: halogen, cyano, hydroxy, oxo, nitro, amino, alkylamino, cycloalkylamino, aminocarbonyl, sulfonyl, aminosulfonyl, carbonylamino, sulfonylamino, methyl, ethyl, methoxy, ethoxy, trifluoromethyl, trifluoroethyl, trifluoromethoxy, trifluoroethoxy, polyoxyethylene, polyoxypropylene, $C_{1-3}$ alkyl polyoxyethylene, or $C_{1-3}$ alkyl polyoxypropylene; or, Y or Z may not be present in the structure.

In some cases, in the compound represented by formula I, R may be hydrogen, methyl or trifluoromethyl. In some specific cases, in the compound represented by formula I, R may be hydrogen or methyl. For example, in the compound represented by formula I, R may be hydrogen.

In some cases, in the compound represented by formula I, $Ar_1$ may be imidazolyl, triazolyl, tetrazolyl, furanyl, thienyl, oxazolyl, isoxazolyl, thiazolyl, isothiazolyl, oxadiazolyl, thiadiazolyl, phenyl, pyridyl, pyridazinyl, pyrimidinyl, pyrazinyl or triazinyl, optionally with a substituent; and the aromatic ring optionally with a substituent is substituted by halogen, oxo, cyano, methyl, methoxy, trifluoromethyl or trifluoromethoxy.

In some cases, in the compound represented by formula I, $Ar_1$ may be selected from aromatic groups in Table (1).

Table 1 Structure of $Ar_1$ in formula I

| | | | | |
|---|---|---|---|---|
| 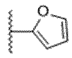 | 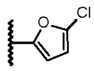 | 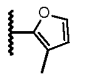 | 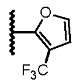 | 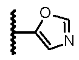 |
| 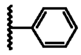 | 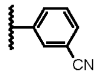 | 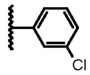 | 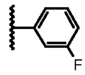 | 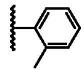 |
| 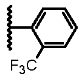 | 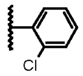 | 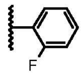 | 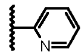 | 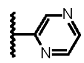 |

In some cases, in the compound represented by formula I, $Ar_1$ may be 2-furanyl.

In some cases, in the compound represented by formula I, $Ar_2$ may be imidazolyl, triazolyl, tetrazolyl, furanyl, thienyl, oxazolyl, isoxazolyl, thiazolyl, isothiazolyl, oxadiazolyl, thiadiazolyl, phenyl, pyridyl, pyridazinyl, pyrimidinyl, pyrazinyl, triazinyl, indolyl, isoindolyl, indazolyl, benzimidazolyl, azaindolyl, azaindazolyl, purinyl, benzofuranyl, isobenzofuranyl, benzothienyl, benzisoxazolyl, benzisothiazole, benzoxazolyl, benzothiadiazole, quinolinyl, isoquinolinyl, quinoxalinyl, phthalazinyl, quinazolinyl, cinnolinyl, naphthyridinyl, pyridopyrimidinyl, pyridopyrazine, or pteridyl, optionally with a substituent; and the aromatic ring optionally with a substituent may be substituted by halogen, hydroxy, cyano, nitro, methyl, methoxy, trifluoromethyl or trifluoromethoxy.

In some cases, in the compound represented by formula I, $Ar_2$ may be phenyl, pyridyl, pyridazinyl or pyrimidinyl, optionally with a substituent; and the aromatic ring optionally with a substituent may be substituted by halogen, hydroxy, cyano, methyl, methoxy, trifluoromethyl or trifluoromethoxy.

In some cases, in the compound represented by formula I, $Ar_2$ may be phenyl or pyridyl, and the phenyl or pyridyl may be optionally substituted by halogen or hydroxy.

In some cases, in the compound represented by formula I, X is oxygen or nitrogen. For example, in the compound represented by formula I, X is nitrogen.

In some cases, in the compound represented by formula I, Y and Z may each independently be hydrogen, $C_{2-5}$ alkyl optionally with a substituent, $C_{3-5}$ cycloalkyl optionally with a substituent, $C_{2-5}$ alkenyl optionally with a substituent, $C_{3-5}$ cycloalkenyl optionally with a substituent, aryl optionally with a substituent, heteroaryl optionally with a substituent, aralkyl optionally with a substituent, heteroaralkyl optionally with a substituent, heterocycloalkyl optionally with a substituent, or heterocycloalkylalkyl optionally with a substituent; and any above group optionally with a substituent may be substituted by halogen, cyano, methoxy, ethoxy, trifluoromethoxy, trifluoroethoxy or $C_{1-3}$ alkyl polyoxyethylene.

In some cases, in the compound represented by formula I, Y and Z may each independently be hydrogen, $C_{2-3}$ alkyl optionally with a substituent, or heterocycloalkyl optionally with a substituent; and any above group optionally with a substituent may be substituted by methoxy, ethoxy, trifluoromethoxy or trifluoroethoxy.

In some cases, in the compound represented by formula I, Y and Z may each independently be hydrogen, ethyl optionally with a substituent, or oxetanyl optionally with a substituent; and any above group optionally with a substituent may be substituted by methoxy, ethoxy, trifluoromethoxy or trifluoroethoxy.

In some cases, in the compound represented by formula I, Y and Z may be linked to form a ring optionally with a substituent and having 4 to 8 ring atoms; and the substituent in any above group optionally with a substituent may be halogen, oxo, methoxy, ethoxy, trifluoromethoxy or trifluoroethoxy.

In some cases, in the compound represented by formula I, Y and Z may be linked to form heterocycloalkane optionally with a substituent and having 5 to 7 ring atoms; and any above group optionally with a substituent may be substituted by halogen, oxo, methoxy, ethoxy, trifluoromethoxy or trifluoroethoxy.

In some cases, in the compound represented by formula I, Y and Z may be linked to form a morpholinyl ring optionally with a substituent; and any above group optionally with a substituent may be substituted by halogen, methoxy, ethoxy, trifluoromethoxy or trifluoroethoxy.

In another aspect, the present application provides use of a triazolotriazine compound represented by formula II, as well as a hydrate, solvate, pharmaceutically acceptable salt, prodrug thereof and their complexes, in a drug for treating diseases:

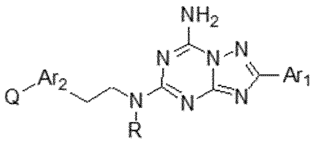, formula II,

wherein R may be hydrogen or C1-5 alkyl optionally with a substituent; any alkyl optionally with a substituent may be substituted by halogen, cyano, hydroxy, nitro, amino, alkylamino, cycloalkylamino, methyl, ethyl, methoxy, ethoxy, trifluoromethyl, trifluoroethyl, trifluoromethoxy or trifluoroethoxy; $Ar_1$ may be a 5-6 membered aromatic ring, which may be optionally substituted by halogen, oxo, cyano, methyl, methoxy, trifluoromethyl or trifluoromethoxy; $Ar_2$ may be a monocyclic or bicyclic aromatic ring, which may be optionally substituted by halogen, hydroxy, cyano, methyl, methoxy, trifluoromethyl or trifluoromethoxy;

Q may be a monocyclic or bicyclic aromatic ring optionally substituted by X, aminocarbonyl optionally substituted by Y and Z on the nitrogen atom, an aminosulfonyl optionally substituted by Y and Z on the nitrogen atom, nitro, or cyano. X may be halogen, cyano, hydroxy, nitro, amino, alkylamino, methoxy, ethoxy, trifluoromethoxy, trifluoroethoxy, optionally substituted $C_{1-9}$ , $C_{1-9}$ cycloalkyl optionally with a substituent, $C_{1-9}$ alkenyl optionally with a substituent, $C_{1-9}$ cycloalkenyl optionally with a substituent, aryl optionally with a substituent, heteroaryl optionally with a substituent, aralkyl optionally with a substituent, heteroaralkyl optionally with a substituent, heterocycloalkyl optionally with a substituent, or heterocycloalkenyl optionally with a substituent; and the foregoing group optionally with a substituent may be substituted by halogen, cyano, hydroxy, nitro, amino, alkylamino, cycloalkylamino, aminocarbonyl, sulfonyl, aminosulfonyl, carbonylamino, sulfonylamino, methyl, ethyl, methoxy, ethoxy, trifluoromethyl, trifluoroethyl, trifluoromethoxy, trifluoroethoxy, heterocycloalkyl, aryl, heteroaryl, polyoxyethylene, polyoxypropylene, $C_{1-3}$ alkyl polyoxyethylene, or $C_{1-3}$ alkyl polyoxypropylene. Y and Z may each independently be hydrogen, $C_{1-9}$ alkyl optionally with a substituent, monocyclic or bicyclic $C_{1-9}$ cycloalkyl optionally with a substituent, $C_{1-9}$ alkenyl optionally with a substituent, monocyclic or bicyclic $C_{1-9}$ cycloalkenyl optionally with a substituent, aryl optionally with a substituent, heteroaryl optionally with a substituent, aralkyl optionally with a substituent, heteroaralkyl optionally with a substituent, heterocycloalkyl optionally with a substituent, or heterocycloalkenyl optionally with a substituent; any above group optionally with a substituent may be substituted by halogen, cyano, hydroxy, nitro, amino, alkylamino, cycloalkylamino, aminocarbonyl, sulfonyl, aminosulfonyl, carbonylamino, sulfonylamino, methyl, ethyl, methoxy, ethoxy, trifluoromethyl, trifluoroethyl, trifluoromethoxy, trifluoroethoxy, polyoxyethylene, polyoxypropylene, $C_{1-3}$ alkyl polyoxyethylene, or $C_{1-3}$ alkyl polyoxypropylene; or Y and Z may be linked to form a ring optionally with a substituent and having 3 to 10 ring atoms; the group optionally with a substituent is substituted by halogen, cyano, hydroxy, oxo, nitro, amino, alkylamino, cycloalkylamino, aminocarbonyl, sulfonyl, aminosulfonyl, carbonylamino, sulfonylamino, methyl, ethyl, methoxy, ethoxy, trifluoromethyl, trifluoroethyl, trifluoromethoxy, trifluoroethoxy, polyoxyethylene, polyoxypropylene, $C_{1-3}$ alkyl polyoxyethylene, or $C_{1-3}$ alkyl polyoxypropylene.

In some cases, in the compound represented by formula II, R may be hydrogen, methyl or trifluoromethyl. In some specific cases, in the compound represented by formula II, R may be hydrogen or methyl. For example, in the compound represented by formula II, R is hydrogen.

In some other cases, in the triazolotriazine compound represented by formula II, $Ar_1$ may be imidazolyl, triazolyl, tetrazolyl, furanyl, thienyl, oxazolyl, isoxazolyl, thiazolyl, isothiazolyl, oxadiazolyl, thiadiazolyl, phenyl, pyridyl, pyridazinyl, pyrimidinyl or triazinyl, possibly with a substituent; and any above group with a substituent may be substituted by halogen, oxo, cyano, methyl, methoxy, trifluoromethyl or trifluoromethoxy.

In some other cases, in the compound represented by formula II, $Ar_1$ is selected from aromatic groups in Table (2). For example, in the compound represented by formula II, $Ar_1$ is 2-furanyl.

Table 2 Structure of $Ar_1$ in formula II

| | | | | |
|---|---|---|---|---|
| 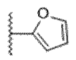 | 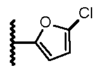 | 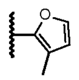 | 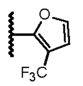 | 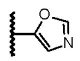 |
| 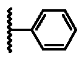 | 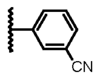 | 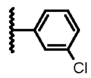 | 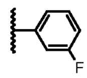 | 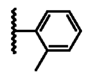 |
| 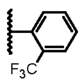 | 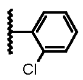 | 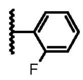 | 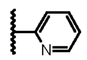 | 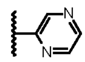 |

In some cases, in the compound represented by formula II, $Ar_2$ may be imidazolyl, triazolyl, tetrazolyl, furanyl, thienyl, oxazolyl, isoxazolyl, thiazolyl, isothiazolyl, oxadiazolyl, thiadiazolyl, phenyl, pyridyl, pyridazinyl, pyrimidinyl, pyrazinyl, triazinyl, indolyl, isoindolyl, indazolyl, benzimidazolyl, azaindolyl, azaindazolyl, purinyl, benzofuranyl, isobenzofuranyl, benzothienyl, benzisoxazolyl, benzisothiazole, benzoxazolyl, benzothiadiazole, quinolinyl, isoquinolinyl, quinoxalinyl, phthalazinyl, quinazolinyl, cinnolinyl, naphthyridinyl, pyridopyrimidinyl, pyridopyrazine, or pteridyl, optionally with a substituent; and the aromatic ring optionally with a substituent is substituted by halogen, hydroxy, cyano, nitro, methyl, methoxy, trifluoromethyl or trifluoromethoxy.

In some cases, in the compound represented by formula II, $Ar_2$ may be phenyl, pyridyl, pyridazinyl or pyrimidinyl, optionally with a substituent; and any above aromatic ring optionally with a substituent may be substituted by halogen, hydroxy, cyano, methyl, methoxy, trifluoromethyl or trifluoromethoxy. In some cases, in the compound represented by formula II, $Ar_2$ may be phenyl or pyridyl optionally substituted by halogen or hydroxy.

In some cases, in the compound represented by formula II, Q may be a monocyclic or bicyclic aromatic ring; X may be halogen, cyano, hydroxy, nitro, amino, alkylamino, methoxy, ethoxy, trifluoromethoxy, trifluoroethoxy, $C_{1-9}$ alkyl optionally with a substituent, $C_{1-9}$ cycloalkyl optionally with a substituent, $C_{1-9}$ alkenyl optionally with a substituent, $C_{1-9}$ cycloalkenyl optionally with a substituent, aryl optionally with a substituent, heteroaryl optionally with a substituent, aralkyl optionally with a substituent, heteroaralkyl optionally with a substituent, heterocycloalkyl optionally with a substituent, or heterocycloalkenyl optionally with a substituent; and the above group optionally with a substituent may be substituted by halogen, cyano, hydroxy, nitro, amino, alkylamino, cycloalkylamino, aminocarbonyl, sulfonyl, aminosulfonyl, carbonylamino, sulfonylamino, methyl, ethyl, methoxy, ethoxy, trifluoromethyl, trifluoroethyl, trifluoromethoxy, trifluoroethoxy, heterocycloalkyl, aryl, heteroaryl, polyoxyethylene, polyoxypropylene, $C_{1-3}$ alkyl polyoxyethylene, or $C_{1-3}$ alkyl polyoxypropylene.

In some cases, in the compound represented by formula II, Q may be a 5-6 membered aromatic ring optionally with a substituent. X may be halogen, cyano, hydroxy, nitro, amino, alkylamino, methoxy, ethoxy, trifluoromethoxy, trifluoroethoxy, $C_{1-5}$ alkyl possibly with a substituent, $C_{1-5}$ cycloalkyl possibly with a substituent, $C_{1-5}$ alkenyl possibly with a substituent, $C_{1-5}$ cycloalkenyl possibly with a substituent, aryl possibly with a substituent, heteroaryl possibly with a substituent, aralkyl possibly with a substituent, heteroaralkyl possibly with a substituent, heterocycloalkyl possibly with a substituent, or heterocycloalkenyl possibly with a substituent; and above group with a substituent is substituted by halogen, cyano, hydroxy, nitro, amino, alkylamino, cycloalkylamino, aminocarbonyl, sulfonyl, aminosulfonyl, carbonylamino, sulfonylamino, methyl, ethyl, methoxy, ethoxy, trifluoromethyl, trifluoroethyl, trifluoromethoxy, trifluoroethoxy, heterocycloalkyl, aryl, heteroaryl, polyoxyethylene, polyoxypropylene, $C_{1-3}$ alkyl polyoxyethylene, or $C_{1-3}$ alkyl polyoxypropylene.

In some cases, in the compound represented by formula II, Q may be tetrazole ring optionally substituted by X. X may be $C_{1-3}$ alkyl optionally with a substituent, or heterocycloalkyl optionally with a substituent; and any above group optionally with a substituent may be substituted by halogen, cyano, hydroxy, methyl, ethyl, methoxy, ethoxy, trifluoromethyl, trifluoroethyl, trifluoromethoxy, trifluoroethoxy, heterocycloalkyl, aryl or heteroaryl.

The compound of the present application may exist in the form of one or more geometric isomers, enantiomers, diastereomers or tautomers. The compound of the present application may comprise all these forms of isomers, comprising racemates and other forms of mixtures.

In some cases, the compound of the present application may exist in the form of solvates or non-solvates. The term "solvate" is used herein to describe a compound complex comprising a compound of the present invention and a pharmaceutically acceptable solvent molecule such as water and ethanol molecules. The compound of the present application comprises all their solvate or non-solvate forms. In some cases, the compound of the present application may exist in the form of pharmaceutically acceptable salts. The term "pharmaceutically acceptable salt" refers to a physiologically or toxicologically allowable salt, and when appropriate, comprises pharmaceutically acceptable base addition salt and acid addition salt thereof. The compound of the present application comprises all pharmaceutically acceptable salts thereof. For example, the pharmaceutically acceptable salt may comprise benzenesulfonate and/or

acetate.

In some cases, the compound of the present application may exist in the form of pharmaceutically acceptable nanoparticles. The nanoparticles containing the compound may be designed to improve the pharmacokinetics and biodistribution properties of the drug. For example, the compound may be encapsulated in a liposome, which may be able to extend the life of the drug in an in vivo distribution process. Since the nanoparticles would preferentially leak out in porous blood vessels around tumor cells, the nanoparticles of appropriate size may also have better safety. This may also help reduce the effective dose of the drug.

In some cases, the compound of the present application may exist in the form of prodrugs. The term "prodrug" generally refers to a compound that may be converted into the compound of the present application through an in vivo metabolic process (for example, by hydrolysis, reduction or oxidation).

The compound of the present application comprises all forms of prodrugs.

In some cases, the compound of the present application further comprises pharmaceutically acceptable isotopic variants, in which one or more atoms are substituted by atoms having the same atomic number but different atomic masses. Atoms suitable for such isotopic replacement comprise hydrogen, carbon, nitrogen, oxygen, phosphorus, sulfur, fluorine, iodine, and chlorine. Some isotopic variants of the compound, such as deuterium-substituted compounds, may have a better therapeutic effect in some cases due to their better metabolic stability. Those skilled in the art may prepare the isotopic variants of the compound with conventional techniques known in the art.

In the present application, the compound may comprise the following compounds, or pharmaceutically acceptable salts thereof:

Table 3 Exemplary compound of the present application

| Compound No. | Designation | Structural formula |
|---|---|---|
| Compound 1 | N-(4-(2-((7-amino-2-(furan-2-yl)-[1,2,4]triazolo[1,5-a][1,3,5]triazin-5-yl)amino)ethyl)phenyl)-3-morpholine propanamide; | 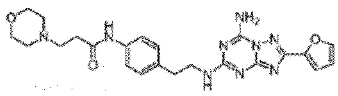 |
| Compound 2 | N-(4-(2-((7-amino-2-(furan-2-yl)-[1,2,4]triazolo[1,5-a][1,3,5]triazin-5-yl)amino)ethyl)phenyl)-2-morpholine propanamide; | 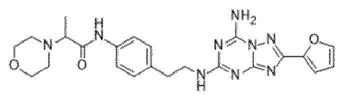 |
| Compound 3 | N-(4-(2-((7-amino-2-(furan-2-yl)-[1,2,4]triazolo[1,5-a][1,3,5]triazin-5-yl)amino)ethyl)phenyl)pivalamide; | 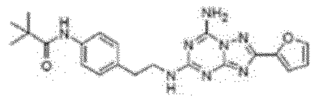 |
| Compound 4 | N-(4-(2-((7-amino-2-(furan-2-yl)-[1,2,4]triazolo[1,5-a][1,3,5]triazin-5-yl)amino)ethyl)phenyl)-3 -hydroxy- 2,2-dimethylpropionamide; | 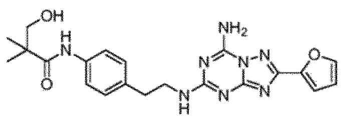 |
| Compound 5 | N-(4-(2-((7-amino-2-(furan-2-yl)-[1,2,4]triazolo[1,5-a][1,3,5]triazin-5-yl)amino)ethyl)phenyl)-2-hydroxy-2-methylpropionamide; | 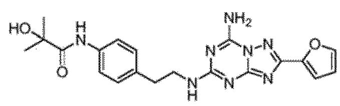 |
| Compound 6 | N-(4-(2-((7-amino-2-(furan-2-yl)-[1,2,4]triazolo[1,5-a][1,3,5]triazin-5-yl)amino)ethyl)phenyl)-4-methyltetrahydro-2H-pyran-4-carboxamide; | 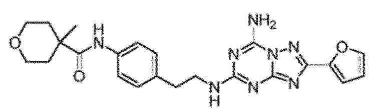 |
| Compound 7 | 2-(furan-2-yl)-N5-(2-(pyrimidin-4-yl)ethyl)-[1,2,4]triazolo[1,5- a][1,3,5]triazine-5,7-diamine; | 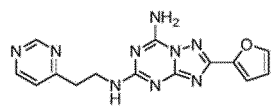 |
| Compound 8 | 2-(furan-2-yl)-N5-(2-(pyridin-3-yl)ethyl)-[1,2,4]triazolo[1,5-a][1,3,5]triazine-5,7-diamine; | 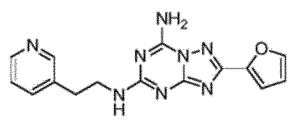 |

(continued)

| Compound No. | Designation | Structural formula |
|---|---|---|
| Compound 9 | 2-(furan-2-yl)-N5-(4-(2-(pyrrolidin-1-yl)ethoxy) phenethyl)-[1,2,4]triazolo[1,5-a][1,3,5]-triazine-5,7-diamine; | 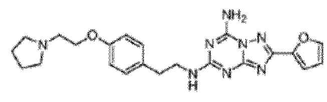 |
| Compound 10 | N5-(4-(2-(azetidine-1-yl)ethoxy)phenethyl)-2-(furan-2-yl)-[1,2,4]triazolo[1,5-a][1,3,5]triazine-5,7-diamine; | 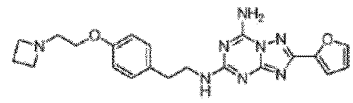 |
| Compound 11 | 2-(furan-2-yl)-N5-(2-(6-(2-methyl-2H-tetrazol-5-yl)pyridin-3-yl)ethyl)-[1,2,4]triazolo[1,5-a][1,3,5]triazine-5,7-diamine | 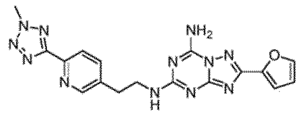 |
| Compound 12 | 2-(furan-2-yl)-N5-(4-(2-(4-methylpiperazin-1-yl)ethoxy) phenethyl)-[1,2,4]triazolo[1,5-a][1,3,5]triazine-5,7-diamine; | 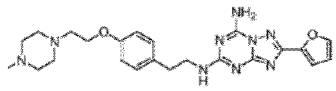 |
| Compound 13 | 2-(furan-2-yl)-N5-(4-(3-(4-methylpiperazin-1-yl)propoxy) phenethyl)-[1,2,4]triazolo[1,5-a][1,3,5]triazine-5,7-diamine; | 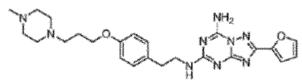 |
| Compound 14 | N5 -(4-(2-(3,3 -difluoropyrrolidin-1-yl)ethoxy)phenethyl)-2-(furan-2-yl)-[1,2,4]triazolo[1,5-a][1,3,5]triazine-5,7-diamine; | 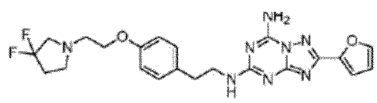 |
| Compound 15 | (R)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine; | 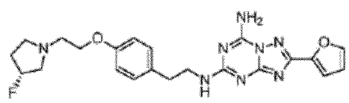 |
| Compound 16 | (S)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine; | 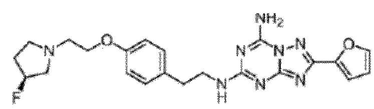 |
| Compound 17 | 2-(furan-2-yl)-N5-(4-((2-morpholinoethyl)amino) phenethyl)-[1,2,4]triazolo[1,5-a][1,3,5]triazine-5,7-diamine; | 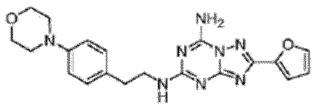 |
| Compound 18 | 2-(furan-2-yl)-N5-(4-((tetrahydro-2H-pyran-4-yl)amino) phenethyl)-[1,2,4]triazolo[1,5-a] [1,3,5]triazine-5,7-diamine; | 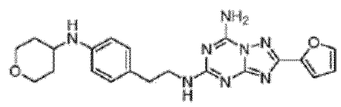 |
| Compound 19 | 2-(furan-2-yl)-N5-(4-((tetrahydrofuran-3-yl)amino) phenethyl)-[1,2,4]triazolo[1,5-a][1,3,5]triazine-5,7-diamine; | 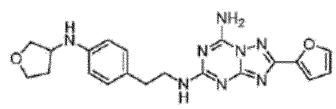 |
| Compound 20 | 2-(furan-2-yl)-N5-(4-(4-methylpiperazin-1-yl) phenethyl)-[1,2,4]triazolo[1,5-a][1,3,5]triazine-5,7-diamine; | 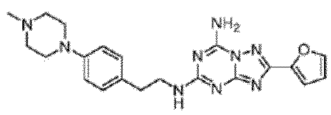 |
| Compound 21 | N5-(4-(bis(2-methoxyethyl)amino)phenethyl)-2-(furan-2-yl)-[1,2,4]triazolo[1,5-a][1,3,5]triazine-5,7-diamine; | 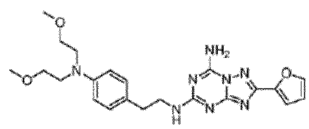 |

(continued)

| Compound No. | Designation | Structural formula |
|---|---|---|
| Compound 22 | 2-(furan-2-yl)-N5-(4-(oxetan-3-ylamino)phenethyl)-[1,2,4] triazolo[1,5-a][1,3,5]triazine-5,7-diamine; | 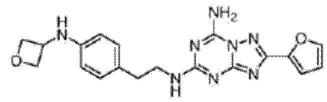 |
| Compound 23 | 2-(furan-2-yl)-N5-(4-(oxetan-3-ylmethyl)phenethyl)-[1,2,4] triazolo[1,5-a] [1,3,5]triazine-5,7-diamine; | 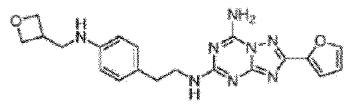 |
| Compound 24 | 2-(furan-2-yl)-N5-(4-((1-methoxypropane-2-yl)amino) phenethyl)-[1,2,4]triazolo[1,5-a][1,3,5]triazine-5,7-diamine; | 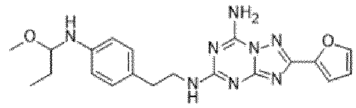 |
| Compound 25 | 2-(furan-2-yl)-N5-(4-((2-methoxyethyl)(methyl)amino) phenethyl)-[1,2,4]triazolo[1,5-a][1,3,5]triazine-5,7-diamine; | 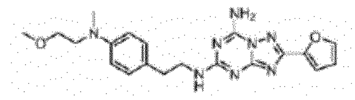 |
| Compound 26 | N5-(4-(ethyl(2-methoxyethyl)amino)phenethyl)-2-(furan-2-yl)-[1,2,4]triazolo[1,5-a][1,3,5]triazine-5,7-diamine; | 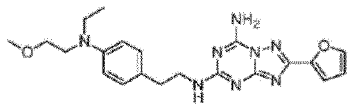 |
| Compound 27 | 2-(furan-2-yl)-N5-(4-((2-methoxyethyl)amino) phenethyl)-[1,2,4]triazolo[1,5-a] [1,3,5]triazine-5,7-diamine; | 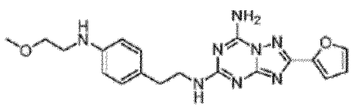 |
| Compound 28 | 2-(furan-2-yl)-N5-(4-((2-(2-methoxyethoxy)ethyl)amino) phenethyl)-[1,2,4]triazolo[1,5-a][1,3,5]triazine-5,7-diamine; | 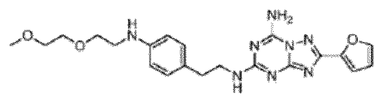 |
| Compound 29 | 2-(furan-2-yl)-N5-(4-((2-(2-(2-methoxyethoxy)ethoxy) ethyl)amino)phenethyl)-[1,2,4]triazolo[1,5-a][1,3,5] triazine-5,7-diamine; | 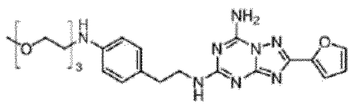 |
| Compound 30 | N5-(4-((2-ethoxyethyl)amino)phenethyl)-2-(furan-2-yl)-[1,2,4]triazolo[1,5-a][1,3,5]triazine-5,7-diamine; | 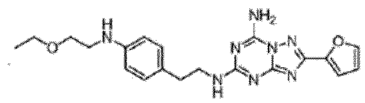 |
| Compound 31 | 2-(furan-2-yl)-N5-(4-((2-(2,2,2-trifluoroethoxy)ethyl)amino) phenethyl)-[1,2,4]triazolo[1,5-a][1,3,5]triazine-5,7-diamine; | 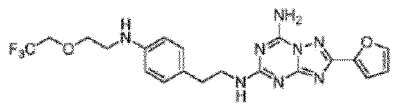 |
| Compound 32 | 2-(furan-2-yl)-N5-(4-((3-methoxypropyl)amino) phenethyl)-[1,2,4]triazolo[1,5-a][1,3,5]triazine-5,7-diamine; | 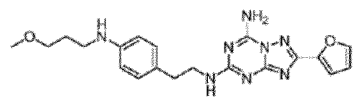 |
| Compound 33 | N5-(4-(2-methyl-2H-tetrazol-5-yl)phenethyl)-2-phenyl-[1,2,4]triazolo[1,5-a][1,3,5]triazine-5,7-diamine | 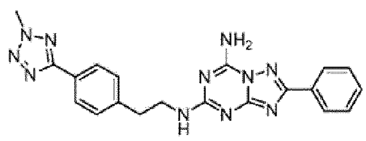 |
| Compound 34 | 2-((4-(2-((7-amino-2-(furan-2-yl)-[1,2,4]triazolo[1,5-a] [1,3,5]triazine-5-yl)amino)ethyl)-phenyl)amino)acetonitrile; | 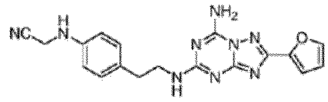 |

(continued)

| Compound No. | Designation | Structural formula |
|---|---|---|
| Compound 35 | N5-(4-(1,3-dimethoxypropane-2-ylamino)phenethyl)-2-(furan-2-yl)-[1,2,4]triazolo[1,5-a][1,3,5]triazine-5,7-diamine; | 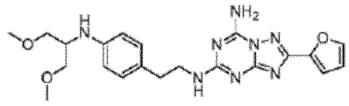 |
| Compound 36 | 2-(3-fluorophenyl)-N5-(4-((2-methoxyethyl)amino) phenethyl)-[1,2,4]triazolo[1,5-a][1,3,5]triazine-5,7-diamine | 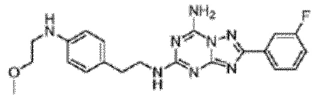 |
| Compound 37 | 2-(3-fluorophenyl)-N5-(4-(oxetan-3-ylamino) phenethyl)-[1,2,4]triazolo[1,5-a][1,3,5]triazine-5,7-diamine | 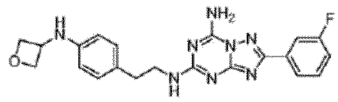 |
| Compound 38 | 4-(2-(7-amino-2-(furan-2-yl)-[1,2,4]triazolo[1,5-a][1,3,5] triazine-5-ylamino)ethyl)-N,N-dimethylbenzamide; | 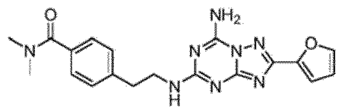 |
| Compound 39 | N5-(4-(1-methyl-1H-tetrazol-5-yl)phenethyl)-2-(furan-2-yl)-[1,2,4]triazolo[1,5-a][1,3,5]triazine-5,7-diamine; | 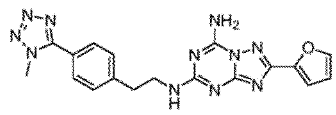 |
| Compound 40 | N5-(4-(2-methyl-2H-tetrazol-5-yl)phenethyl)-2-(furan-2-yl)-[1,2,4]triazolo[1,5-a][1,3,5]triazine-5,7-diamine; | 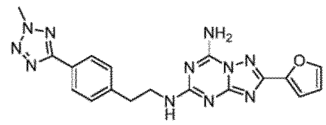 |
| Compound 41 | N5-(4-(2-ethyl-2H-tetrazol-5-yl)phenethyl)-2-(furan-2-yl)-[1,2,4]triazolo[1,5-a][1,3,5]triazine-5,7-diamine; | 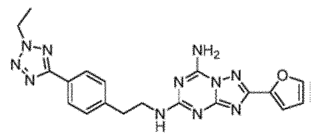 |
| Compound 42 | N5-(4-(2-isopropyl-2H-tetrazol-5-yl)phenethyl)-2-(furan-2-yl)-[1,2,4]triazolo[1,5-a][1,3,5]triazine-5,7-diamine; | 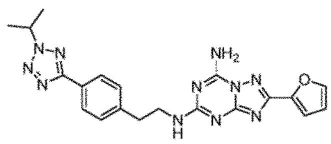 |
| Compound 43 | N5-(4-(2-propyl-2H-tetrazol-5-yl)phenethyl)-2-(furan-2-yl)-[1,2,4]triazolo[1,5-a][1,3,5]triazine-5,7-diamine; | 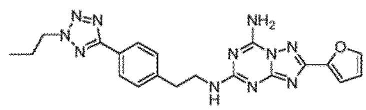 |
| Compound 44 | 2-(5-(4-(2-(7-amino-2-(furan-2-yl)-[1,2,4]triazolo[1,5-a] [1,3,5]triazine-5-ylamino)ethyl)-phenyl)-2H-tetrazol- 2-yl) acetonitrile; | 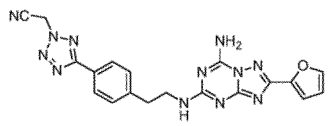 |
| Compound 45 | 4-(2-(7-amino-2-(furan-2-yl)-[1,2,4]triazolo[1,5-a][1,3,5] triazine-5-ylamino)ethyl)-benzonitrile; | 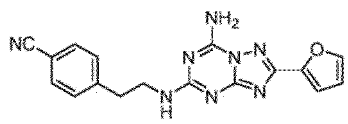 |
| Compound 46 | N5-(4-(2-(2,2,2-trifluoroethyl)-2H-tetrazol-5-yl)phenethyl)-2-(furan-2-yl)-[1,2,4]triazolo[1,5-a][1,3,5]triazine-5,7-diamine; | 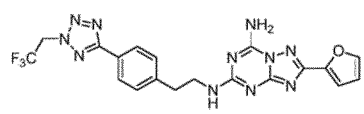 |

(continued)

| Compound No. | Designation | Structural formula |
|---|---|---|
| Compound 47 | N5-(4-(2-(2-methoxyethyl)-2H-tetrazol-5-yl)phenethyl)-2-(furan-2-yl)-[1,2,4]triazolo[1,5-a][1,3,5]triazine-5,7-diamine; | 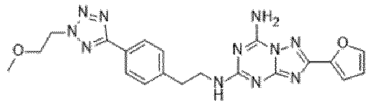 |
| Compound 48 | 2-(furan-2-yl)-N5-(4-(pyridin-2-yl)phenethyl)-[1,2,4]triazolo[1,5-a][1,3,5]triazine-5,7-diamine; | 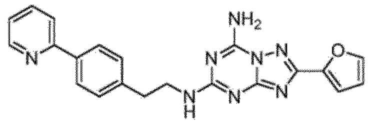 |
| Compound 49 | 2-(furan-2-yl)-N5-(4-(pyridin-3-yl)phenethyl)-[1,2,4]triazolo[1,5-a][1,3,5]triazine-5,7-diamine; | 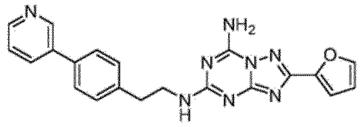 |
| Compound 50 | 2-(furan-2-yl)-N5-(4-(pyridin-4-yl)phenethyl)-[1,2,4]triazolo[1,5-a][1,3,5]triazine-5,7-diamine; | 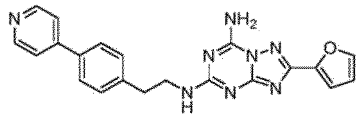 |
| Compound 51 | 2-(furan-2-yl)-N5-(4-(pyrimidin-2-yl)phenethyl)-[1,2,4]triazolo[1,5-a][1,3,5]triazine-5,7-diamine; | 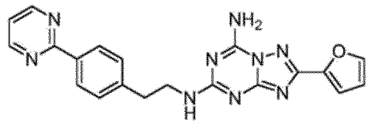 |
| Compound 52 | 2-(furan-2-yl)-N5-(4-(1-methyl-1H-1,2,4-triazol-3-yl)phenethyl)-[1,2,4]triazolo[1,5-a][1,3,5]triazine-5,7-diamine; | 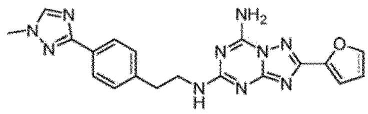 |
| Compound 53 | N5-(4-(2H-tetrazol-5-yl)phenethyl)-2-(furan-2-yl)-[1,2,4]triazolo[1,5-a][1,3,5]triazine-5,7-diamine; | 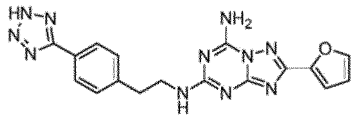 |
| Compound 54 | 2-(furan-2-yl)-N-(4-(methylsulfonyl)phenethyl)-[1,2,4]triazolo[1,5-a][1,3,5]triazine-5-amine; | 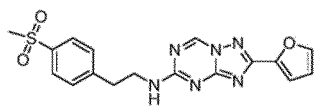 |
| Compound 55 | 2-(3-fluorophenyl)-N5-(4-(2-methyl-2H-tetrazol-5-yl)phenethyl)-[1,2,4]triazolo[1,5-a][1,3,5]triazine-5,7-diamine; | 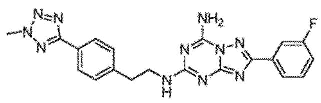 |

**Preparation of the compound of the present application**

The compound of the present application may be prepared with various synthetic methods. As an illustrative example, two synthetic routes for preparing a compound of interest are listed in synthetic route (1). In the first method, after an intermediate (1A) is obtained with an appropriate preparation method, methylsulfonyl on the intermediate (1A) is substituted by alkylamino to obtain a triazolotriazine compound (1C). In the second method, after an intermediate (IB) is obtained in an appropriate manner, phenoxy on the intermediate (IB) is substituted by alkylamino to obtain the triazolotriazine compound (1C).

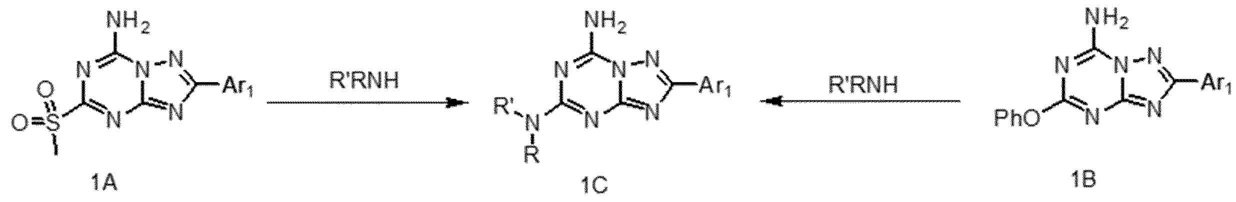


1A 1C 1B

Synthetic Route 1

The intermediates (1A) and (IB) required in the synthetic route (1) may also be prepared with various synthetic methods. As an illustrative example, a synthetic route of the intermediate compound (1A) is shown in the synthetic route (2). In the first step, appropriate aryl hydrazide (2A) reacts with S-methyl isothiourea (2B) in an aqueous solution of sodium hydroxide to obtain an intermediate (2C). In the following steps, (2C) is first heated in an aqueous medium to obtain an intermediate (2D), and the intermediate (2D) reacts with N-cyanodithioimino carbonate (2E) to obtain a sulfide intermediate (2F). Afterwards, the intermediate (2F) is oxidized with m-chloroperoxybenzoic acid to obtain the desired intermediate sulfone (1A). As shown in the synthetic route (1), the compound of interest (1C) can be obtained by nucleophilic substitution of methylsulfonyl with suitable alkylamine (J. Chem. Soc., Perkin Trans. 1 1995, 801-808; Structural Chemistry. 2013, 24, 1241-1251).

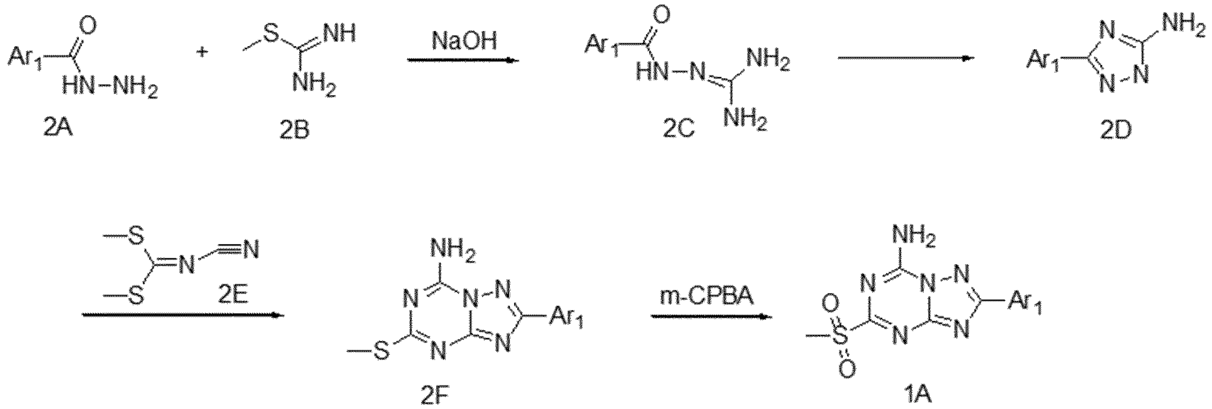


2A 2B 2C 2D 2E 2F 1A

Synthetic Route 2

The intermediate (2D) in the synthetic route (2) may also be prepared with various synthetic methods. As an illustrative example, synthetic route (3) shows three synthetic routes for the intermediate (2D). It should be noted that among the three methods, the synthetic route starting with methyl or ethyl (3F) can generally increase efficiency and achieve a higher reaction yield.

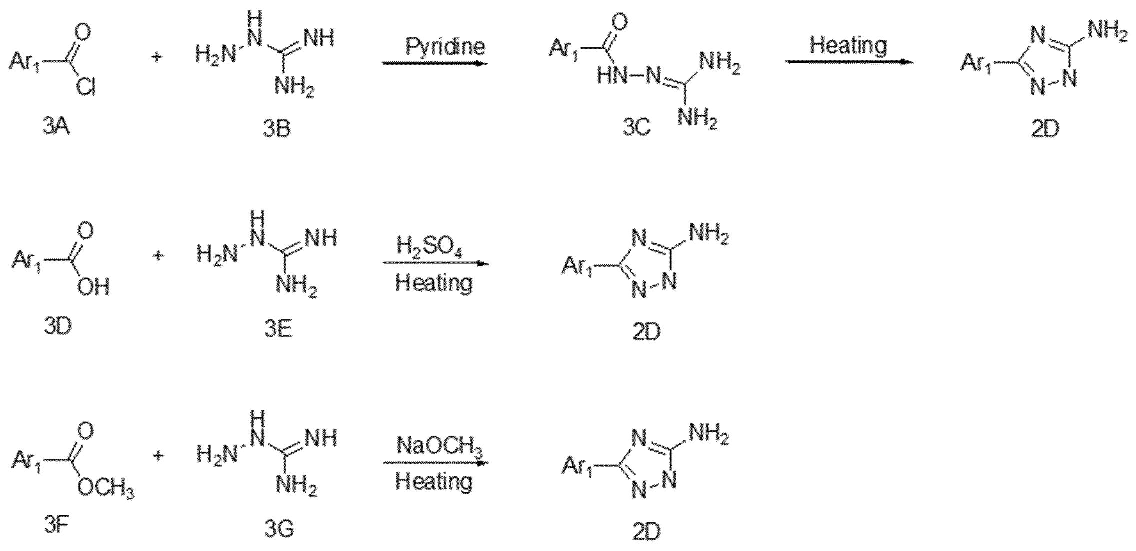


Synthetic Route 3

The intermediate (IB) in the synthetic route (1) may be prepared by a synthetic route shown in the synthetic route (4). The preparation first starts with cyanuric chloride (4A), and refluxing is carried out in phenol to obtain 2,4,6-triphenoxy-1,3,5-triazine (4B). In the next step, a reaction occurs with hydrazine hydrate to obtain 2-hydrazine-4,6-diphenoxy-1,3,5-triazine (4C), which reacts with suitable acyl chloride to obtain acylhydrazide (4D). Then, hydrazide (4D) undergoes a cyclization reaction under a dehydration condition to obtain 2-substituted 5,7-diphenoxytriazolotriazine (4E). In the last step, (4E) is refluxed in carbinolamine to obtain the key intermediate (IB) (J. Chem. Soc., Perkin Trans. 1 1995, 801-808). As shown in route (1), the compound of interest (1C) can be prepared by reacting (IB) with a suitable alkylamine.

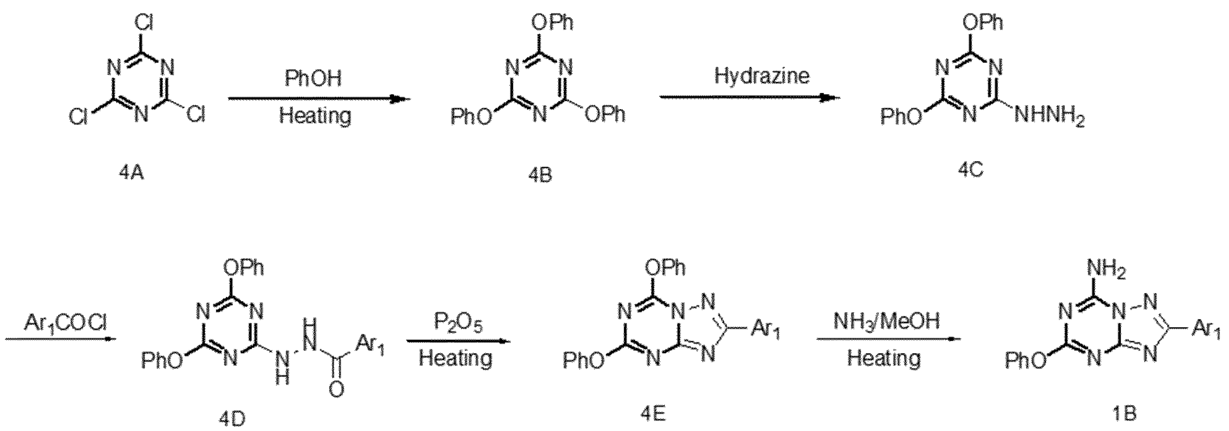


Synthetic Route 4

The compound of the present application may bind to adenosine receptors, for example, adenosine A2A receptors. The compound of the present application may bind to free adenosine receptor proteins or adenosine receptors on cell surfaces. The compound of the present application may selectively bind to the adenosine receptors. For example, the capability for the compound of the present application to bind the adenosine A2A receptors is at least 10 folds, 20 folds, 30 folds, 40 folds, 50 folds, 100 folds, 200 folds, 300 folds, 500 folds or above the capability for the same to bind adenosine A1, A2B, and A3 receptors. For example, with the radioisotope ligand competitive binding technology, the $IC_{50}$ value for the compound of the present application to bind the adenosine A2A receptors is at least 10 folds, 20 folds, 30 folds, 40 folds, 50 folds, 100 folds, 200 folds, 300 folds, 500 folds or above the $IC_{50}$ value for the same to bind the adenosine A1, A2B, and A3 receptors.

The compound of the present application may inhibit the activity of adenosine receptors, for example, the activity of adenosine A2A receptors. The compound of the present application may inhibit the activity of free adenosine receptor

proteins, or the activity of adenosine receptors on cell surfaces. The compound of the present application may selectively inhibit the activity of adenosine receptors. For example, the capability for the compound of the present application to inhibit the activity of the adenosine A2A receptors is at least 10 folds, 20 folds, 30 folds, 40 folds, 50 folds, 100 folds, 200 folds, 300 folds, 500 folds or above the capability for the same to inhibit the adenosine A1, A2B, and A3 receptors. For example, with the accumulation assay or calcium flux experiment, the $IC_{50}$ value for the compound of the present application to inhibit the activity of the adenosine A2A receptors is at least 10 folds, 20 folds, 30 folds, 40 folds, 50 folds, 100 folds, 200 folds, 300 folds, 500 folds or above the $IC_{50}$ value for the same to inhibit the activity of the adenosine A1, A2B, and A3 receptors.

**Uses and methods**

**Parkinson's Disease**

The compound of the present application can be used to prepare a drug for treating diseases, wherein the diseases may comprise Parkinson's disease.

In another aspect, the present application provides a method for treating Parkinson's disease, which may comprise the following steps: administering a therapeutically effective dose of the compound of the present application to a subject in need thereof.

In some embodiments, the subject may exhibit early signs of the Parkinson's disease, and the method comprises verifying that a patient exhibits early signs of the Parkinson's disease, and administering the compound or the pharmaceutically acceptable solvate or salt thereof at a dose determined therefrom to the patient at regular intervals to effectively treat the patient.

The early signs may include structural or functional changes in the brain, which may be detected for example physically, for example, by PET and SPECT studies, by transcranial echocardiography (Becker, J Neurol 249, Suppl 3. 2002, III/40; Prunier C, et al. Neuroimage. 2003 Jul; 19(3):810-6), or by detecting biochemical markers such as neuromelanin (WO 02/31499).

In some embodiments, the rate of progression of Parkinson's disease in the subject may be quantified by the total Unified Parkinson's Disease Rating Scale (total UPDRS) scores. The early signs may also include olfactory disorders, depression, vison disorders, and cognitive impairment or sleep disorders, which can be diagnosed at an early stage by using a combination of different tests (Becker, J Neurol 249, Suppl 3,2002 111/40; Stern, Annals of Neurol 56, 2004, 169).

In some embodiments, the subject may exhibit functional decline associated with the Parkinson's disease, and the functional decline generally refers to the deterioration of the symptoms of a patient with Parkinson's disease over time as determined by the total UPDRS score.

In some embodiments, the subject may exhibit fatigue associated with the Parkinson's disease, and the fatigue generally refers to a fatigue state of a patient with Parkinson's disease as determined by the total UPDRS score.

In some embodiments, the subject may exhibit non-motor symptoms associated with the Parkinson's disease, and the non-motor symptoms generally refer to non-motor symptoms of a patent with Parkinson's disease as determined by the total UPDRS score. The non-motor symptoms associated with the Parkinson's disease may comprise autonomic nervous system dysfunction, neuropsychiatric disorders (comprising changes in mood, cognition, behavior, and thought), sensory disorders, sleep disorders, etc.

In some embodiments, the subject has previously received or not received a therapy for treating Parkinson's disease, comprising medications, rehabilitation (such as physical training, gait improvement, slow rotation of limbs or torso, stretching exercises, oral guidance, Lee Silverman voice treatment, stepping exercise, environmental reconstruction, rhythm initiation, abdominal respiration and meditation), palliative medical treatment, dietotherapy, repetitive transcranial magnetic stimulation, cautery, deep brain stimulation surgery, and pallidectomy.

In some embodiments, the subject may have previously received or not received a drug for treating Parkinson's disease. Drugs for treating the Parkinson's disease may comprise, but are not limited to, L-dopa, dopamine receptor agonists, monoamine oxidase inhibitors, and other drugs such as amantadine and anticholinergics. Drugs for treating the Parkinson's disease may comprise: L-dopa, bromocriptine, benztropine, levodopa, ropinirole, pramipexole, rotigotine, cabergoline, safinamide, selegiline, rasagiline, entacapone, tolcapone, amantadine, pergolide, apomorphine, lisuride, or eldepryl.

In some embodiments, the described treating may comprise reducing the rate of progression of the Parkinson's disease in the subject, and/or delaying the clinical progression of the Parkinson's disease in the subject. After starting to administer the compound of the present application or the pharmaceutically acceptable solvate or salt thereof, the rate of disease progression is manifested as a decrease in the average increase of the total UPDRS score.

In some embodiments, the described treating may comprise delaying the need of the subject for symptomatic treatment against Parkinson's disease. In some embodiments, the described treating may comprise determining that a patient is in the stage of Parkinson's disease, and administering the compound or the pharmaceutically acceptable

solvate or salt thereof at a dose determined therefrom to the patient at regular intervals to effectively delay his/her need for the symptomatic treatment against Parkinson's disease.

In some embodiments, the described treating may comprise reducing a risk in the need the subject for the symptomatic treatment against Parkinson's disease. In some embodiments, the described treating comprises administering the compound of the present application or the pharmaceutically acceptable solvate or salt thereof at certain dose to the patient with Parkinson's disease at regular intervals to effectively reduce the risk in need for the symptomatic treatment against Parkinson's disease.

In some embodiments, the described treating may comprise reducing the functional decline of the subject. In some embodiments, the method comprises determining that the patient is in the stage of Parkinson's disease, and administering the compound or the pharmaceutically acceptable solvate or salt thereof at a dose determined therefrom to the patient at regular intervals to effectively reduce his/her functional decline. In some embodiments, the functional decline of a patient with Parkinson's disease is quantified by the total Unified Parkinson's Disease Rating Scale (total UPDRS) scores, an increase of which represents functional decline.

In some embodiments, the described treating may comprise reducing the fatigue of the subject. In some embodiments, the method comprises determining that the patient is in the stage of Parkinson's disease, and administering the compound of the present application or the pharmaceutically acceptable solvate or salt thereof at a dose determined therefrom to the patient at regular intervals to effectively reduce fatigue.

In some embodiments, the described treating may comprise reducing the severity of motor symptoms of the subject. The motor symptoms may mainly comprise tremor (such as tremor at rest), limb stiffness, slow movement, posture instability, hypokinesia, bradykinesia, abnormal posture, speech and swallowing, etc.

In some embodiments, the described treating may comprise reducing the severity of the non-motor symptoms of the subject. In some embodiments, the method comprises determining that the patient is in the stage of Parkinson's disease, and administering the compound or the pharmaceutically acceptable solvate or salt thereof at a dose determined therefrom to the patient at regular intervals to effectively reduce the severity of the non-motor symptoms. In some embodiments, the non-motor symptoms are defined by the Unified Scoring Scale for Parkinson's Disease (UPDRS), Fourth edition, Part I.

The total UPDRS (Unified Parkinson's Disease Rating Scale) score represents the severity of Parkinson's disease, and is used to measure changes in efficacy parameters from baseline during treatment. UPDRS consists of the following tests:

UPDRS consists of the following parts: Part I: assessment of mentation, behavior and mood; Part II: self-assessment of activities of daily living (ADL), comprising speech, swallowing, handwriting, dressing, hygiene, falling, salivation, rest in bed, walking and cutting food; Part III: assessment of monitored motor based on clinician score; Part IV: treatment of complications; Part V: Hoehn and Yahr staging for severity of Parkinson's disease; and Part VI: Schwab and England ADL scale.

The MDS-UPDRS issued by the Movement Disorder Association may also be used to score the Parkinson's disease. It consists of four parts and a total of 50 subscales, including: (1) non-motor aspects of experiences of daily living (13 items); (2): motor aspects of experiences of daily living (13 items); (3) physical motor examination (18 items); and (4) motor complications (6 items). The score for each subscale is 0-4, with 0 = normal, 1 = slight, 2 = mild, 3 = moderate, and 4 = severe. A score of 0-4 is obtained by completing each item, with 0 representing no disorder and 4 representing the most severe disorder.

The compound of the present application may decrease the score(s) of one or more UPDRS subscale items of a subject from 4 to 3, from 4 to 2, from 4 to 1, from 4 to 0, from 3 to 2, from 3 to 1, from 3 to 0, from 2 to 1, from 2 to 0, or from 1 to 0.

The compound of the present application may increase the walking distance, walking time, number of standings and standing time of a subject.

For example, compared with the case without the administration of the compound of the present application, the walking distance of a subject using the compound of the present application can be increased by at least 10%, at least 20%, at least 30%, at least 40%, at least 50%, at least 60%, at least 70%, at least 80%, at least 90%, at least 100% or higher, as detected in a mouse dyskinesia model induced by reserpine.

For example, compared with the case without the administration of the compound of the present application, the walking time of a subject using the compound of the present application can be increased by at least 10%, at least 20%, at least 30%, at least 40%, at least 50%, at least 60%, at least 70%, at least 80%, at least 90%, at least 100% or higher, as detected in a reserpine-induced mouse dyskinesia model.

For example, compared with the case without the administration of the compound of the present application, the number of standings of a subject using the compound of the present application can be increased by at least 10%, at least 20%, at least 30%, at least 40%, at least 50%, at least 60%, at least 70%, at least 80%, at least 90%, at least 100% or higher, as detected in a reserpine-induced mouse dyskinesia model.

For example, compared with the case without the administration of the compound of the present application,

the standing time of a subject using the compound of the present application can be increased by at least 10%, at least 20%, at least 30%, at least 40%, at least 50%, at least 60%, at least 70%, at least 80%, at least 90%, at least 100% or higher, as detected in a reserpine-induced mouse dyskinesia model.

The compound of the present application can reduce the on-bar time and increase the standing time for the subject, and effectively ameliorate the stiff symptoms of the subject.

For example, compared with the case without the administration of the compound of the present application, the on-bar time of a subject using the compound of the present application can be reduced by at least 10%, at least 20%, at least 30%, at least 40%, at least 50%, at least 60%, at least 70%, at least 80%, at least 90%, at least 100%, at least 200%, at least 300% or higher, as detected in a haloperidol-induced rat Parkinson's disease stiffness model.

For example, compared with the case without the administration of the compound of the present application, the standing time of a subject using the compound of the present application may be increased by at least 10%, at least 20%, at least 30%, at least 40%, at least 50%, at least 60%, at least 70%, at least 80%, at least 90%, at least 100%, at least 200%, at least 300% or higher, as detected in a haloperidol-induced rat Parkinson's disease stiffness model or in an MPTP-induced mouse Parkinson's disease model.

For example, compared with the case without the administration of the compound of the present application, the walking distance of a subject using the compound of the present application can be increased by at least 10%, at least 20%, at least 30%, at least 40%, at least 50%, at least 60%, at least 70%, at least 80%, at least 90%, at least 100%, at least 200%, at least 300% or higher, as detected in an MPTP-induced mouse Parkinson's disease model.

In the present application, in the treatment of Parkinson's disease, the administered dose of the compound may be about 0.001 to about 500 mg/kg, about 0.001 to about 500 mg/kg, about 0.01 to about 500 mg/kg, about 0.1 to about 500 mg/kg, about 1 to about 500 mg/kg, about 0.001 to about 400 mg/kg, about 0.001 to about 300 mg/kg, about 0.001 to about 200 mg/kg, about 0.001 to about 100 mg/kg, about 0.001 to about 50 mg/kg, about 1 to about 200 mg/kg, about 1 to about 100 mg/kg, about 1 to about 50 mg/kg, about 1 to about 30 mg/kg, about 10 to about 30 mg/kg, about 3 to about 30 mg/kg or about 5 to about 30 mg/kg. The administered dose of the compound may be about 1 mg/kg, about 3 mg/kg, about 5 mg/kg, about 7.5 mg/kg, about 10 mg/kg, about 30 mg/kg, about 50mg/kg or about 100 mg/kg.

The compound of the present application can inhibit the drug resistance of a subject to anti-Parkinson drugs, for example, to levodopa (L-DOPA) or Benderizine.

**Tumor**

The compound of the present application can further be used to prepare a drug for treating or preventing cancers in a host and related diseases associated with abnormal cell proliferation in the host. The host may be any multicellular vertebrate, comprising humans and non-human mammals. The host may be a human. The diseases comprise tumors. The tumors may comprise solid tumors and non-solid tumors. In the present application, the solid tumors may comprise a colon cancer and melanoma. In the present application, the non-solid tumors may comprise lymphoma, for example, B-cell lymphoma.

In the present application, in the treatment of tumors, the administered dose of the compound may be about 0.001 to about 500 mg/kg, about 0.001 to about 500 mg/kg, about 0.01 to about 500 mg/kg, about 0.1 to about 500 mg/kg, about 1 to about 500 mg/kg, about 0.001 to about 400 mg/kg, about 0.001 to about 300 mg/kg, about 0.001 to about 200 mg/kg, about 0.001 to about 100 mg/kg, about 0.001 to about 50 mg/kg, about 1 to about 200 mg/kg, about 1 to about 100 mg/kg, about 1 to about 50 mg/kg, about 1 to about 30 mg/kg, about 10 to about 30 mg/kg, about 3 to about 30 mg/kg or about 5 to about 30 mg/kg. The administered dose of the compound may be about 1 mg/kg, about 3 mg/kg, about 5 mg/kg, about 7.5 mg/kg, about 10 mg/kg, about 30 mg/kg, about 50mg/kg or about 100 mg/kg.

The compound of the present application can be used to improve the anti-tumor activity of immune cells in the host. The compound can reduce the immune anergy of T cells or the tolerance of T cells to a cancer, make the cancer cells more susceptible to immune clearance, inhibit the proliferation of regulatory T cells, and contribute to the formation of memory T cells. The compound can not only improve the host's own immune response, but also can improve its efficacy on various adaptive immunotherapies.

For example, the drug of the present application can inhibit or delay the development or progression of a disease, reduce the size of a tumor (or even substantially eliminate the tumor), and/or alleviate and/or stabilize a disease state. Examples of inhibiting the growth of tumors or tumor cells comprise: a reduction in tumor growth volume with respect to a corresponding level before intervention.

In another aspect, the present application provides a method for treating tumors. The method may comprise the step of administering the compound of the present application at a therapeutically effective dose to a subject in need thereof.

In order to make it more effective, the compound of the present application may also be used in combination with other anti-tumor treatment methods (for example, chemotherapies, tumor vaccines, and various immune checkpoint inhibitors), thereby achieving a synergistic effect. The term "combined treatment" refers to the simultaneous administration

of active agents, or the sequential administration in different orders.

In some embodiments, the method for treating or preventing abnormal cell proliferation in the host may comprise administering the compound of the present application and an immune checkpoint inhibitor in combination or alternately to a patient. The immune checkpoint inhibitors here may be selected from the group consisting of: PD-1 inhibitors, PD-L1 inhibitors, PD-L2 inhibitors, CTLA-4 inhibitors, BTLA inhibitors, LAG3 inhibitors, TIM-3 inhibitors, B7-H3 inhibitors, B7-H4 inhibitors, KIR inhibitors, TIGIT inhibitors or VISTA inhibitors, etc. For example, the described other anti-tumor treatment methods may include the PD-1 inhibitors.

In another embodiment, the method for treating or preventing abnormal cell proliferation in the host may comprise administering the compound of the present application and a cell vaccine in combination or alternately to a patient. The cell vaccine is based on cells matched with the tumor to be prevented. For example, if a host suffers from a prostate cancer, or is at risk of suffering from a prostate cancer, the cell vaccine will be based on prostate cancer cells. In this case, the cells usually undergo certain radioactive irradiation, or lose their replication function in other ways. The cells may also be genetically modified to secrete colony stimulating factors.

In another embodiment, the method for treating or preventing abnormal cell proliferation may comprise administering the compound and a chimeric antigen receptor CAR-T cell therapy in combination or alternately to a patient.

In another embodiment, the method for treating or preventing abnormal cell proliferation may comprise administering the compound and another anti-cancer drug in combination or alternately to a patient so as to treat the abnormal cell proliferation. This anti-cancer drug may be an alkylating agent, an antimetabolic drug, an anthracycline derivative, a plant alkaloid, a topoisomerase inhibitor, anti-tumor antibiotics, a kinase inhibitor, or a monoclonal antibody against tumor antigens.

The compound of the present application has good pharmacokinetic properties. For example, the compound of the present application has higher exposure and bioavailability. In some embodiments, the compound of the present application has higher in vivo exposure and higher bioavailability for oral administration. For example, the blood $T_{1/2}$ of the compound of the present application is about 0.1 h to about 10 h, for example, about 0.2 h to about 10 h, about 0.3 h to about 10 h, about 0.4 h to about 10 h, about 0.5 h to about 10 h, about 0.5 h to about 9 h, about 0.5 h to about 8 h, about 0.5 h to about 7 h, about 0.5 h to about 6 h, about 0.5 h to about 5 h, about 0.5 h to about 3 h, about 0.5 h to about 2 h, and about 0.5 h to about 1 h.

**Pharmaceutical Composition**

During administration, the compound of the present application may be prepared into an appropriate pharmaceutical composition. The specific composition of the pharmaceutical composition is determined by the selected route of administration, which comprises oral, parenteral, intravenous, intramuscular, nasal, oral, topical, transdermal, or subcutaneous administration. During the treatment of diseases (such as Parkinson’s disease or tumors), the dose of the compound of the application contained in the pharmaceutical composition should be sufficient to exert an effective therapeutic effect, without leading to serious toxic and side effects to the host. The drug may be administrated daily or every few days, lasting for several days, several weeks, several months, or even several years. A specific dose may be administrated multiple times at intervals, for example, once a day, twice a day or more, once a week, once every two weeks, once every three weeks, once a month, or once every two or more months. For example, the drug may be administered once a day or twice a day.

The compound may be administered at intervals before or after other anti-tumor treatment methods. The time of interval may be 1 minute, 2 minutes, 5 minutes, 10 minutes, 20 minutes, 30 minutes, 45 minutes, 1 hour, 2 hours, 3 hours, 4 hours, 5 hours, 6 hours, 12 hours, 18. hours, 1 day, 2 days, 3 days, 1 week, 2 weeks, 3 weeks, 1 month, 2 months, 3 months or longer. In some embodiments, the compound of the present application may be administered by the same route of administration as other anti-tumor treatment methods or by a different route of administration.

The compound may be administered at intervals before or after other drugs for treating Parkinson’s disease. The time of interval may be 1 minute, 2 minutes, 5 minutes, 10 minutes, 20 minutes, 30 minutes, 45 minutes, 1 hour, 2 hours, 3 hours, 4 hours, 5 hours, 6 hours, 12 hours, 18. hours, 1 day, 2 days, 3 days, 1 week, 2 weeks, 3 weeks, 1 month, 2 months, 3 months or longer. In some embodiments, the compound of the present application may be administered by the same route of administration as other drugs for treating Parkinson’s disease or by a different route of administration.

In some embodiments, the compound may be administered orally. The ingredients of an oral drug generally comprise an inert diluent or an edible carrier. The drug may be encapsulated in gelatin capsules or compressed into tablets. Tablets, pills, capsules, lozenges and other dosage forms may contain the following ingredients: binders such as microcrystalline cellulose, gum tragacanth or gelatin; accessories such as starch or lactose; disintegrants such as alginic acid or corn starch; lubricants such as magnesium stearate; glidants such as colloidal silica; sweeteners such as sucrose or saccharin; flavoring agents such as peppermint, methyl salicylate or orange flavoring agents; wetting agents or emulsifiers; preservatives; and pH buffering agents such as phosphate buffer, sodium acetate or sorbitan

monolaurate. When the pharmaceutical composition is placed in a capsule, a liquid carrier such as fatty oil may also be placed into the same. In addition, the ingredients of the drug may further comprise various other materials, for example, sugar coating, shellac or other enteric agents.

The compound of the present application may further be administered as components of elixirs, suspensions, syrups, cakes, or chewing gum, etc. Besides triazolotriazine derivatives, the syrup may further contain sweeteners such as sucrose, preservatives, coloring agents and flavoring agents. Pharmaceutical solutions or suspensions for oral, parenteral, intradermal, subcutaneous or topical administration may comprise: sterile diluents such as water, saline solutions, Ringer's solution, fixed oil, polyethylene glycol (such as PEG400), glycerin, propylene glycol; fatty acids such as oleic acids and derivatives thereof, polar solvents (such as dimethylacetamide (DMAC)) or other synthetic solvents; antibacterial agents such as benzyl alcohol or methyl p-benzene; antioxidants such as ascorbic acid or sodium sulfite, chelating agents such as ethylene diamine tetraacetic acid; buffers such as phosphate buffer, acetic acid, citric acid or phosphate; and tension or osmotic-pressure regulators such as sodium chloride or glucose; stabilizers such as cyclodextrin; surfactants such as hydroxystearate; etc. A stock solution of the drug may be filled into ampoules, disposable syringes, or multi-dose vials made of glass or plastic.

To prevent the drug from being cleared rapidly from the body, the compound of the present application may further be placed in a carrier capable of exerting such a protective effect. Various other methods capable of controlling the release rate of drug may further be applied, for example, a method using an implant and a microcapsule delivery system.

The compound of the present application may further be administered by using a sprayer, a dry-powder inhaler, or a metered spray inhaler or other modes of administration. The drug administered in such a way may be prepared into a solution with saline, and benzyl alcohol or other suitable preservatives, absorption enhancers, fluorocarbons and other commonly used solubilizing or dispersing agents may be added therein.

Generally speaking, for a patient, a therapeutic regimen and a dose to be used would depend on many factors, comprising the properties of a specific compound used, the age, weight, general health and gender of a patient, diet, administration time, excretion rate, the pathological condition of the patient, therapeutic goal and doctor's judgment. In the case of combined treatment, the amount of active ingredients would also depend on which drug is used for the combined treatment.

In another aspect, the present application provides a drug combination and/or kit, which comprises (1) the compound of the present application, and (2) an immunotherapy for treating tumors.

In another aspect, the present application provides a drug combination and/or kit, which comprises (1) the compound, and (2) a drug for treating Parkinson's disease.

Not wishing to be bound by any particular theory, the following examples are merely to illustrate the compounds, preparation methods, uses, etc. according to the present application, and are not intended to limit the scope of the present invention.

## Examples

In the examples of the present application, the compounds and their names are correspondingly listed in Table 4 below:

Table 4 Compounds in examples of the present application

| Compound No. | Designation | Structural formula |
|---|---|---|
| Compound 1 | N-(4-(2-((7-amino-2-(furan-2-yl)-[1,2,4]triazolo[1,5-a][1,3,5]triazin-5-yl)amino)ethyl)phenyl)-3-morpholinepropanamide; | 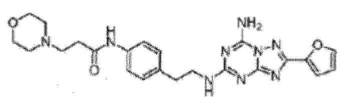 |
| Compound 2 | N-(4-(2-((7-amino-2-(furan-2-yl)-[1,2,4]triazolo[1,5-a][1,3,5]triazin-5-yl)amino)ethyl)phenyl)-2-morpholine propanamide; | 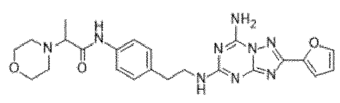 |
| Compound 3 | N-(4-(2-((7-amino-2-(furan-2-yl)-[1,2,4]triazolo[1,5-a][1,3,5]triazin-5-yl)amino)ethyl)phenyl)pivalamide; | 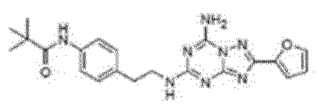 |

(continued)

| Compound No. | Designation | Structural formula |
|---|---|---|
| Compound 4 | N-(4-(2-((7-amino-2-(furan-2-yl)-[1,2,4]triazolo[1,5-a][1,3,5]triazin-5-yl)amino)ethyl)phenyl)-3 -hydroxy- 2,2-dimethylpropionamide; | 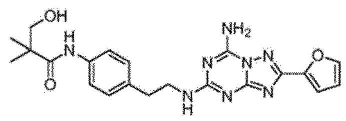 |
| Compound 5 | N-(4-(2-((7-amino-2-(furan-2-yl)-[1,2,4]triazolo[1,5-a][1,3,5]triazin-5-yl)amino)ethyl)phenyl)-2-hydroxy-2-methylpropionamide; | 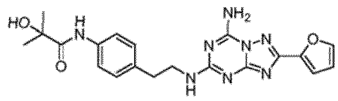 |
| Compound 6 | N-(4-(2-((7-amino-2-(furan-2-yl)-[1,2,4]triazolo[1,5-a][1,3,5]triazin-5-yl)amino)ethyl)phenyl)-4-methyltetrahydro-2H-pyran-4-carboxamide; | 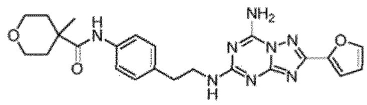 |
| Compound 7 | 2-(furan-2-yl)-N5-(2-(pyrimidin-4-yl)ethyl)-[1,2,4]triazolo[1,5-a][1,3,5]triazine-5,7-diamine; | 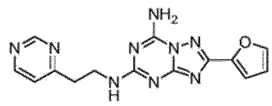 |
| Compound 8 | 2-(furan-2-yl)-N5-(2-(pyridin-3-yl)ethyl)-[1,2,4]triazolo[1,5-a][1,3,5]triazine-5,7-diamine; | 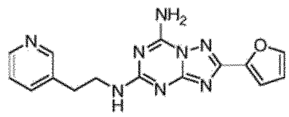 |
| Compound 9 | 2-(furan-2-yl)-N5-(4-(2-(pyrrolidin-1-yl)ethoxy)phenethyl)-[1,2,4]triazolo[1,5- a][1,3,5]-triazine-5,7-diamine; | 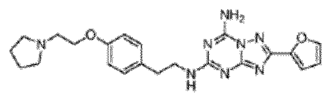 |
| Compound 10 | N5-(4-(2-(azetidine-1-yl)ethoxy)phenethy1)-2-(furan-2-yl)-[1,2,4]triazolo[1,5-a][1,3,5]triazine-5,7-diamine; | 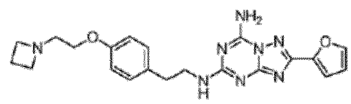 |
| Compound 11 | 2-(furan-2-yl)-N5-(2-(6-(2-methyl-2H-tetrazol-5-yl)pyridin-3-yl)ethyl)-[1,2,4]triazolo[1,5-a][1,3,5]triazine-5,7-diamine | 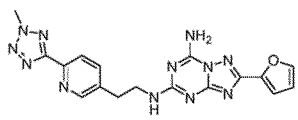 |
| Compound 12 | 2-(furan-2-yl)-N5-(4-(2-(4-methylpiperazin-1-yl)ethoxy)phenethyl)-[1,2,4]triazolo[1,5-a][1,3,5]triazine-5,7-diamine; | 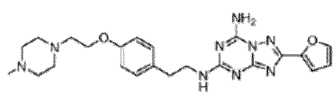 |
| Compound 13 | 2-(furan-2-yl)-N5-(4-(3-(4-methylpiperazin-1-yl)propoxy)phenethyl)-[1,2,4]triazolo[1,5-a][1,3,5]triazine-5,7-diamine; | 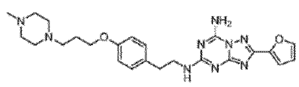 |
| Compound 14 | N5 -(4-(2-(3,3 -difluoropyrrolidin-1-yl)ethoxy)phenethyl)-2-(furan-2-yl)-[1,2,4]triazolo[1,5-a][1,3,5]triazine-5,7-diamine; | 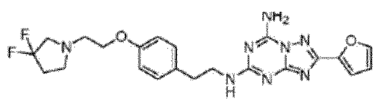 |
| Compound 15 | (R)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine; | 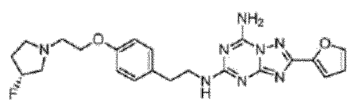 |
| Compound 16 | (S)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine; | 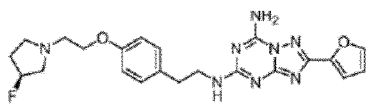 |

(continued)

| Compound No. | Designation | Structural formula |
|---|---|---|
| Compound 17 | 2-(furan-2-yl)-N5-(4-((2-morpholinoethyl)amino) phenethyl)-[1,2,4]triazolo[1,5-a][1,3,5]triazine-5,7-diamine; | 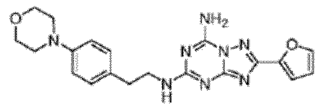 |
| Compound 18 | 2-(furan-2-yl)-N5-(4-((tetrahydro-2H-pyran-4-yl)amino) phenethyl)-[1,2,4]triazolo[1,5-a][1,3,5]triazine-5,7-diamine; | 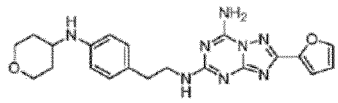 |
| Compound 19 | 2-(furan-2-yl)-N5-(4-((tetrahydrofuran-3-yl)amino) phenethyl)-[1,2,4]triazolo[1,5-a][1,3,5]triazine-5,7-diamine; | 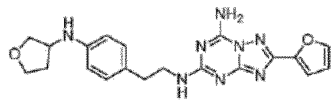 |
| Compound 20 | 2-(furan-2-yl)-N5-(4-(4-methylpiperazin-1-yl) phenethyl)-[1,2,4]triazolo[1,5-a][1,3,5]triazine-5,7-diamine; | 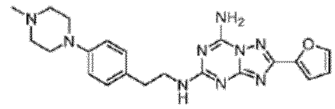 |
| Compound 21 | N5-(4-(bis(2-methoxyethyl)amino)phenethyl)-2-(furan-2-yl)-[1,2,4]triazolo[1,5-a][1,3,5]triazine-5,7-diamine; | 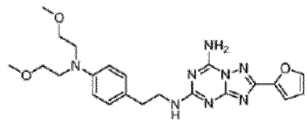 |
| Compound 22 | 2-(furan-2-yl)-N5-(4-(oxetan-3-ylamino)phenethyl)-[1,2,4] triazolo[1,5-a][1,3,5]triazine-5,7-diamine; | 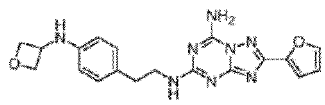 |
| Compound 23 | 2-(furan-2-yl)-N5-(4-(oxetan-3-ylmethyl)phenethyl)-[1,2,4] triazolo[1,5-a][1,3,5]triazine-5,7-diamine; | 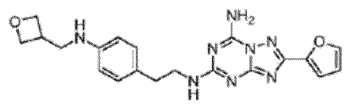 |
| Compound 24 | 2-(furan-2-yl)-N5-(4-((1-methoxypropane-2-yl)amino) phenethyl)-[1,2,4]triazolo[1,5 - a][1,3,5]triazine-5,7-diamine; | 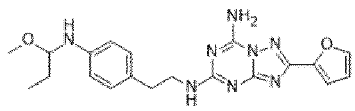 |
| Compound 25 | 2-(furan-2-yl)-N5-(4-((2-methoxyethyl)(methyl)amino) phenethyl)-[1,2,4]triazolo[1,5-a][1,3,5]triazine-5,7-diamine; | 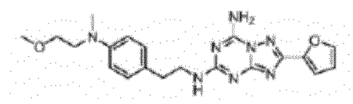 |
| Compound 26 | N5-(4-(ethyl(2-methoxyethyl)amino)phenethyl)-2-(furan-2-yl)-[1,2,4]triazolo[1,5-a][1,3,5]triazine-5,7-diamine; | 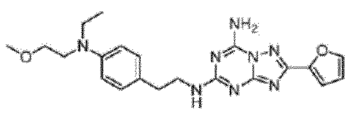 |
| Compound 27 | 2-(furan-2-yl)-N5-(4-((2-methoxyethyl)amino) phenethyl)-[1,2,4]triazolo[1,5-a][1,3,5]triazine-5,7-diamine; | 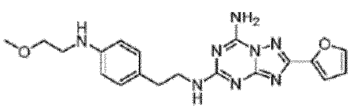 |
| Compound 28 | 2-(furan-2-yl)-N5-(4-((2-(2-methoxyethoxy)ethyl)amino) phenethyl)-[1,2,4]triazolo[1,5-a][1,3,5]triazine-5,7-diamine; | 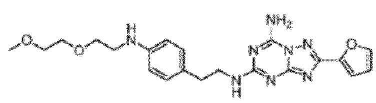 |

(continued)

| Compound No. | Designation | Structural formula |
|---|---|---|
| Compound 29 | 2-(furan-2-yl)-N5-(4-((2-(2-(2-methoxyethoxy)ethoxy) ethyl)amino)phenethyl)-[1,2,4]triazolo[1,5-a][1,3,5] triazine-5,7-diamine; | 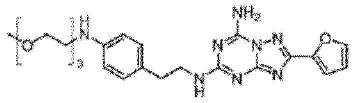 |
| Compound 30 | N5-(4-((2-ethoxyethyl)amino)phenethyl)-2-(furan-2-yl)-[1,2,4]triazolo[1,5-a][1,3,5]triazine-5,7-diamine; | 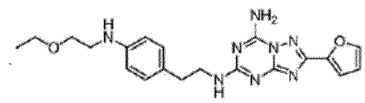 |
| Compound 31 | 2-(furan-2-yl)-N5-(4-((2-(2,2,2-trifluoroethoxy)ethyl)amino) phenethyl)-[1,2,4]triazolo[1,5-a][1,3,5]triazine-5,7-diamine; | 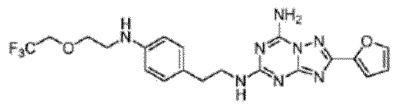 |
| Compound 32 | 2-(furan-2-yl)-N5-(4-((3-methoxypropyl)amino) phenethyl)-[1,2,4]triazolo[1,5-a][1,3,5]triazine-5,7-diamine; | 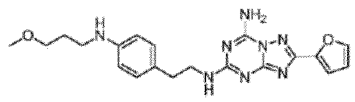 |
| Compound 56 | N5-(4-(2-methyl-2H-tetrazol-5-yl)phenethyl)-2-phenyl-[1,2,4]triazolo[1,5-a][1,3,5]triazine-5,7-diamine | 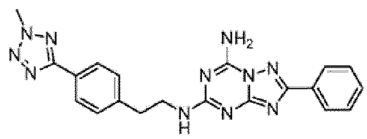 |
| Compound 34 | 2-((4-(2-((7-amino-2-(furan-2-yl)-[1,2,4]triazolo[1,5-a] [1,3,5]triazine-5-yl)amino)ethyl)-phenyl)amino)acetonitrile; | 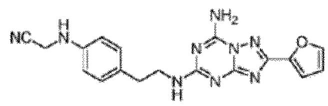 |
| Compound 35 | N5-(4-(1,3-dimethoxypropane-2-ylamino)phenethyl)-2-(furan-2-yl)-[1,2,4]triazolo[1,5-a][1,3,5]triazine-5,7-diamine; | 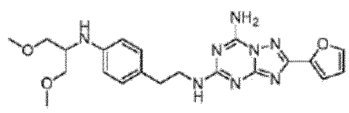 |
| Compound 36 | 2-(3-fluorophenyl)-N5-(4-((2-methoxyethyl)amino) phenethyl)-[1,2,4]triazolo[1,5 -<br><br>a][1,3,5]triazine-5,7-diamine | 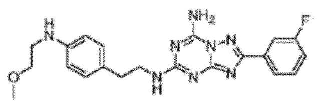 |
| Compound 37 | 2-(3-fluorophenyl)-N5-(4-(oxetan-3-ylamino) phenethyl)-[1,2,4]triazolo[1,5-a][1,3,5]triazine-5,7-diamine | 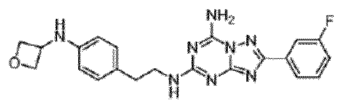 |
| Compound 38 | 4-(2-(7-amino-2-(furan-2-yl)-[1,2,4]triazolo[1,5-a][1,3,5] triazine-5-ylamino)ethyl)-N,N-dimethylbenzamide; | 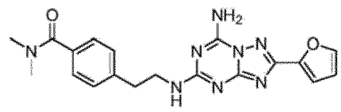 |
| Compound 39 | N5-(4-(1-methyl-1H-tetrazol-5 -yl)phenethyl)-2-(furan-2-yl)-[1,2,4]triazolo[1,5-a][1,3,5]triazine-5,7-diamine; | 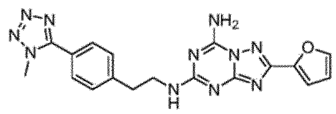 |
| Compound 40 | N5-(4-(2-methyl-2H-tetrazol-5-yl)phenethyl)-2-(furan-2-yl)-[1,2,4]triazolo[1,5-a][1,3,5]triazine-5,7-diamine; | 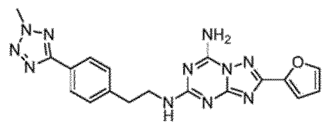 |

(continued)

| Compound No. | Designation | Structural formula |
|---|---|---|
| Compound 41 | N5-(4-(2-ethyl-2H-tetrazol-5-yl)phenethyl)-2-(furan-2-yl)-[1,2,4]triazolo[1,5-a][1,3,5]triazine-5,7-diamine; | 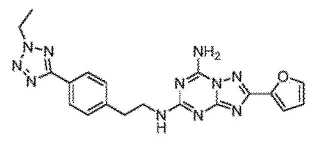 |
| Compound 42 | N5-(4-(2-isopropyl-2H-tetrazol-5-yl)phenethyl)-2-(furan-2-yl)-[1,2,4]triazolo[1,5-a][1,3,5]triazine-5,7-diamine; | 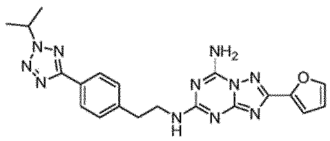 |
| Compound 43 | N5-(4-(2-propyl-2H-tetrazol-5-yl)phenethyl)-2-(furan-2-yl)-[1,2,4]triazolo[1,5-a][1,3,5]triazine-5,7-diamine; | 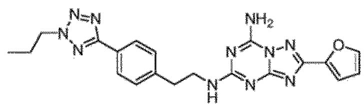 |
| Compound 44 | 2-(5-(4-(2-(7-amino-2-(furan-2-yl)-[1,2,4]triazolo[1,5-a][1,3,5]triazine-5-ylamino)ethyl)-phenyl)-2H-tetrazol- 2-yl) acetonitrile; | 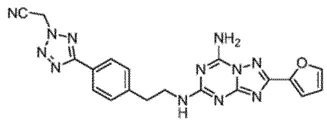 |
| Compound 45 | 4-(2-(7-amino-2-(furan-2-yl)-[1,2,4]triazolo[1,5-a][1,3,5] triazine-5-ylamino)ethyl)-benzonitrile; | 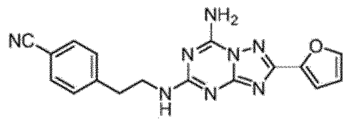 |
| Compound 46 | N5-(4-(2-(2,2,2-trifluoroethyl)-2H-tetrazol-5-yl)phenethyl)-2-(furan-2-yl)-[1,2,4]triazolo[1,5-a][1,3,5]triazine-5,7-diamine; | 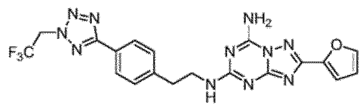 |
| Compound 47 | N5-(4-(2-(2-methoxyethyl)-2H-tetrazol-5-yl)phenethyl)-2-(furan-2-yl)-[1,2,4]triazolo[1,5-a][1,3,5]triazine-5,7-diamine; | 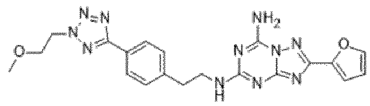 |
| Compound 48 | 2-(furan-2-yl)-N5-(4-(pyridin-2-yl)phenethyl)-[1,2,4]triazolo [1,5-a][1,3,5]triazine-5,7-diamine; | 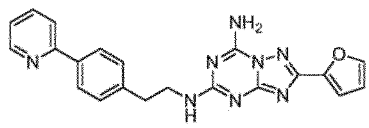 |
| Compound 49 | 2-(furan-2-yl)-N5-(4-(pyridin-3-yl)phenethyl)-[1,2,4]triazolo [1,5-a][1,3,5]triazine-5,7-diamine; | 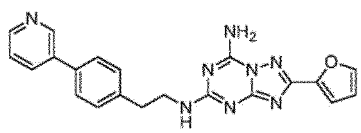 |
| Compound 50 | 2-(furan-2-yl)-N5-(4-(pyridin-4-yl)phenethyl)-[1,2,4]triazolo [1,5-a][1,3,5]triazine-5,7-diamine; | 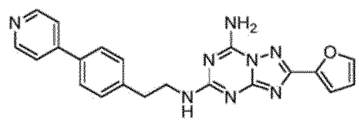 |
| Compound 51 | 2-(furan-2-yl)-N5-(4-(pyrimidin-2-yl)phenethyl)-[1,2,4] triazolo[1,5-a][1,3,5]triazine-5,7-diamine; | 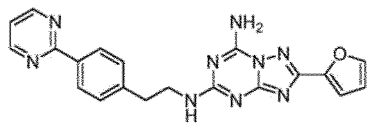 |

(continued)

| Compound No. | Designation | Structural formula |
|---|---|---|
| Compound 52 | 2-(furan-2-yl)-N5-(4-(1-methyl-1H-1,2,4-triazol-3-yl) phenethyl)-[1,2,4]triazolo[1,5-a][1,3,5]triazine-5,7-diamine; | 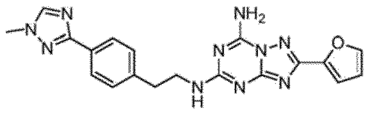 |
| Compound 53 | N5-(4-(2H-tetrazol-5-yl)phenethyl)-2-(furan-2-yl)-[1,2,4] triazolo[1,5-a][1,3,5]triazine-5,7-diamine; | 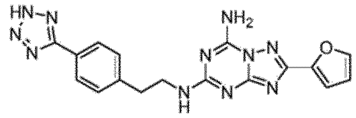 |
| Compound 54 | 2-(furan-2-yl)-N-(4-(methylsulfonyl)phenethyl)-[1,2,4] triazolo[1,5-a][1,3,5]triazine-5-amine; | 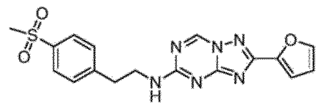 |
| Compound 55 | 2-(3-fluorophenyl)-N5-(4-(2-methyl-2H-tetrazol-5-yl) phenethyl)-[1,2,4]triazolo[1,5-a][1,3,5]triazine-5,7-diamine; | 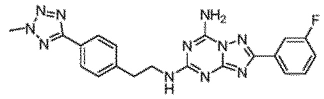 |

**Example 1 Preparation, analysis and characterization of 2-(furan-2-yl)-N5-(4-(oxetane-3-ylamino)phenethyl)-[1,2,4]triazolo[1,5-a][1,3,5]triazine-5,7-diamine acetate (acetate of Compound 22)**

A. 2-(furan-2-yl)-$N^5$-(4-(oxetane-3-ylamino)phenethyl)-[1,2,4]triazolo[1,5-a][1,3,5]triazine-5,7-diamine acetate is prepared as follows:

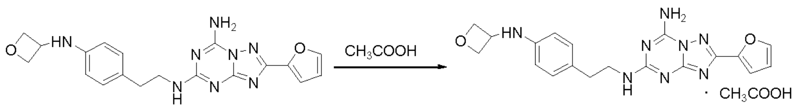


500 mg of free base (A05746-093A15) was dispersed in 7.5 mL of ethyl acetate. 115 mg of acetic acid was dissolved in 2.5 mL of ethyl acetate to obtain an acetic acid solution. The acetic acid solution was slowly added dropwise, over stirring, to the suspension of free base. The suspension of free base was stirred overnight and filtered to obtain solids, which were then dried overnight at 40°C under vacuum. The resulting product was subjected to a characterization test.

Following the same steps, target solids were obtained by the same operation with methyl tert-butyl ether (MTBE), acetone, methyl acetate (MAC), and isopropanol (IPA), and then subjected to characterization tests.

B. Analysis method

1) X-ray powder diffraction (XRPD)

Solid samples were analyzed by Empyrean X-ray powder diffractometer (PANalytical) or D8 Advance X-ray powder diffraction analyzer (Bruker). The Empyrean X-ray powder diffractometer was equipped with a $PIXcel^{1D}$ detector. During XRPD analysis, the 2θ scan angle ranged from 3° to 40°, and the scan step was 0.013°. The light-tube voltage and the light-tube current were 45 KV and 40 mA, respectively. The D8 Advance X-ray powder diffraction analyzer was equipped with a LynxEye detector. The 2θ scan angle ranged from 3° to 40°, and the scan step was 0.02°. The light-tube voltage and the light-tube current were 40 KV and 40 mA, respectively.

2) Differential scanning calorimetry (DSC)

The instrument model for differential scanning calorimetry was DSC 250 (TA Instruments, US). The sample was accurately weighed and then placed in a DSC perforated sample pan, and the accurate mass of the sample was recorded. The sample was heated from 25°C to a final temperature at a temperature rise rate of 10°C/min.

3) Thermogravimetric analysis (TGA)

The model of a thermogravimetric analyzer was TGA 55 (TA Instruments, US). A sample was placed in a balanced open aluminum sample pan, and its mass was automatically weighed in a TGA heating furnace. The sample was heated to a final temperature at a rate of 10°C/min.

4) Dynamic vapor sorption (DVS)

The instrument model used for dynamic moisture sorption and desorption analysis was Vsorp dynamic moisture sorption analyzer (ProUmid GmbH & Co. KG, Germany) or DVS Intrinsic (SMS, UK). The sample was placed in the sample pan with the tare weight deducted, and then automatically weighed. The parameters of the Vsorp dynamic moisture sorption analyzer were set as follows:

| Item | Parameter |
|---|---|
| Sample temperature | 25 °C |
| Cycle time | 10 min |
| Minimum time per cycle | 50 min |
| Maximum time per cycle | 120 min |
| Weight limit | 100% |
| Balance condition | 0.01%/45 min |
| Cycle #1 | Humidity: 0% to 0%, temperature: 40°C, 3 hours |
| Cycle #2 | The temperature was 25°C, and the humidity was increased from 0% to 90%. |
| Cycle #3 | The temperature was 25°C, and the humidity returned to 0% from 80%. |
| Sorption | 0, 10, 20, 30, 40, 50, 60, 70, 80, 90 |
| Desorption | 80, 70, 60, 50, 40, 30, 20, 10, 0 |

The parameters of the DVS Intrinsic instrument were set as follows:

| Item | Parameter |
|---|---|
| Step time | 60 min |
| Sample temperature | 25 °C |
| Cycle | Full cycle |
| Sorption | 0, 10, 20, 30, 40, 50, 60, 70, 80, 90 |
| Desorption | 80, 70, 60, 50, 40, 30, 20, 10, 0 |
| Data saving rate | 5 S |
| Total flow | 200 sccm |
| Post-test total flow | 200 sccm |

5) Polarized light microscopy (PLM)

The instrument model used for PLM analysis was ECLIPSE LV100POL polarized light microscope (Nikon, Japan).

6) Nuclear magnetic resonance ($^1$H-NMR)

For $^1$H-NMR, Bruker AVANCE III HD 300 or 400 equipped with a Sample Xpress 60 autosampler, or Bruker Advance 300 equipped with a B-ACS 120 autosampler was used.

7) High-performance liquid chromatography (HPLC)

An Agilent HPLC 1260 series instrument was used in an HPLC analysis method. The HPLC method for testing solubility and stability was as follows:

| | |
|---|---|
| Column | Atlantis T3, 4.6*150 mm, 3 μm |
| Mobile phase | A: 10 mmol aqueous solution of ammonium acetate<br>B: Acetonitrile |
| Gradient (T/B%) | 0/5%, 12/5%, 13/20%, 40/40%, 45/50%, 45.1/95%, 50/95% |
| Column temperature | 25 °C |
| Detection wavelength | DAD; 250 nm |
| Flow rate | 1.0 mL/min |
| Injection volume | 5 μL |
| Run time | 55 minutes |
| Post-run time | 0 |
| Diluent | DMSO |

C. Data and conclusions

From the XRPD results (FIG. 1), it can be seen that acetates are obtained in all solvents, with a crystal form different from that of the free base. The product obtained by the reaction in MTBE still contains a small amount of free-base crystal forms. The acetates obtained in different solvents have the same crystal form.

From the $^1$H-NMR spectrum (FIG. 2) in combination with the XRPD spectrum (FIG. 1), it can be seen that all of the acetone, ethyl acetate, methyl acetate and isopropanol can be turned into acetates.

The results of DSC and TGA (FIG. 3) indicate that the sample has a weight loss of about 13.4% before 210°C, which is presumed to be caused by the removal of acetic acid. Abroad endothermic peak is observed at the initial temperature of 148.9°C, which is caused by the removal of acetic acid. The endothermic peak at the initial temperature of 226.0°C is presumed to be caused by the dissolution of the anhydrous crystal form of the free base.

4) Solubility

In the experiment, the solubilities of the free-base crystal form and the acetate crystal form were determined at 37°C in biological vehicles (simulated gastric fluid (SGF), fasted state stimulated intestinal fluid (FaSSIF) and fed state stimulated intestinal fluid (FeSSIF)). 3 mg of a sample was added to a sample bottle, and 1 mL of biologically relevant vehicle was added to the same. The suspension was shaken at a speed of 500 rpm at 37°C. After 0.5 h and 2 h, 500 μL of the suspension was taken and filtered respectively, and the filtrates were tested by HPLC to obtain the solubility.

Table 5 Solubility of Compound 22 in the forms of free base and acetate

| Sample | Vehicle | Solubility | |
|---|---|---|---|
| | | 0.5 h | 2 h |
| Free base | SGF | 0.6223 | 0.7121 |
| | FaSSIF | 0.0037 | 0.0042 |
| | FeSSIF | 0.0070 | 0.0088 |
| Acetate | SGF | >2.887 | >2.887 |
| | FaSSIF | 0.0093 | 0.0099 |
| | FeSSIF | 0.0331 | 0.0338 |

The results in Table 5 show that the solubility of Compound 22 in the forms of free base and acetate was strongly related to the pH of the vehicle. They show maximum solubility in SGF, with the solubility of acetate being more

than 2.887 mg/mL and the solubility of free base being 0.712 mg/mL. The solubilities of acetate in three biologically relevant vehicles were greater than those of the free base.

5) Stability results and conclusions

A certain amount of acetate crystal was held at 60°C in a closed condition and at 40°C/75% RH in an open condition for a maximum of 7 days. Samples were taken on days 0, 3, and 7 and diluted with a diluent to about 0.3 mg/mL for HPLC purity testing. The solid samples were tested by XRPD to determine the crystal form.

Table 6 XRPD results of acetate of Compound 22

| Sample | Initial purity (area%) | Purity over 7 days (area%) | | XPRD | |
|---|---|---|---|---|---|
| | | 40°C/75%RH | 60°C | 40oC/75%RH | 60°C |
| Acetate | 98.79 | 98.99 | 99.01 | Transformed into free-base crystal form | Unchanged |

The results in Table 6 show that no significant degradation is observed for the acetate of Compound 22 under test conditions. The acetate crystal form keeps physically and chemically stable at 60°C. Under the condition of 40°C/75% RH, part of the acetate crystal form will be transformed into the free-base crystal form.

**Example 2 Preparation of $N^5$-(4-(2-methyl-2H-tetrazol-5-yl)phenethyl)-2-(furan-2-yl)-[1,2,4]triazolo[1,5-a][1,3,5] triazine-5,7-diamine benzene sulfonate (benzene sulfonate of compound 40)**

A reaction formula for preparation was as follows:

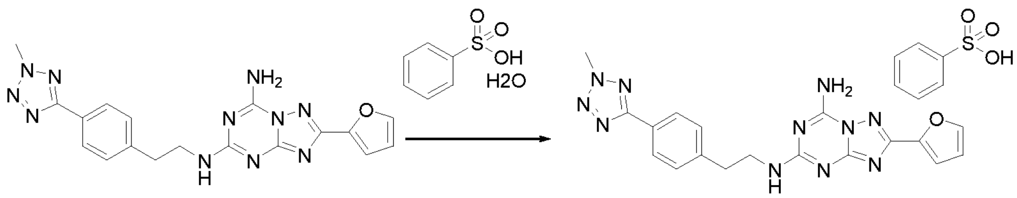


Benzenesulfonic acid hydrate (4g, 25.4mol) was added to methanol (18 mL). The solid of $N^5$-(4-(2-methyl-2H-tetrazol-5-yl)phenethyl)-2-(furan-2-yl)-[1,2,4]triazolo[1,5-a][1,3,5]triazine-5,7-diamine (2.56g, 6.35 mol) was added portionwise at room temperature, which was dissolved during the portionwise addition. After the addition was completed, a small amount of solids were found to be undissolved, and then were all dissolved after sonication. After stirring for 5 minutes, a large amount of white solids precipitate out. 50 mL ethyl acetate was added to make a slurry, and the slurry was filtered and dried to obtain the target compound as a white solid (2.0 g, yield: 56%). $^1$H NMR (500 MHz, DMSO-$d_6$) $\delta$ 9.04 - 8.71 (m, 1H), 8.25 (d, *J* = 0.7 Hz, 2H), 8.02 - 7.96 (m, 3H), 7.61 (dd, *J* = 7.7, 1.8 Hz, 2H), 7.46 (t, *J* = 9.7 Hz. 2H), 7.39 - 7.26 (m, 3H), 7.19 (d, *J* = 2.8 Hz, 1H), 6.75 (s, 1H), 4.42 (s, 3H), 3.65 - 3.53 (m, 2H), 2.96 (t, 2H).

**Example 3 Inhibition ability of present compounds against activity of adenosine receptors A2A** 1. Experimental method

The inhibitory effects of the test compounds on the adenosine receptors A2A were evaluated by TR-FRET-based cAMP accumulation assay. The method included, in HEK293 cells (hADORA2A-HEK293) expressing A2AR, the production of cAMP stimulated by NECA and the inhibitory effect of A2A receptor antagonists on cAMP. All cells were cultured in a complete medium at 37°C in the presence of 5% $CO_2$. After being digested with trypsin, the cells were collected by centrifugation at 200 g for 5 minutes. The cells were resuspended in a fresh complete medium, and then the cell viability was counted by a trypan blue exclusion method. The subsequent cAMP production measurement experiment could be performed only when the cell viability was above 95%. The cells were diluted with Hank's buffer solution containing HEPES (5 mM), BSA stabilizer (0.1%) and Rolipram (10 $\mu$M), then added to a 384-well white non-transparent plate (cells/well, 10 $\mu$l/well), and incubated with the test compound at an appropriate concentration (11 concentrations) for 20 minutes at room temperature. Then, the A2A receptor agonist NECA (final concentration = EC80, which was determined in the same experiment performed earlier) was added to the sample, and the sample was incubated again at 37°C for 30 minutes. Finally, a Eu-cAMP tracer and Ulight-anti-cAMP were added, and the yield of cAMP was

determined by determining a ratio of TR-FRET at 665 nm to fluorescence emission at 615 nm. ZM241385 was selected as a reference compound and istradefylline was selected as a positive control compound. Istradefylline was a selective A2A receptor antagonist, which could improve the motor function of a PD patient by changing the activity of neurons. It was clinically used to treat PD and improve the dyskinesia associated with PD.

2. Calculation

An inhibition rate (%) was calculated according to the following formula, and an IC50 value was calculated from a concentration-inhibition (%) curve after log conversion.

$$\%Inhibition = \left(1 - \frac{Ratio_{665nm/615nm\ high} - Ratio_{665nm/615nm\ cmpd}}{Ratio_{665nm/615nm\ high} - Ratio_{665nm/615nm\ low}}\right) \times 100\%$$

%Inhibitor: %inhibition rate;
$Ratio_{665/615\ high}$: the high fluorescence ratio of the reference compound;
$Ratio_{665/615\ low}$: the low fluorescence ratio of the reference compound;
$Ratio_{665/615\ cmpd}$: the fluorescence ratio of the test compound;
A curve corresponding to the dose was fitted by using XLfit software and the $IC_{50}$ was determined.

3. Results and conclusions

Table 7 Inhibition of present compounds against activity of adenosine receptors A2A

| Compound No. | A2AR activity ($IC_{50}$, nM) |
|---|---|
| Compound 6 | 9.24 |
| Compound 17 | 3.01 |
| Compound 18 | 3.83 |
| Compound 21 | 1.62 |
| Compound 22 | 7.07 |
| Compound 27 | 10.57 |
| Compound 28 | 6.77 |
| Compound 30 | 3.52 |
| Compound 35 | 4.88 |
| Compound 37 | 7.07 |
| Compound 38 | 8.71 |
| Compound 40 | 9.66 |
| Compound 41 | 7.87 |
| Compound 42 | 9.08 |
| Compound 47 | 13.88 |
| ZM241385 | 9.00 |
| Istradefylline | 28.73 |

From the data in the above table, it can be seen that all the compounds of the present application in the list exhibit excellent A2A receptor antagonism, with $IC_{50}$ at a relatively low level, about 10 nM. These inhibitory activities against the A2A receptors were higher than that of istradefylline as the positive control compound.

**Example 4 Selective inhibition of present compounds against adenosine receptors**

**4.1 Detection of inhibitory activity of present compounds on other adenosine receptors by cAMP accumulation assay**

The inhibitory effects of test Compound 22 and istradefylline (positive control drug) on another three adenosine receptors A2b, A1 and A3 were evaluated by the TR-FRET-based cAMP accumulation assay.

1. Experimental method

(1) cAMP experiment on Gs-coupled adenosine receptor A2b:

In this experiment, a HEK293 cell line stably expressing the adenosine receptor A2b was taken as a study object (PerkinElmer, ES-013-C) for the experiment. Cells were cultured in an EMEM medium (ATCC, 30-2003) containing 10% FBS (Hyclone, SH30406.05), 100 U/mL penicillin-streptomycin mixed solution (Gibco, 15140-122) and 100 μg/mL G418 (Gibco, 11811031). The cells were digested and passaged approximately 3 times a week with TrypLE™ Express (Gibco, 12604-013), and were maintained at the confluence of approximately 70% to 90%. On the day of the experiment, the cells were inoculated into a 384-well microwell plate (Corning, 3570) at a density of 500 cells per well. A solution system for cell inoculation was 10 μL of cAMP experimental buffer. The cAMP experiment buffer was prepared by adding 75 μL of 1 MHEPES (Gibco, 15630080), 200 μL of 7.5% BSA stabilizer (PerkinElmer, TRF0263) and 7.5 μL of 20 mM rolipram (Sigma, R6520) into 14 mL of 1 ×HBSS (Gibco, 14025-076). A reference agonist NECA (Sigma, E2387) and a reference inhibitor CVT-6883 (Aobious, AOB4675) were prepared at 1 mM (1000×final concentration), and then 3-fold gradient dilution was carried out in 100% DMSO (Millipore, 1029312500). Similarly, 10 mM test compound was also subjected to 3-fold gradient dilution in 100% DMSO. The gradient-diluted reference compound and test compound were added to a 384-well compound plate (Labcyte, PP-0200). 10 nL of the gradient-diluted reference agonist NECA per well was transferred to a test plate containing cells by using Echo (Labcyte, 550), and the test plate was incubated for 30 minutes at 37°C. 5 μL of Eu-cAMP tracer working solution and 5 μL of ULight-anti-cAMP working solution (PerkinElmer, TRF0263) were added to the test plate per well, and the test plate was incubated for 30 minutes at 25°C. The fluorescence signal value (λex = 320 nm, λem = 615 nm and 665 nm) of each well in the test plate was read by using the EnVision microplate reader (PerkinElmer, 2009-0030), and the EC50 and EC80 values were calculated by using the output fluorescence signal ratio (Ratio 665 /615) and concentration of the reference agonist NECA. 1000 × EC80 NECA was prepared with 100% DMSO. 10 nL of the gradient-diluted reference inhibitor CVT-6883 per well and 10 nL of the gradient-diluted test compound per well were transferred to a test plate containing the cells. The test plate was incubated for 20 minutes at 25°C, and then 10 nL of the 1000×EC80 NECA per well was transferred to the test plate. The test plate was incubated for 30 minutes at 37°C. 5 μL of Eu-cAMP tracer working solution and 5 μL of ULight-anti-cAMP working solution were added to the test plate per well, and the test plate was incubated for 30 minutes at 25°C. The fluorescence signal ratio of each well in the test plate was read, and the IC50 value of the compound was calculated by using the output data of the reference inhibitor and test compound and the concentration of the compound.

(2) cAMP experiment on Gi-coupled adenosine receptor A1:

In this experiment, a CHO cell strain stably expressing the adenosine receptor A1 was taken as a study object (PerkinElmer, ES-010-C) for the experiment. Cells were cultured in an F12 medium (Gibco, 11765-062) containing 10% FBS (Hyclone, SH30406.05), 100 U/mL penicillin-streptomycin mixed solution (Gibco, 15140-122) and 400 μg/mL G418 (Gibco, 11811031). The cells were digested and passaged approximately 3 times a week with TrypLE™ Express (Gibco, 12604-013), and were maintained at the confluence of approximately 70% to 90%. On the day of the experiment, the cells were inoculated into a 384-well microwell plate (Corning, 3570) at a density of 2000 cells per well. A solution system for cell inoculation was 10 μL of cAMP experimental buffer. The cAMP experiment buffer was prepared by adding 75 μL of 1 M HEPES (Gibco, 15630080), 200 μL of 7.5% BSA stabilizer (PerkinElmer, TRF0263) and 7.5 μL of 20 mM rolipram (Sigma, R6520) into 14 mL of 1 ×HBSS (Gibco, 14025-076). A reference agonist NECA (Sigma, E2387) and a reference inhibitor DPCPX (Sigma, C101) were prepared at 1 mM (1000×final concentration), and then 3-fold gradient dilution was carried out in 100% DMSO (Millipore, 1029312500). Likewise, 10 mM test compound was also subjected to 3-fold gradient dilution in 100% DMSO. 1 mM forskolin (Sigma, F3917) was prepared with 100% DMSO. The gradient-diluted reference compound and test compound and the 1 mM forskolin were added to a 384-well compound plate (Labcyte, PP-0200). 10 nL of the 1 mM forskolin per well and 10 nL of the gradient-diluted reference agonist NECA per well were transferred to a test plate containing cells by using Echo (Labcyte, 550), and the test plate was incubated for 30 minutes at 37°C. 5 μL of Eu-cAMP tracer working solution and 5 μL of ULight-anti-cAMP working solution (PerkinElmer, TRF0263) were added to the test plate per well, and the test plate was incubated for 30 minutes at 25°C. The fluorescence

signal value ($\lambda$ex = 320 nm, $\lambda$em = 615 nm and 665 nm) of each well in the test plate was read by using the EnVision microplate reader (PerkinElmer, 2009-0030), and the EC50 and EC80 values were calculated by using the output data and concentration of the reference agonist NECA. 1000 $\times$ EC80 NECA was prepared with 100% DMSO. 10 nL of the reference inhibitor DPCPX gradient-diluted per well and 10 nL of the test compound gradient-diluted per well per well were transferred to a test plate containing the cells, and the test plate was incubated for 20 minutes at 25°C. 10 nL of 1000$\times$EC80 NECA per well and 10 nL of 1 mM forskolin per well were transferred to the test plate The test plate was incubated for 30 minutes at 37°C. 5 $\mu$L of Eu-cAMP tracer working solution and 5 $\mu$L of ULight-anti-cAMP working solution were added to the test plate per well, and the test plate was incubated for 30 minutes at 25°C. The fluorescence signal ratio of each well in the test plate was read, and the IC50 value of the compound was calculated by using the output data of the reference inhibitor and test compound and the concentration of the compound.

(3) cAMP experiment on Gi-coupled adenosine receptor A3:

Similarly, a CHO cell strain stably expressing the adenosine receptor A3 was taken as a study object (PerkinElmer, ES-012-C) for the experiment. The IC50 value of the compound was calculated.

2. Data analysis

1) cAMP experiment on Gs-coupled adenosine receptor A2b:

$$\%\,\text{Inhibition} = 100\% \times \frac{Ratio_{\text{Compound}} - Ratio_{Low\,Control}}{Ratio_{High\,Control} - Ratio_{Low\,Control}}$$

%Inhibitor: %inhibition rate;
Ratio $_{\text{High control}}$: the high fluorescence ratio of the reference compound;
Ratio $_{\text{Low Control}}$: the low fluorescence ratio of the reference compound;
Ratio compound: the fluorescence ratio of the test compound;

2) cAMP experiment on Gi-coupled adenosine receptors A1 and A3:

$$\%\,\text{Inhibition} = (1 - \frac{Ratio_{Compound} - Ratio_{Low\,Control}}{Ratio_{High\,Control} - Ratio_{Low\,Control}}) * 100\%$$

%Inhibitor: %inhibition rate;
Ratio $_{\text{High Control}}$: the high fluorescence ratio of the reference compound;
Ratio $_{\text{Low Control}}$: the low fluorescence ratio of the reference compound;
Ratio compound: the fluorescence ratio of the test compound;

3. Experimental results

Table 8 Inhibitory activities of present compounds against adenosine receptors

| | Summary of IC50 (nM) Results | | |
|---|---|---|---|
| | CHO cells (ADORA1) | HEK293 cells (ADORA2B) | CHO cells (ADORA3) |
| Compound 22 | 928.06 | 379.79 | 584.94 |
| Compound 40 | 387.84 | 342.60 | 928.11 |

(continued)

| | Summary of IC50 (nM) Results | | |
|---|---|---|---|
| | CHO cells (ADORA1) | HEK293 cells (ADORA2B) | CHO cells (ADORA3) |
| Compound 27 | 679.02 | 307.38 | 458.34 |
| Istradefylline | 1199.34 | 236.07 | 2075.30 |

In conjunction with the data in Example 3, the $IC_{50}$s for Compounds 22, 27 and 40 to inhibit the activity of the adenosine A2A receptor $ADORA_{2A}$ is about 10 nM, and the $IC_{50}$s for inhibiting the activities of adenosine A2b, A1 and A3 receptors are all greater than 300 nM. The results show that under the experimental conditions used in this experiment, both Compounds 22, 27 and 40 and the istradefylline show a better selective inhibitory activity on the adenosine $ADORA_{2A}$ than on another three adenosine receptors.

**4.2 Determination of selective inhibitory activity of present compounds against adenosine receptors by calcium flow test**

1. Test principle:

In this experiment, stably transfected cell lines over-expressing adenosine receptor coupled to human Gq were used, and the antagonistic function of Compound 22 was evaluated by testing the changes in intracellular calcium ion concentration. The binding of the $A_{2A}R$ agonist to $A_{2A}R$ could activate a downstream pathway to increase the intracellular calcium ion concentration. The antagonist would competitively occupy the binding site where the agonist binds to $A_{2A}R$, thereby interrupting the intracellular signal transduction and leading to a change in the intracellular calcium ion concentration. By detecting the strength of the intracellular calcium ion signal, the antagonistic activities of the compound on human $ADORA_1$, $ADORA_{2A}$, $ADORA_{2B}$ and $ADORA_3$ adenosine receptors were determined.

2. Cell strain information:

The stably transfected cell lines of ADORA1, ADORA2A, ADORA2B and ADORA3 receptors were all constructed by the biology department of Wuxi Apptec Co., Ltd., where receptor plasmids and G15 plasmids were transferred into host cells by means of ThermoFisher Lipofectamine 2000 transfection reagent, and then, single clones were picked to construct stably transfected cell lines. The specific information and culture conditions were as follows:

| Target | Reference sequence | Host cell | Culture condition |
|---|---|---|---|
| ADORA1 | NM_000674 | HEK293 | DMEM+10%FBS; G418 300μg/mL; BS: 2μg/mL |
| ADORA2A | NM_000681 | CHO | F12+10%FBS; G418 300μg/mL; BS: 2μg/mL |
| ADORA2B | NM_000676.2 | HEK293 | DMEM+10%FBS; G418 300μg/mL; BS: 2μg/mL |
| ADORA3 | NM_000677 | CHO | F12+10%FBS; G418 300μg/mL; BS: 2μg/mL |

3. Reagents and consumables:

| Reagent/compound name | Article No. | Manufacturer |
|---|---|---|
| DMEM medium | 11960-051 | Invitrogen |
| F12 medium | 11765-047 | Invitrogen |
| L-glutamate | 25030-081 | Invitrogen |
| Fetal bovine serum | SV30087.03 | Hyclone |
| G418 | 10131-027 | Invitrogen |
| Blasticidin | A11139-03 | Invitrogen |

(continued)

| Reagent/compound name | Article No. | Manufacturer |
|---|---|---|
| Fluo-4 Direct kit | F10471 | Invitrogen |
| 384-well polylysine-coated cell plate | 781946 | Greiner |
| 384-well compound plate | 781280 | Greiner |
| NECA (a reference compound, as an agonist) | E2387 | Sigma |
| CGS-15943 (a reference compound, as an inhibitor) | C199 | Sigma |

4. Instrument and equipment:

| Instrument | Model | Factory |
|---|---|---|
| FLIPR | FT-0249 | Molecular devices |
| Echo | Echo555 | Labcyte |
| Cell counter | AN16097 | Beckman |

5. Test Method:

Preparation of cell plate:

1) A tube of cells was taken from a liquid nitrogen tank and quickly dissolved in a water bath at 37°C.
2) The cell suspension was added to a centrifuge tube containing 20 mL of pre-warmed medium in advance.
3) Centrifugation was carried out at 1000 rpm for 5 minutes at room temperature.
4) The supernatant was discarded slowly to avoid affecting the sedimental cells.
5) The cells were resuspended in 10-30 mL of medium, and gently blown by a pipette.
6) The viability and number of cells were calculated by a cell counter.
7) The cells were diluted to $1.0 \times 10^6$ cells/mL with the medium, seeded at 20 μL/well into a 384-well polylysine-coated cell plate, and incubated with 5% $CO_2$ for 16-20 hours in an incubator at 37°C.

Note: Only the cells with the viability reaching 90% or more can it be used in the test.
Formulation of Fluo4-Direct dye:
Formulation of 250 mM probenecid solution: following the operating instructions of kit, 1 mL of FLIPR buffer salt solution was added to 77 mg of probenecid, and the resultant was prepared for immediate use.
Formulation of 2 × (8mM) Fluo-4Direct™ loading buffer: Fluo-4Direct™ was dissolved in advance in burettes with a required number for experiment; 10 mL of FLIPR buffer salt solution was added to each burette; 0.2 mL of 250 mM probenecid solution was added to each burette; and the burettes were shaken in the dark for vortexing for more than 5 minutes, and the resultant was prepared for immediate use.
Compound preparation and experimental operation:

1) The compound was diluted 30000/900*6=200 folds during the entire process of the experiment. Compound preparation: 4 compounds to be tested were prepared with DMSO into 10 mM stock solution, which was stored in a refrigerator at 4°C.
2) Preparation of reference compound of agonist: the agonist was diluted with Echo at gradient of 1:4; 10 points were set, with duplicate wells at left and right; next, 900 nL of the resultant was transferred to a corresponding compound plate; and then 30 μL of experimental buffer salt solution was added to the corresponding compound plate.
3) Preparation of reference and to-be-tested compound plates: a 10-point dose-response curve was made by diluting the 2 mM (achieved by 5-fold dilution of 10 mM stock solution to 2 mM by using DMSO) compound under test at a gradient of 1:4. For the reference compound CGS-15943, a 10-point dose-response curve was made with Echo by the gradient dilution of 1:4. the initial concentration of CGS-15943 for ADORA1, ADORA2B and ADORA3 receptors was 200 μM, and the initial concentration of CGS-15943 for ADORA2A receptors was 20 μM.
4) 30 μL of FLIPR buffer salt solution was added to the corresponding compound plate for later use.

5) The cell plate prepared the day before from the incubator; 20 μL of 2 × Fluo-4Direct™ buffer was added to each well; and the cell plate was incubated for 50 minutes in the incubator at 37°C in the presence of 5% CO2, and held in the dark for 10 minutes at room temperature.
6) The compound plate, cell plate and pipette tip were placed in the FLIPR instrument.
7) The FLIPR instrument software was operated; following a set program, 10 μL of experimental buffer salt solution was added to the cell plate; and fluorescence signals were read. Then, 10 μL of the reference compound of agonist at a predetermined concentration was added to the cell plate, and the fluorescence signals were read. After reading, data was exported by means of "Max-Min" and "Read 91 to Maximum allowed" in the software, the EC80 of each cell line was calculated, and an agonist with a concentration of 6×EC80 was prepared.
8) The reference compound of agonist with the corresponding cell concentration of 6×EC80 was prepared with the buffer salt solution, and added at 30 μL/well to the corresponding compound plate for later use.
9) The FLIPR instrument software was operated; following a set program, 10 μL of the test compound and 10 μL of the reference compound were added at a predetermined concentration to the cell plate; and the fluorescence signals were read. Then, 10 μL of the reference compound of agonist at a concentration of 6×EC80 was added to the cell plate, and the fluorescence signal were read. For the antagonist detection of the compound, data was exported by means of "Max-Min" and "Read 1 to 90" in the software.
10) Data analysis was carried out by Prism statistical software.

The formula for analyzing the data was as follows:

$$\text{Antagonist \% Inhibition}=100-(\text{RLU}-\text{LC})/(\text{DMSO}-\text{LC})*100$$

RLU: Relative light absorbance value, with a reading value from 1 to 90;
HC: Mean value of fluorescence signals in the DMSO group;
LC: Mean value of fluorescence signals at the highest concentration point of antagonist.

The data was fitted with "log(inhibitor) vs response-variable slope" in GraphPad Prism 5.0 to obtain IC50.

5. Results and conclusions:

Table 9 Inhibitory activities of present compounds against adenosine receptors

| Compound | Results on adenosine receptor | | | |
|---|---|---|---|---|
| | ADORA1 (IC50, nM, n=3) | ADORA2A (IC50, nM, n=3) | ADORA2B (IC50, nM, n=3) | ADORA3 (IC50, nM, n=3) |
| Compound 22 | 218.37±147.67 | 0.35±0.07 | 106.07±29.18 | 930.90±54.13 |
| Compound 40 | 228.27±104.72 | 1.17±0.62 | 194.30±25.50 | 6300.00±2886.45 |
| CGS-15943 | 4.37±2.01 | 0.50±0.03 | 7.35±2.12 | 32.14±4.52 |

The results show that Compound 22 and Compound 40 have stronger inhibitory ability on $ADORA_{2A}$-mediated signal transduction, but weaker inhibitory ability on another three adenosine receptors. That is, they show high selectivity in the inhibition of $ADORA_{2A}$.

**Example 5 Binding ability of present compounds to adenosine receptors**

1. Test principle

In this experiment, the binding effects of the test compound and the reference compound CGS-15943 on the adenosine receptors ADORA1, ADORA2A, ADORA2B, and ADORA3 were detected at a biochemical level by means of the radioisotope ligand competitive binding technology.

2. Reagent/compound and consumable

| Name of reagent consumable | Article No. | Supplier |
|---|---|---|
| Human adenosine ADORA1 receptor | ES-010-M400UA | PerkinElmer |
| membrane protein | | |
| Human adenosine ADORA2A receptor membrane protein | RBHA2AM400UA | PerkinElmer |
| Human adenosine ADORA2B receptor membrane protein | ES-013-M400UA | PerkinElmer |
| Human adenosine ADORA3 receptor membrane protein | ES-012-M400UA | PerkinElmer |
| [3H] DPCPX | NET974001MC | PerkinElmer |
| [3H] CGS-21680 | NET1021250UC | PerkinElmer |
| [3H] HEMADO | ART1456 | ARC |
| CGS-15943 | C199-25MG | Sigma |
| NECA | E2387 | Sigma |
| PEI | P3143 | Sigma |
| Microscint 20 scintillation solution | 6013329 | PerkinElmer |
| Top Seal-A plate sealing film | 6005250 | PerkinElmer |
| 96-well conical polypropylene plate | 5042-1385 | Agilent |
| Unifilter-96 GF/C filter plate | 6005174 | PerkinElmer |

3. Instrument and equipment

| Instrument | Model | Factory |
|---|---|---|
| Harvester | C961961 | PerkinElmer |
| Microbeta2 | 2450 Microplate counter | PerkinElmer |

4. Test Method

1) Preparation of detection buffer:

ADORA1: 25 mM HEPES pH 7.4, 5 mM $MgCl_2$, 1 mM $CaCl_2$, 100 mM NaCl
ADORA2A: 50 mM Tris-HCl pH 7.4, 10 mM $MgCl_2$, 1 mM EDTA
ADORA2B: 50 mM HEPES pH 7.0, 5 mM $MgCl_2$, 1 mM EDTA
ADORA3: 25 mM HEPES pH 7.4, 10 mM $MgCl_2$, 1 mM CaCl2, 0.5% BSA

2) Preparation of washing solution:

ADORA1: 25 mM HEPES pH 7.4, 5 mM $MgCl_2$, 1 mM $CaCl_2$, 100 mM NaCl.
ADORA2A: 50 mM Tris-HCl pH 7.4, 154 mM NaCl
ADORA2B: 50 mM HEPES pH 6.5, 5 mM $MgCl_2$, 1 mM EDTA, 0.2% BSA
ADORA3: 50 mM Tris-HCl pH 7.4

3) Reaction system: 100 μL of cell membrane, 100 μL of isotope, and 1 μL of compound

4) Preparation of compounds: the test compounds and the reference compounds were separately diluted with 100% DMSO. For the test compounds, the initial concentration was 10 μM, with double secondary wells; 4-fold gradient dilution was carried out at 10 points; and the final working concentration was 0.038 nM-10 μM (for the second and third detection of the test compound against an ADORA2A target, the initial concentration was 2 μM, with double secondary wells, 4-fold gradient dilution was carried out at 10 points, and the final working concentration was 0.0076 nM-2 μM). For the ADORA1, ADORA2A, ADORA2B, and ADORA3 reference compounds, the initial concentration was 1 μM, with double secondary wells, 4-fold gradient dilution was carried out at 10 points, and the final working concentration was 0.0038 nM-1 μM.

5) Preparation of cell membrane: the ADORA receptor cell membrane was prepared with the detection buffer into the corresponding concentration.

6) Preparation of isotope: the isotope was prepared with the detection buffer into the corresponding concentration.

| Target | Cell membrane concentration | Isotope | Final isotope concentration |
|---|---|---|---|
| ADORA1 | 2.5 μg/well | [3H] DPCPX | 1 nM |
| ADORA2A | 5 μg/well | [3H] CGS-21680 | 6 nM |
| ADORA2B | 20 μg/well | [3H] DPCPX | 8 nM |
| ADORA3 | 2 μg/well | [3H] HEMADO | 1 nM |

7) Determination of IC50s for to-be-tested compound and reference compound:

1 μL of a sample to be tested and 1 μL of a reference compound were added to a 96-well plate in sequence. The high signal control wells were 0.5% DMSO, and the ADORA1, ADORA2A, ADORA2B, and ADORA3 low signal control wells were CGS-15943. 100 μL of cells and 100 μL of isotopes were added to all experimental wells. The 96-well plate was sealed; ADORA1, ADORA2B, and ADORA3 were incubated for 1 hour on a shaker (300 rpm) at room temperature (22°C); and ADORA2A was incubated for 2 hours on a shaker (300 rpm) at room temperature (22°C). At the same time, a GF/C filter plate was soaked with ADORA1, ADORA2A, ADORA2B, and ADORA3 by using 0.3% PEI for at least 30 minutes at room temperature, at 50 μL/well. After the incubation was completed, the incubated reaction system was collected onto the GF/C filter plate with a Harvester, washed 4 times and then dried for 0.5 hours in an oven at 50°C. The bottom of the dried GF/C filter plate was sealed with a film, 50 μL of scintillation solution was added to each well, which was then sealed with a Top-seal-A film. With Microbeta readings, the data was analyzed by GraphPad Prism 5.0.

5. Data processing and analysis

% Inhibition = 100 × (1- (Sample_raw value - Low control_Average) / (High control_Average - Low control_Average))

%Inhibitor: %inhibition rate;
Sample_raw value: the raw reading of a test compound;
Low control Average: the low level reading of a reference compound;
High control_Average: the high level reading of a reference compound;
The data was fitted with "log(inhibitor) vs response-variable slope" in GraphPad Prism 5.0 to obtain IC50.

6. Test results

Table 10 Results of adenosine receptor binding test (IC50)

| | ADORA1 (IC50, nM, n=3) | ADORA2A (IC50, nM, n=3) | ADORA2B (IC50, nM, n=3) | ADORA3 (IC50, nM, n=3) |
|---|---|---|---|---|
| Compound 22 | 820.70±54.21 | 0.96 ±0.11 | 344.27±181.41 | 872.10±492.41 |
| Compound 40 | 552.47±283.33 | 1.85 ±0.27 | 268.43±87.47 | 1995.50±1033.89 |

(continued)

| | ADORA1 (IC50, nM, n=3) | ADORA2A (IC50, nM, n=3) | ADORA2B (IC50, nM, n=3) | ADORA3 (IC50, nM, n=3) |
|---|---|---|---|---|
| CGS-15943 | 4.48±2.06 | 0.99 ±0.30 | 4.71±0.90 | 18.02±11.42 |

Table 11 Results of adenosine receptor binding test

| | ADORA1 (IC50, nM, n=3) | ADORA2A (IC50, nM, n=3) | ADORA2B (IC50, nM, n=3) | ADORA3 (IC50, nM, n=3) |
|---|---|---|---|---|
| Compound 22 | 563.61 | 0.85 | 309.82 | 702.89 |
| Compound 40 | 441.33 | 1.64 | 241.57 | 1608.32 |
| CGS-15943 | 3.55 | 0.88 | 4.24 | 14.52 |

The results show that both Compounds 22 and 40 have an extremely high binding effect on $ADORA_{2A}$, but weak binding effect on another three adenosine receptors, indicating that the compounds of the present application have high binding selectivity to $ADORA_{2A}$.

**Example 6 Effects of present compounds in mouse reserpine-induced dyskinesia model**

**6.1 Effects of Compound 22 in mouse reserpine-induced dyskinesia model**

1. Test principle

Reserpine is an indole alkaloid drug, which can inhibit the reuptake of noradrenergic neuron terminals to lead to slowed movement or incompetence, thereby inducing clinical symptoms similar to those of the Parkinson's disease. Its mechanism is as follows: the reserpine can block the transport of irreversible monoamines such as DA, 5-hydroxytryptamine (5-HT) and norepinephrine (NA) in vesicles, such that the monoamines are degraded in cells to consume central and peripheral monoamines, and rapidly reduce the level of monoamines, resulting in dyskinesias and other Parkinson's disease-like symptoms. An A2A receptor antagonist was administered to evaluate if the compound under test can ameliorate the dyskinesia of the mice. The evaluation indicators include walking distance, walking time, number of standings and standing time.

2. Test design

Group 1: a solvent control group (vehicle 1 + vehicle 2), with vehicle 1 (0.1% acetic acid, 10 mL/kg, subcutaneous injection) + vehicle 2 (acetic acid-sodium acetate containing 0.5% MC, 10 mL/kg, intragastric infusion, administrated 60 min before a behavioral test), and n=15;
Group 2: a model group (reserpine + vehicle 2), with reserpine (0.6 mg/kg, 10 mL/kg, subcutaneous injection) + vehicle 2 (acetic acid-sodium acetate containing 0.5% MC, 10 mL/kg, intragastric infusion, administrated 60 min before a behavioral test), and n=15;
Group 3: a group of reserpine + istradefylline, with reserpine (0.6 mg/kg, 10 mL/kg, subcutaneous injection) + istradefylline (10 mg/kg, 10 mL/kg, intragastric administration, administrated 60 min before a behavioral test), and n=15;
Group 4: a group of reserpine + acetate of Compound 22, with reserpine (0.6 mg/kg, 10 mL/kg, subcutaneous injection) + Compound 22 (3 mg/kg, 10 mL/kg, intragastric infusion, administered 60 minutes before a behavioral testing), and n=15;
Group 5: a group of reserpine + acetate of Compound 22, with reserpine (0.6 mg/kg, 10 mL/kg, subcutaneous injection) + Compound 22 (10 mg/kg, 10 mL/kg, intragastric infusion, administered 60 minutes before a behavioral testing), and n=15;
Group 6: a group of reserpine + acetate of Compound 22, with reserpine (0.6 mg/kg, 10 mL/kg, subcutaneous injection) + Compound 22 (30 mg/kg, 10 mL/kg, intragastric administration, administered 60 minutes before a behavioral testing), and n=15;

Male CD1 mice were weighed 29.5±0.1g. The total number of actually used animals was 90. The animals were purchased from Charles River Laboratories in Beijing (Animal Certificate Number: 1100112011009984) and raised in the animal house of ChemPartner in Shanghai (Experimental Animal License: SYXK (Shanghai) 2017-0018). The animal house has an SPF environment.

3. Main instruments and reagents

| Designation | Supplier | Model |
|---|---|---|
| Laboras animal spontaneous activity platform | Metris B.V, Netherlands | NA |

| Name of reagent consumable | Article No./Batch No. | Supplier |
|---|---|---|
| Methylcellulose | M0430-100G/SLCB9094 | SIGMA |
| Sodium acetate trihydrate | G70149B/P1536840 | GENERAL-REAGENT |
| Reserpine | R007/UQNCN-EH | TCI |
| Istradefy lline | I1100/2PJUO | Tokyo Chemical Industry Co., Ltd. |

4. Test Steps

1) Formulation of compound

A reserpine solution with a concentration of 0.06 mg/mL, an istradefylline solution with a concentration of 1 mg/ml, and the acetate solutions of Compound 22 respectively with concentrations of 3 mg/ml (concentration 1), 1 mg/ml (concentration 2) and 0.3 mg/ml (concentration 3) were prepared.

2) Test

A 3-channel timer, a syringe, a stainless steel workbench, a work stool, and related record forms required for the test should be placed in the test laboratory in advance one day before the start of the test. After the states of animals were checked, conditions such as underweight, docking, alopecia areata, and poor physical state were not found in this batch of animals. There were a total of 90 experimental animals, and a random grouping sequence was generated before the test. According to the random sequence, the experimental animals were randomly divided into the solvent control group, the model group, the istradefylline group, and groups of acetates of Compound 22 at 3, 10, and 30 mg/kg on the day of the test.

On the first day of the test, a single subcutaneous injection of 0.1% acetic acid or 0.6 mg/kg reserpine was performed based on the pre-set grouping and time points. After the injection, the animals were put back to rearing cages and pushed back to the original rearing room.

The activity test was carried out on the second day of the test, and before the test, the Laboras system needed to be calibrated in advance to make it in a normal working state. Then, based on the pre-set time points (17 hr after the single subcutaneous injection of 0.1% acetic acid or 1 mg/kg reserpine), vehicle 2 (acetic acid-sodium acetate (pH=4.0) buffer containing 0.5% MC), istradefylline or the acetate of Compound 22 was administrated by intragastric infusion. The mice were put into an independent Laboras test box 50 minutes after the administration, and the Laboras behavior recording system was started up after 10 minutes of adaptation. The current moment is the 1st hour after the last administration for the animals, and the animals were recorded continuously for 60 minutes.

After the experiment was completed, the experimental animals were put back to the rearing cages, and the Laboras test cages were cleaned.

After the test was completed, the Laboras behavior system automatically analyzed the walking distance (m), walking time (sec), the number of standings and standing time (sec) of the mice at 0-30 min, 30-60 min, 0-60 min and every 10 min.

All measurement data were represented as mean ± standard error of mean (mean ± SEM), and check analysis was performed by Prism 8.0 statistical software.

5. Results and conclusions

Table 12 Impact of present compounds on walking distance of mice

| Group | Walking distance (m, mean ± SEM, n = 15) | | |
|---|---|---|---|
| | 0-30 min | 30-60 min | 0-60 min |
| Group 1 | 8.9±1.0 | 4.6±1.6 | 13.6±2.3 |
| Group 2 | 2.4±0.7 | 0.3±0.1 | 2.8±0.7 |
| Group 3 | 11.9±1.4 | 11.8±2.0 | 23.7±3.2 |
| Group 4 | 4.3±0.7 | 2.6±1.6 | 6.9±2.1 |
| Group 5 | 10.7±1.0 | 8.5±2.3 | 19.2±2.9 |
| Group 6 | 11.3±1.1 | 15.9±1.9 | 27.1±2.5 |

Table 13 Impact of present compounds on spontaneous walking time of mice

| Group | Walking time (sec, mean ± SEM, n = 15) | | |
|---|---|---|---|
| | 0-30 min | 30-60 min | 0-60 min |
| Group 1 | 164.1±17.1 | 62.7±15.5 | 226.8±29.7 |
| Group 2 | 43.9±11.4 | 7.4±2.7 | 51.3±10.9 |
| Group 3 | 177.5±16.0 | 120.8±16.0 | 298.3±28.4 |
| Group 4 | 77.8±11.0 | 30.7±13.9 | 108.5±23.0 |
| Group 5 | 160.5±10.8 | 84.9±22.4 | 245.4±29.1 |
| Group 6 | 175.6±12.4 | 135.4±12.8 | 311.0±22.3 |

Table 14 Impact of present compounds on the number of standings of mice

| Group | Number of standings (sec, mean ± SEM, n = 15) | | |
|---|---|---|---|
| | 0-30 min | 30-60 min | 0-60 min |
| Group 1 | 86.8±12.1 | 35.9±11.6 | 122.6±21.5 |
| Group 2 | 17.4±5.6 | 2.4±1.5 | 19.8±5.5 |
| Group 3 | 94.3±11.1 | 70.6±10.6 | 164.8±20.0 |
| Group 4 | 36.7±5.9 | 12.9±6.8 | 49.6±12.2 |
| Group 5 | 75.6±8.0 | 40.3±11.4 | 115.9±15.7 |
| Group 6 | 98.7±12.6 | 79.4±10.1 | 177.9±20.9 |

The results are shown in Tables 12-14. The results show that the oral (OP) administration of the acetates of Compound 22 at 10 mg/kg and 30 mg/kg can significantly increase walking distance, walking time, and the number of standings. The test show that the acetate of Compound 22 has a therapeutic effect in the mouse reserpine-induced dyskinesia model, and the pharmaceutical effect exhibits a significant dose-effect relationship. In addition, istradefylline as the control drug shows approximately the same pharmaceutical effect in this test. The test with the mouse reserpine-induced dyskinesia model provides support to the application of acetate of Compound 22 to the treatment of Parkinson's disease.

**6.2 Effects of acetate of Compound 22 in rat reserpine-induced dyskinesia model**

1. Experimental design

Group 1: a solvent control group (vehicle1 + vehicle 2), with vehicle 1 (0.1% acetic acid, 1 mL/kg, subcutaneous injection) + vehicle 2 (acetic acid-sodium acetate containing 0.5% MC, 10 mL/kg, intragastric infusion, administrated 60min before a behavioral test), and n=15;
Group 2: a model group (reserpine + vehicle 2), with reserpine (1 mg/kg, 1 mL/kg, subcutaneous injection) + vehicle 2 (acetic acid-sodium acetate containing 0.5% MC, 10 mL/kg, intragastric infusion, administrated 60min before a behavioral test), and n=15;
Group 3: a group of reserpine + istradefylline, with reserpine (1 mg/kg, 1 mL/kg, subcutaneous injection) + istradefylline (15 mg/kg, 10 mL/kg, intragastric administration, administrated 60 min before a behavioral test), and n=15;
Group 4: a group of reserpine + acetate of Compound 22, with reserpine (1 mg/kg, 1 mL/kg, subcutaneous injection) + acetate of Compound 22 (7.5 mg/kg, 10 mL/kg, intragastric infusion, administered 60 minutes before a behavioral testing), and n=15;
Group 5: a group of reserpine + acetate of Compound 22, with reserpine (1 mg/kg, 1 mL/kg, subcutaneous injection) + acetate of Compound 22 (15 mg/kg, 10 mL/kg, intragastric infusion, administered 60 minutes before a behavioral testing), and n=15; and
Group 6: a group of reserpine + acetate of Compound 22, with reserpine (1 mg/kg, 1 mL/kg, subcutaneous injection) + acetate of Compound 22 (30 mg/kg, 10 mL/kg, intragastric administration, administered 60 minutes before a behavioral testing), and n=15.

2. The walking distance (m), walking time (sec), the number of standings and standing time (sec) of the mice at 0-30 min, 30-60 min, 0-60 min and every 10 min were analyzed by using the same method as that in 11.1.

3. Results and conclusions

Table 15 Impact of present compounds on walking distance of rat

| Group | Walking distance (m, mean ± SEM, n = 15) | | |
|---|---|---|---|
| | 0-30min | 30-60min | 0-60min |
| Group 1 | 2.5±0.4 | 1.8±0.4 | 4.3±0.7 |
| Group 2 | 0.4±0.1 | 0.1±0.0 | 0.4±0.1 |
| Group 3 | 1.6±0.6 | 1.3±0.4 | 2.9±0.9 |
| Group 4 | 0.9±0.3 | 1.0±0.3 | 2.0±0.6 |
| Group 5 | 1.8±0.4 | 2.0±0.4 | 3.8±0.6 |
| Group 6 | 2.5±1.0 | 1.4±0.4 | 3.9±1.4 |

Table 16 Impact of present compounds on spontaneous walking time of rat

| Group | Walking time (sec, mean ± SEM, n = 15) | | |
|---|---|---|---|
| | 0-30min | 30-60min | 0-60min |
| Group 1 | 37.5±6.0 | 32.5±10.6 | 62.1±10.7 |
| Group 2 | 3.4±1.5 | 1.2±0.5 | 4.1±1.5 |
| Group 3 | 19.6±5.9 | 32.0±13.7 | 35.1±10.1 |
| Group 4 | 9.3±4.3 | 16.2±7.6 | 17.8±7.5 |
| Group 5 | 26.3±5.6 | 40.3±15.6 | 48.5±8.2 |

(continued)

| Group | Walking time (sec, mean ± SEM, n = 15) | | |
|---|---|---|---|
| | 0-30min | 30-60min | 0-60min |
| Group 6 | 35.8±12.6 | 33.2±12.2 | 52.9±17.5 |

Table 17 Impact of test compounds on the number of standings of rats

| Group | Number of standings (sec, mean ± SEM, n = 15) | | |
|---|---|---|---|
| | 0-30min | 30-60min | 0-60min |
| Group 1 | 68.1±8.5 | 47.0±8.7 | 115.0±16.0 |
| Group 2 | 13.8±2.9 | 2.7±1.3 | 16.5±3.8 |
| Group 3 | 44.1±7.9 | 51.6±9.7 | 95.7±16.3 |
| Group 4 | 28.5±7.1 | 28.3±6.9 | 56.7±12.6 |
| Group 5 | 51.9±7.4 | 58.2±7.7 | 110.0±12.7 |
| Group 6 | 56.5±9.6 | 41.5±6.7 | 97.9±15.3 |

The test results show that the compound of the present application can significantly increase the walking distance, walking time, standing time and number of standings of animals at doses of 15 mg/kg and 30 mg/kg. Experiments show that the acetate of Compound 22 has a therapeutic effect in the rat reserpine-induced dyskinesia model, and exhibits a dose-effect relationship. In addition, istradefylline as the control drug shows approximately the same pharmaceutical effect in this test. The test with the rat reserpine-induced dyskinesia model provides support to the application of acetate of Compound 22 to the treatment of Parkinson's disease.

**Example 7 Effect of present compounds on rat haloperidol-induced Parkinson's disease stiffness model**

Parkinson's disease (PD) is a common class of degenerative disease of the nervous system with the main pathological changes including the loss of substantia nigra dopaminergic neurons and the decrease of dopamine transmitter content, leading to tremor at rest, muscle stiffness, akinesis, postural reflex disorders and other clinical symptoms. Haloperidol, a dopamine D2 receptor blocker, can block the dopamine D2 receptor in the striatum, leading to mouse dystonic postures, namely, stiffness and hypokinesia. This model is a recognized animal model of Parkinson's syndrome. The ameliorative effect of a test substance on the stiffness symptom of Parkinson's disease induced by haloperidol in CD-1 mice was evaluated.

**7.1 Effects of benzene sulfonate of Compound 40, Compound 27 and acetate of Compound 22 on haloperidol-induced rat Parkinson's disease stiffness model**

1. Test design and method

1) Experiment I:

57 SPF-grade male CD-1 mice (4 weeks old at the time of purchase and 8 weeks old at the time of experiment) with the weight of about 40 g were randomly divided into 5 groups, namely, a vehicle group (n=11), a group of 1 mg/kg benzene sulfonate of Compound 40 (n=11), a group of 3 mg/kg benzene sulfonate of Compound 40 (n=12), a group of 10 mg/kg benzene sulfonate of Compound 40 (n=12), and a group of 30 mg/kg benzene sulfonate of Compound 40 (n=11). 1 mg/kg haloperidol was injected subcutaneously at an injection volume of 10 ml/kg. 30 minutes after the injection, Compound 40 at each dose was intragastrically infused at a volume of 10 ml/kg. At the 1st hour and the 4th hour after the administration was completed, the rearing and bar tests were performed respectively, and the number of standings and on-bar time of the mice were counted to evaluate the ameliorative effect of Compound 40 on the stiffness symptom of haloperidol-induced Parkinson's disease in CD-1 mice.

2) Experiment II:

40 SPF-grade male CD-1 mice (4 weeks old at the time of purchase and 5 weeks old at the time of experiment) with the weight of about 25 g were randomly divided into 5 groups, namely, a vehicle group (n=8), a group of 3mg/kg acetate of Compound 22 (n=8), a group of 10 mg/kg acetate of Compound 22 (n=8), a group of 30 mg/kg acetate of Compound 22 (n=8), and a group of 10mg/kg istradefylline (n=8). Firstly, the acetate of Compound 22 at each dose was intragastrically infused at a volume of 10 ml/kg. After 30 min, 1 mg/kg haloperidol was injected subcutaneously at an injection volume of 10 ml/kg. At the 1st hour and the 4th hour after the administration was completed, the rearing and bar tests were performed respectively, and the number of standings and on-bar time of the mice were counted to evaluate the ameliorative effect of the acetates of Compound 22 on the stiffness symptom of haloperidol-induced Parkinson's disease in CD-1 mice.

3) Experiment III:

40 SPF-grade male CD-1 mice (4 weeks old at the time of purchase and 5 weeks old at the time of experiment) with the weight of about -30g were randomly divided into 5 groups, namely, a vehicle group (n=8), a group of 3mg/kg Compound 27 (n=8), a group of 10 mg/kg Compound 27 (n=8), a group of 30 mg/kg Compound 27 (n=8), and a group of 10 mg/kg istradefylline (n=8). Firstly, Compound 27 at each dose was intragastrically infused at a volume of 10 ml/kg. After 30 min, 1 mg/kg haloperidol was injected subcutaneously at an injection volume of 10 ml/kg. At the 1st hour and the 4th hour after the administration was completed, the rearing and bar tests were performed respectively, and the number of standings and on-bar time of the mice were counted to evaluate the ameliorative effect of Compound 27 on the stiffness symptom of haloperidol-induced Parkinson's disease in CD-1 mice.

2. Information of materials and reagents/compounds

Experimental animals: CD-1 mice, SPF grade, 8 weeks old/5 weeks old, male, about 40g/25g, laboratory animal provider: Shanghai SLAC Laboratory Animal Co., Ltd.; production license number: SCXK (Shanghai) 2017-0005; quality certificate number: 20170005023704 and 20170005024947.

| Name of reagent consumable | Article No./Batch No. | Supplier |
|---|---|---|
| Methylcellulose | P10995 | adamas-beta |
| Haloperidol | H113852-5g / G1411016 | Aladdin |
| Sodium acetate | P1246037 | GENERAL-REAGENT |
| Glacial acetic acid | P1480181 | adamas-beta |
| 2-Hydroxypropyl-β-cyclodextrin | B1922043 | Aladdin |
| Istradefy lline | 2PJUO-EB | TCI |

4. Experimental steps

1) Formulation of drug

Vehicle solvent control: 40% hydroxypropyl β-cyclodextrin solution

20.00 g of hydroxypropyl β-cyclodextrin was weighed; 50 ml of ultrapure water was added to the hydroxypropyl β-cyclodextrin; the resulting mixture was vortexed, shaken and sonicated for 20 minutes until the solution was clear; and the resultant was stored at room temperature for later use. Vehicle solvent control: acetic acid-sodium acetate (pH≈4.0) buffer containing 0.5% MC

Preparation of 2M acetic acid solution: 11.6 mL of acetic acid was added to a 100mL graduated cylinder, and then ultrapure water was added to 100mL to obtain the 2 M acetic acid, which was stored in a 100 mL brown bottle.

Preparation of acetic acid-sodium acetate (pH≈4.0) buffer containing 0.5% MC: 0.0904 g of sodium acetate was weighed and added with 1.95 mL of 2M acetic acid; the resultant was added to a 100mL graduated cylinder; ultrapure water was added to about 80 mL; pH was measured with test paper, which was about 3.5-4; then, ultrapure water was added to 100 mL; then, 0.5013 g of MC was added and mixed well, until MC was completely dissolved; and the resultant was stored in a 100 mL brown bottle at room temperature.

Formulation of haloperidol (dosage volume: 10 ml/kg)

This formulation was carried out for immediate use. 0.0037 g of haloperidol was weighed and added with 7.4 ml of 0.9% normal saline; the resulting mixture was vortexed and shaken, added with 20 ul of 1M HCl, and vortexed, shaken and sonicated for 20 min for even mixing, where the concentration here was 0.5 mg/ml; and the resulting mixture was subjected to 5-fold dilution to reach 0.1 mg/ml as a working concentration, which was stored at room temperature in the dark.

Formulation of benzene sulfonate solution of Compound 40 (dosage volume: 10 ml/kg)

This formulation was carried out for immediate use, with a correction factor of 1.415. 0.0199 g of the compound was weighed and added with 4.7 ml of 40% HP-β-CD; the resulting mixture was vortexed and sonicated for 10 min at room temperature for even mixing, where the working concentration here was 3 mg/ml; 1 ml of the resulting solution with the concentration of 3 mg/ml was pipetted and added with 2 ml of 40% HP-β-CD, where the working concentration here was 1 mg/ml; 0.2 ml of the solution with the concentration of 3 mg/ml was pipetted and added with 1.8 ml of 40% HP-β-CD, where the working concentration here was 0.3 mg/ml; and 0.2 ml of the solution with the concentration of 1 mg/ml was pipetted and added with 1.8 ml of 40% HP-β-CD, where the working concentration here was 0.1 mg/ml.

Formulation of acetate solution of Compound 22 (dosage volume: 10 ml/kg)

This formulation was carried out for immediate use, with a correction factor of 1.153. 0.0225g of the compound was weighed and added with 6.5 ml of acetate-sodium acetate buffer containing 0.5% MC; the resulting mixture was vortexed and sonicated for 10 min at room temperature for even mixing, where the working concentration here was 3 mg/ml; 1 ml of the resulting solution with the concentration of 3 mg/ml was pipetted and added with 2 ml of acetate-sodium acetate buffer containing 0.5% MC, where the working concentration here was 1 mg/ml; and 0.2 ml of the solution with the concentration of 3 mg/ml was pipetted and added with 1.8 ml of acetate-sodium acetate buffer containing 0.5% MC, where the working concentration here was 0.3 mg/ml.

Formulation of Compound 27 (dosage volume: 10 ml/kg)

This formulation was carried out for immediate use, with a correction factor of 1.013. 0.0182g of the compound was weighed and added with 6ml of 40% HP-β-CD; the resulting mixture was vortexed and sonicated for 10 min at room temperature for even mixing, where the working concentration here was 3 mg/ml; 1 ml of the resulting solution with the concentration of 3 mg/ml was pipetted and added with 2 ml of 40% UP-β-CD, where the working concentration here was 1 mg/ml; and 0.2 ml of the solution with the concentration of 3 mg/ml was pipetted and added with 1.8 ml of 40% HP-β-CD, where the working concentration here was 0.3 mg/ml.

Formulation of istradefylline (dosage volume: 10 ml/kg)

This formulation was carried out for immediate use, with the concentration of 1 mg/ml. 0.0040g of istradefylline was weighed and added with 4 ml of 40% HP-β-CD or acetate-sodium acetate buffer containing 0.5% MC; the resulting mixture was vortexed and sonicated for 10 min at room temperature for even mixing.

2) Modeling with haloperidol

The mice were subcutaneously injected with haloperidol at abdomen at a dose of 1 mg/kg, with the concentration of a working solution of 0.1 mg/ml and an injection volume of 10 ml/kg.

3) Dosing

It was conducted following the experimental design.

4) Rearing detection method

At the 1st hour and the 4th hour after drug intervention, the mice were put into a white transparent plastic bucket with a diameter of 20 cm and a height of 21 cm. The mice were allowed to acclimate in the bucket for 5 min before starting video recording. The spontaneous activity behaviors of the mice within 10 minutes were recorded, whereby the number of standings was counted. The more the standings, the more frequent the spontaneous exploration activities of

the mice, and the lower the degree of stiffness.

5) Bar test method

At the time points of 1st and 4th hours after the rearing test was completed, bar test was carried out. The mice were placed on a long wooden bar with a diameter of 6 cm and a height of 6 cm. The mice were let to hold the wooden bar with forelimbs, only with the buttocks or hind limbs on the ground. The timing was stopped when the hind limbs left the bench top or the fore limbs left the wooden bar, and the on-bar time of the mice was recorded. Each mouse was tested 3 times, and the maximum on-bar time and the average on-bar time were counted. The longer the on-bar time, the more severe the stiffness of the mice.

6) Statistical analysis

The statistical analysis between groups was performed on the data by using Prism GraphPad 7.0 independent sample T test, and $P < 0.05$ was considered as a significant difference.

**5. Experimental results and conclusions**

Table 18 Impacts of different doses of benzene sulfonate of Compound 40 on behavioral test results of mice ($\bar{x} \pm SEM$)

| Time points | 1st hour | | | 4th hour | | |
|---|---|---|---|---|---|---|
| Group | Maximum on-bar time (sec) | Average on-bar time (sec) | Number of standings | Maximum on-bar time (sec) | Average on-bar time (sec) | Number of standings |
| Solvent blank group | 399.22±54.6 6 | 295.11±47.47 | 9.56±2.11 | 378.89±60.31 | 253.78±44.36 | 4.56±1.54 |
| Benzene sulfonate of Compound 40 (1 mg/kg) | 356.43±55.7 9 | 237.67±40.20 | 17.00±1.99 | 361.29±53.92 | 241.14±31.32 | 8.71±2.68 |
| Benzene sulfonate of Compound 40 (3 mg/kg) | 184.00±50.5 5* | 120.05±35.31 * | 17.00±2.99 | 225.14±51.96 | 171.76±52.86 | 14.86±1.89** |
| Benzene sulfonate of Compound 40 (10 mg/kg) | 57.00±11.21 *** | 36.10±5.88*** | 34.90±4.75*** | 96.10±23.06** | 66.07±13.96** | 17.80±4.32* |
| Benzene sulfonate of Compound 40 (30 mg/kg) | 34.88±6.68*** | 26.50±5.88*** | 43.25±3.89*** | 24.13±1.67*** | 20.00±1.28*** | 25.13±3.25*** |
| Note: Compared to the vehicle group, *P < 0.05, **P < 0.01, and ***P < 0.001. | | | | | | |

Table 18 shows that after the mice are injected with haloperidol, they show a state of few activities, reduced spontaneous activity, and prolonged stiffness time. In the group administrated with the benzene sulfonate of Compound 40, the behaviors of mice were significantly improved, with increases in on-bar time and number of standings, and a certain dose-effect relationship was exhibited.

Table 19 Impacts of different doses of acetate of Compound 22 on behavioral test results of mice ($\bar{x} \pm SEM$)

| Time points | 1h | | 4h | |
|---|---|---|---|---|
| Group | Maximum on-bar time (sec) | Average on-bar time (sec) | Maximum on-bar time (sec) | Average on-bar time (sec) |
| Solvent blank group | 248.25±49.94 | 177.54±32.84 | 233.50±44.36 | 182.13±32.98 |
| Acetate of Compound 22 (3 mg/kg) | 157.50±18.11 | 109.38±13.74 | 172.25±58.28 | 109.33±26.37 |
| Acetate of Compound 22 (10 mg/kg) | 77.13±25.78* | 41.88±10.56** | 50.50±12.24** | 33.17±6.69** |
| Acetate of Compound 22 (30 mg/kg) | 42.25±8.18** | 26.17±3.88*** | 32.88±6.21*** | 24.13±4.28*** |
| Istradefy lline 10mg/kg | 30.38±5.84** | 23.04±4.88*** | 35.75±8.73** | 24.67±5.21*** |
| Note: Compared to the vehicle group, *P < 0.05, **P < 0.01, and ***P < 0.001. | | | | |

Table 19 shows that during the bar test, the acetate of Compound 22 can reduce the on-bar time and prolong the standing time of mice, and the results show a good dose-response dependence, that is, as the dose of the acetate of Compound 22 increases, the stiffness time is reduced and the on-bar time is shortened. The pharmaceutical effect of the 10 mg/kg group and the 30 mg/kg group is equivalent to that of the positive drug.

Table 20 Impacts of different doses of Compound 27 on behavioral test results of mice ($\bar{x} \pm SEM$)

| Time points | 1h | | 4h | |
|---|---|---|---|---|
| Group | Maximum on-bar time (sec) | Average on-bar time (sec) | Maximum on-bar time (sec) | Average on-bar time (sec) |
| Solvent blank group | 147.63±15.82 | 111.92±13.49 | 170.25±26.73 | 126.71±16.14 |
| Compound 27 (3 mg/kg) | 88.00±22.07 | 56.83±11.67* | 109.33±28.68 | 75.11±19.52 |
| Compound 27 (10 mg/kg) | 61.38±15.59** | 34.83±8.63*** | 55.13±18.32** | 39.13±14.33** |
| Compound 27 (30 mg/kg) | 35.00±8.59*** | 22.04±4.19*** | 31.88±5.75*** | 23.00±4.46*** |
| Istradefy lline (10mg/kg) | 40.75±13.17*** | 23.71±6.50*** | 54.75±15.58** | 34.08±6.69*** |
| Note: Compared to the vehicle group, *P < 0.05, **P < 0.01, and ***P < 0.001. | | | | |

Table 20 shows that during the bar test, Compound 27 can reduce the on-bar time and prolong the standing time of mice, and the results show a good dose dependence, that is, as the dose of the compound increases, the stiffness time is reduced and the on-bar time is shortened. The pharmaceutical effect of the 10 mg/kg group is equivalent to that of the positive drug, and the pharmaceutical effect of the 30 mg/kg group is better than that of the positive drug group.

The test results show that, after single oral administration of each of three types of test compounds to the

haloperidol-induced stiffness mice, the test mice can be extremely significantly improved with reduced stiffness time and shortened on-bar time, which indicates that the test compound can resist or prevent the haloperidol-induced stiffness symptom and exhibits a dose-effect relationship. In addition, istradefylline as the control drug shows approximately the same pharmaceutical effect in this test. The test with the haloperidol-induced mouse Parkinson's disease stiffness model provides support to the application of test compound to the treatment of Parkinson's disease.

**7.2 Effects of acetate of Compound 22 on haloperidol-induced rat Parkinson's disease stiffness model**

The effects of the acetate of compound in the following dosage regimens on the haloperidol-induced rat Parkinson's disease stiffness model were tested.

1. Experimental design

Group 1: a blank control group (vehicle 1 + vehicle 2), with vehicle 1 (acetic acid-sodium acetate containing 0.5% MC, 10mL/kg, intragastric infusion, administrated 30 min before haloperidol injection) + vehicle 2 (acetic acid aqueous solution, 5 mL/kg, intraperitoneal administration, administrated before the test), and n = 10;
Group 2: a model group (vehicle 1 + haloperidol), with vehicle 1 (acetic acid-sodium acetate containing 0.5% MC, 10mL/kg, intragastric infusion, administrated 30 min before haloperidol injection) + haloperidol (1 mg/kg, 5 mL/kg, intraperitoneal administration, administrated before the test), and n = 10;
Group 3: a group of istradefylline + haloperidol model, with istradefylline (10 mg/kg, 10 mL/kg, intragastric infusion, administrated 30 min before haloperidol injection) + haloperidol (1 mg/kg, 5 mL/kg, intraperitoneal administration, administrated before the test), and n = 10;
Group 4: a group of acetate of Compound 22 + haloperidol model, with acetate of Compound 22 (3 mg/kg, 10 mL/kg, intragastric infusion, administrated 30 min before haloperidol injection) + haloperidol (1 mg/kg, 5 mL/kg, intraperitoneal administration, administrated before the test), and n = 10;
Group 5: a group of acetate of Compound 22 + haloperidol model, with acetate of Compound 22 (10 mg/kg, 10 mL/kg, intragastric infusion, administrated 30 min before haloperidol injection) + haloperidol (1 mg/kg, 5 mL/kg, intraperitoneal administration, administrated before the test), and n = 10;
Group 6: a group of acetate of Compound 22 + haloperidol model, with acetate of Compound 22 (30 mg/kg, 10 mL/kg, intragastric infusion, administrated 30 min before haloperidol injection) + haloperidol (1 mg/kg, 5 mL/kg, intraperitoneal administration, administered before the test), and n=10.

2. Experimental method

The animals required for the day were transferred from the rearing room to an operation room in advance, and were let to acclimate for at least 30 min before the test was initiated. According to the preset grouping, the test animals were divided into the blank control group, the model group, the istradefylline group, and groups of acetates of Compound 22 at 3, 10, and 30 mg/kg. The test animals were orally administered with vehicle 1 (acetic acid-sodium acetate (pH=4.0) buffer containing 0.5% MC), istradefylline, or acetate of Compound 22, and then the animals were put back to the original rearing cages. After 30 minutes, the test animals were intraperitoneally injected with haloperidol or vehicle 2 without haloperidol, and put back to the original rearing cages.

At 60 and 120 minutes after the injection of haloperidol or vehicle 2, the forelimbs of the rat were gently placed vertically on a horizontal metal rod with a length of 20.5 cm, a diameter of 1.2 cm, and a height of 8.5 cm above the workbench, then its hind limbs were gently placed on a test box, and timing started.

When the rat moved or climbed down on the metal rod with forelimbs, the timing was stopped, and the duration for the two forelimbs of the rat to maintain the posture on the metal rod was recorded, i.e., being recorded as stiffness latency. If the rat's front paws were not put down, the observation was terminated after 600 sec, and the stiffness latency of this animal was recorded as 600 sec. When the rod climbing test was completed that day, 5 animals from each group were perfused and fixed, and whole brains were removed for preservation.

Stiffness latency: the time for the two forelimbs of a rat to keep still on the metal rod. The longest stiffness latency was 600 sec.

$$\text{Stiffness rate\%} = (\text{stiffness latency}/600) * 100\%$$

All measurement data were represented as mean ± standard error of mean (mean ± SEM), and check analysis

was performed by Prism 8.0 statistical software.

3. Results and conclusions

Table 21 Impact of present compounds on stiffness latency of rats

| Group | 60 min (sec, mean ± SEM) | *p* value | 120 min (sec, mean ± SEM) | *p* value |
|---|---|---|---|---|
| Group 1 | 0.1±0.1 | n/a | 0±0 | n/a |
| Group 2 | 295.1±61.6 | p=0.0001 | 396.2±63.0 | P<0.0001 |
| Group 3 | 1.4±0.8 | P<0.0001 | 0.4±0.3 | P<0.0001 |
| Group 4 | 9.3±4.5 | P<0.0001 | 24.8±11.2 | P<0.0001 |
| Group 5 | 0.7±0.4 | P<0.0001 | 2.6±1.5 | P<0.0001 |
| Group 6 | 0.6±0.3 | P<0.0001 | 0.6±0.4 | P<0.0001 |

Table 22 Impact of present compounds on stiffness rate of rat

| Group | 60 min (sec, mean ± SEM) | *p* value | 120 min (sec, mean ± SEM) | *p* value |
|---|---|---|---|---|
| Group 1 | 0.02±0.02 | n/a | 0±0 | n/a |
| Group 2 | 49.2±10.3 | p=0.0001 | 66.0±10.5 | P<0.0001 |
| Group 3 | 0.2±0.1 | P<0.0001 | 0.07±0.05 | P<0.0001 |
| Group 4 | 1.6±0.8 | P<0.0001 | 4.1±1.9 | P<0.0001 |
| Group 5 | 0.1±0.06 | P<0.0001 | 0.4±0.2 | P<0.0001 |
| Group 6 | 0.1±0.05 | P<0.0001 | 0.1±0.07 | P<0.0001 |

The test results show that, after single oral administration of the acetate of Compound 22 at each dose of 3, 10 and 30 mg/kg to the haloperidol-induced stiffness rat, the stiffness latency of the test rats can be extremely significantly shortened (i.e., reducing the stiffness rate), which indicates that the acetate of Compound 22 can resist or prevent the haloperidol-induced stiffness symptom and exhibits a dose-effect relationship. In addition, istradefylline as the control drug shows approximately the same pharmaceutical effect in this test. The test with the haloperidol-induced rat Parkinson's disease stiffness model provides support to the application of the acetate of Compound 22 to the treatment of Parkinson's disease.

**7.3 Pharmacodynamic evaluation of benzene sulfonate of Compound 40 in haloperidol-induced Wistar rat stiffness model**

1. Test design and method

5 groups were set up for the experiment, including a modeling group of a vehicle, the groups of benzene sulfonates of Compound 40 (at 3 mg/kg, 10 mg/kg and 30 mg/kg), and a group of istradefylline (10 mg/kg), with n = 8, 8, 9, 8, and 8. The vehicle, the benzene sulfonates of Compound 40 (at 3 mg/kg, 10 mg/kg, and 30mg/kg), and the 10 mg/kg istradefylline were intragastrically administered at a dosage volume of 10 mL/kg; after 30 min, the intraperitoneal injection of 1 mg/kg haloperidol was started at a dosage volume of 5 mL/kg; after modeling, the rearing test was performed to obtain the number of standings; and after the rearing test was completed, the rats had a rest of 30 min and then underwent the bar test.

**2. Experimental steps**

**1) Formulation of drug**

Selection of vehicle: 40% HP-β-CD (2-hydroxypropyl)-β-cyclodextrin)
Vehicle solvent control (Ctrl): 40% HP-β-CD
1.40%HP-β-CD: 120.2 g of HP-β-CD was weighed and added with about 250 mL of ultrapure water; the resulting mixture was magnetically stirred at 900 rpm at room temperature for complete dissolution; then ultrapure water was added to reach 300 mL; and the resultant was stored in a 500 mL brown bottle in the dark at room temperature.

Benzene sulfonate of Compound 40: it was prepared for immediate use. For the concentration of at 3 mg/mL, 0.1777 g was weighed out and added with 42.5 mL of 40% HP-β-CD; the resulting mixture was vortexed and shaken, sonicated for 10 min at room temperature, blown well, and then blown well with a 20 mL syringe (replaced with 2.5 mL needle); and the resultant was diluted 3 folds for the concentration of 1 mg/mL, and was diluted 10 folds for the concentration of 0.3 mg/mL. Istradefylline: it was prepared for immediate use. For the concentration of 1 mg/mL, 0.0312 g was weighed out and added with 31.2 mL of 40% HP-β-CD; and the resulting mixture was vortexed and shaken.

Haloperidol inducer: it was prepared for immediate use. For the concentration of 0.2 mg/mL, 0.0148 g was weighed out and added with 100 μL of acetic acid; then, 2 mL of ultrapure water was added; 390 μL of 14 M NaOH was added; the resultant was poured to about 35 mL; the pH (with a pen-type pH meter) was measured till pH=5.86; and ultrapure water was added to reach 74 mL.

2) Bar Test

Wistar rats were administrated intragastrically with the benzene sulfonates of compound 40 at different concentration and istradefylline respectively at a dosage volume of 10 mL/kg; after 30 minutes, haloperidol was administered intraperitoneally (1 mg/kg) at a dosage volume of 5 mL/kg; at the 1st hour after the administration of haloperidol, the number of standings was counted by the rearing test; and after the rearing test was completed, the rats had a rest of 30 min and then underwent the bar test. Time of bar test: The stiffness of the rats was tested by the bar test. That is, a wooden bar with a diameter of 8 mm was fixed between 2 iron stands and was 10 cm from the bench top; and then, the rat's tail was grabbed to make its front paws rest on the wooden bar and its hind paws or buttocks on the ground. Generally, the rat needs to acclimate several times when it was tested for the first time. The time for the forelimbs to drop from the bar or the time for the hind limbs to leave the bench top were tested, each rat was tested 3 times.

3) Rearing

Number of standings: the number of standings of the rat was measured by the rearing test. That is, the rat was placed in a 27*52 cm cage and acclimate for 5 minutes, and the spontaneous activity behavior, i.e., the number of standings, of the rat within 10 minutes was recorded. When the rat's hind limbs stood with two forelimbs raised over the shoulders and touched the ground, one standing is counted, and the one forelimb lift was excluded. Generally, the rat would raise its forelimbs to touch the cage wall. If the rat raised its forelimbs over the shoulders several times without falling to the ground, only one standing was counted.

The time of bar test was taken as a measurement index, including the average time and the maximum time. Long time indicates severe stiffness, short time indicates mild stiffness. After drug treatment, the shorter the time of bar test, the better the effect of the drug on relieving stiffness.

The number of standings was counted by the rearing test. The more the number of standings, the more frequent the spontaneous exploration activity of the rat, and the lower the stiffness.

4) Statistical Analysis

The statistical analysis between groups was performed on the data by using Prism GraphPad 7.0 independent sample T test, and $P < 0.05$ was considered as a significant difference.

5. Results and conclusions

Table 23 Impact of present compounds on stiffness time of rat

| Group | Maximum on-bar time (sec) | Average on-bar time (sec) | Number of standings |
|---|---|---|---|
| Vehicle solvent control | 463.75±53.97 | 302.44±36.24 | 0.00±0.00 |
| Benzene sulfonate of Compound 40 3mg/kg | 266.63±50.73 | 212.67±51.43 | 1.75±0.60* |
| Benzene sulfonate of Compound 40 10mg/kg | 69.67±28.71**** | 41.67±16.50**** | 7.89±0.98**** |
| Benzene sulfonate of Compound 40 30mg/kg | 18.00±2.21**** | 12.29±1.41**** | 13.00±0.96**** |
| Istradefy lline 10mg/kg | 15.38±2.75**** | 10.33±1.58**** | 23.25±2.86**** |
| Note: Compared to the vehicle group, *P < 0.05, **P < 0.01, ***P < 0.001, and ****P < 0.0001. | | | |

The results in Table 23 show that compared with the vehicle group, the rats in the treatment groups with the benzene sulfonates of Compound 40 at the doses of 10 mg/kg and 30 mg/kg and the group with istradefylline at 10 mg/kg have significantly reduced average stiffness time and maximum stiffness time, as well as significantly increased number of standings, all with significant differences (the *P* values of the average stiffness time after treatment were < 0.0001, < 0.0001, and < 0.0001 respectively, the *P* values of the maximum time were < 0.0001, < 0.0001, and < 0.0001 respectively, and the *P* values of the number of standings were < 0.0001, <0.0001, and <0.0001 respectively); and compared with the vehicle group, the rats in the group with the benzene sulfonate of compound 40 at a dose of 3 mg/kg show no statistically significant difference in the average stiffness time and the maximum stiffness time, but with significant difference in the number of standings (for the average stiffness time after treatment, *P* = 0.2741; for the maximum stiffness time after treatment, *P* = 0.0521; and for the number of standings after treatment, *P* = 0.0351). The test with the haloperidol-induced rat Parkinson's disease stiffness model provides support to the application of the benzene sulfonate of Compound 40 to the treatment of Parkinson's disease.

**Example 8 Pharmacodynamic evaluation of present compounds in mouse Parkinson's disease model induced by MPTP (1-methyl-4-phenyl-1,2,3,6-tetrahydropyridine)**

1. Experimental principle

By injecting the substantia nigra selective toxin MPTP, the mitochondria were intervened to induce lipid peroxidation for disturbing the membrane structure and affecting cell function, and finally the substantia nigra dopaminergic (DA) neurons were selectively destroyed, leading to the massive death of substantia nigra DA neurons, the massive loss of striatum tyrosine hydroxylase-positive fibers, and the significant decrease in the levels of striatum DA and its metabolites 3,4-dihydroxyphenylacetic acid and homovanillic acid. There are also the hyperplasia of the substantia nigra striatal microglia and astrocytes and the damage of locus coeruleus, hypothalamus and other areas, which are basically the same as those in patients with Parkinson's disease. The ameliorative effect of a test substance on the stiffness symptom of Parkinson's disease induced by MPTP in mice was evaluated.

2. Experimental design and method

| Group | Number of animals | Dosing | Dose | Administration mode | Administration frequency |
|---|---|---|---|---|---|
| Group 1 | 6 | Vehicle | N/A | p.o. | Once |
| Group 2 | 7 | Compound 22 | 10 mpk | p.o. | Once |
| Group 3 | 7 | Compound 22 | 30 mpk | p.o. | Once |

3. Experimental steps

C57BL/6 male mice of 8 weeks old were selected. The 8-week-old C57 mice were intraperitoneally (i.p.) injected with a vehicle dissolved in saline (normal saline) or MPTP (30 mg/kg body weight) for 5 days to induce the Parkinson's disease.
At 0.5 hours after the last MPTP injection, two doses (10 mpk and 30 mpk) were administered by intragastric administration.
At 1.5 hours after the last MPTP injection, an open field test (total travel distance and number of standings) was performed for 10 minutes.

4. Experimental results and conclusions

Table 24 Impact of present compounds on the motor ability of mice with Parkinson's mice

| Group | Moving distance (cm) | Number of standings |
|---|---|---|
| Model group (n = 6) | 139.5 | 0.83 |
| Compound 22 (10mg/kg) (n=6) | 647.0 | 18.3 |
| Compound 22 (30mg/kg) (n=6) | 1107.3 | 31.0 |

The results show that in the MPTP-induced mouse Parkinson's disease model, Compound 22 at the doses of 10 mg/kg and 30 mg/kg significantly reduces the mice's motor symptoms associated with the Parkinson's disease, and significantly improves both the moving distance and the number of standings, showing a dose-pharmaceutical effect relationship and a very good pharmaceutical effect. The test with the MPTP-induced mouse Parkinson's disease model provides support to the application of test Compound 22 to the treatment of Parkinson's disease.

**Example 9 Evaluation of the efficacy of the compound of the application in the 6-OHDA-induced rat rotation experiment and the evaluation of its overcoming effect on L-DOPA resistance**

1. Experimental principle

After long-term use of levodopa, patients with Parkinson's disease are prone to drug resistance (end-of-dose phenomenon). That is, the duration of its pharmaceutical effect tends to gradually become shorter (shortened opening period), its efficacy will become weaker and weaker, and the symptoms of the patients are becoming more and more uncontrollable. By the combined use with the test compound, the efficacy of levodopa can be restored for patients with Parkinson's disease in the off phase, who have already experienced the end-of-dose phenomenon. The rat model of Parkinson's disease was constructed by fixed-point injection of 6-hydroxydopamine (6-OHDA) to damage the substantia nigra striatum, and then the animals were administrated with levodopa (L-DOPA)/Benserazide (BID) for a long term (3-4 weeks as planned). After observing that the animals show resistance to levodopa, that is, when the rotation time induced by levodopa is significantly reduced (after 3-4 weeks), the animals were simultaneously administrated with the test Compound and levodopa/Benserazide, and the rotation time of the animals was measured to investigate the effect of the test compound on ameliorating the rotation symptoms of rats.

2. Experimental design

In the first stage, the same batch of model rats (a total of 15) were administrated the acetate of Compound 22 at different doses (0, 1, 3, 10, 30 and 100 mg/kg) for a rotation test; after 60-minute counting with a rotation counter, L-DOPA(5 mg/kg)/Benserazide(1.25 mg/kg) was administered by intraperitoneal injection; and then the rotation counting was performed for another 60 min by using the rotation counter. There should be at least an interval of 1 day between rotation tests for respective doses. A total of 6 tests were carried out in the first stage.
Animal grouping and drug treatment in the first stage:

| Group | Number of animals | Acetate of Compound 22 (mg/kg) | Number of dosage | Dosing Route | Dosage volume (mL/kg) | Rotation induction |
|---|---|---|---|---|---|---|
| 1 | 15 | 0 | Single | Oral administration | 5 | L-DOPA (5 mg/kg)/Benserazide (1.25 mg/kg), intraperitoneal injection, single administration |
| | | 1 | Single | Oral administration | 5 | |
| | | 3 | Single | Oral administration | 5 | |
| | | 10 | Single | Oral administration | 5 | |
| | | 30 | Single | Oral administration | 5 | |
| | | 100 | Single | Oral administration | 5 | |

In the second stage, the experimental animals in Groups 2, 3, 4, 5 and 6 were administered with a high dose of L-DOPA (25 mg/kg)/Benserazide (6.25 mg/kg) twice a day by intraperitoneal injection, aiming to induce the onset of drug resistance, wherein on Days 0 (Day 0 as the day before the start of administration of high-dose L-DOPA/Benserazide), 7, 14, 21 and 22, 5 rotation tests were carried out respectively to test the induced rotation of all the experimental animals which were administered with L-DOPA (20 mg/kg)/Benserazide (5 mg/kg) only once by intraperitoneal injection. On Day 22, at 60 minutes before the intraperitoneal injection of L-DOPA (20 mg/kg)/Benserazide (5 mg/kg), each test group was orally administered with the vehicle, test drug or positive drug. In order to evaluate whether the acetate of Compound 22 can prevent animals from developing resistance to L-DOPA/Benserazide, besides the intraperitoneal injection of L-DOPA (25 mg/kg)/Benserazide (6.25 mg/kg) administered twice a day, the experimental animals in Groups 7 and 8 were also orally administered with the test acetate of Compound 22 and the istradefylline as a control drug, twice a day. Four rotation tests were performed on Days 0, 7, 14 and 21 respectively. On the day of test, both the test drug and the control drug were administered orally only once. On this day, all test animals were also administered with L-DOPA (20 mg/kg)/Benserazide (5 mg/kg) once by intraperitoneal injection to induce rotation. In the second stage, the time for counting rotation with the rotation counter is 180 min.

| Group | Number of animals | Therapeutic drug | Administration dose (mg/kg) | Number of dosage | Dosage volume (mL/kg) | Route of administration | Drug resistant model |
|---|---|---|---|---|---|---|---|
| 2 | 10 | Vehicle | - | BID*21 Single (Day 22) | 5 | Oral administration | L-DOPA (25 mg/kg)/ Benseraz ide (6.25 mg/kg), intraperitoneal injection, BID*21) |
| 3 | 10 | Acetate of compound 22 | 7.5 | Single (Day 22) | 5 | Oral administration | |
| 4 | 10 | Acetate of compound 22 | 15 | Single (Day 22) | 5 | Oral administration | |
| 6 | 10 | Acetate of compound 22 | 30 | Single (Day 22) | 5 | Oral administration | |
| 5 | 10 | Istradefylline | 10 | Single (Day 22) | 5 | Oral administration | |
| 7 | 10 | Acetate of compound 22 | 5 or 10 | BID*21 | 5 | Oral administration | |
| 8 | 10 | Istradefylline | 10 | BID*21 | 5 | Oral administration | |

3. Experimental reagents and animals:

First stage: Wistar rats, 6-8 weeks old, male, 178-213 grams, experimental animal provider: Charles River Laboratories in Beijing, production license number: SCXK (Beijing) 2016-0006, quality certificate number: 1100112011004974;
Second stage: Wistar rats, 6-8 weeks old, male, 166-225 grams, experimental animal provider: Charles River Laboratories in Beijing, production license number: SCXK (Beijing) 2016-0006, quality certificate number: 1100112011011824 and 1100112011011826;

| Name of reagent consumable | Article No./Batch No. | Supplier |
|---|---|---|
| Methylcellulose | 079K0054V | Sigma-Aldrich |
| Glacial acetic acid | / | Tianjin Guangfu Technology Development Co., Ltd. |
| Sodium acetate trihydrate | WXBC8551V | Sigma-Aldrich |
| 6-hydroxydopamine | MKCH0942 | Sigma-Aldrich |
| L-ascorbic acid | SLBS0713V | Sigma-Aldrich |
| Apomorphine hydrochloride | / | Central Inspection Institute |
| Levodopa | SLCB0627 | Sigma-Aldrich |
| Benserazide hydrochloride | BCBZ1809 | Sigma-Aldrich |

4. Experimental steps and methods

Modeling of Parkinson's disease rats:
L-DOPA injection volume: each rat was injected with 4 μl of 6-OHDA solution (2.5 μg/μl) on the left medial forebrain bundle (MFB).

Coordinates for injecting L-DOPA: by taking the anterior fontanelle as a standard reference point and with reference to the stereotactic map of rat brain, the injection was performed on the left medial forebrain bundle at: anterior(A), -2.5 mm; latera 1(L), +2.0 mm; ventral (V), -8.5 mm.

L-DOPA injection speed: the injection speed was 1 μl/min; after injection, the needle was left in situ for 5 min, and then slowly withdrawn at a speed of 1 mm/min; and the wound was then sutured. Grouping and administration were carried out according to the method of experimental design.

In the first stage, only the number of rotations of the animal was tested, and only the rotation response time was tested in the second stage.

In the second stage, after the intraperitoneal injection of L-DOPA/Benserazide to the experimental animals, the total number of rotations was recorded every 5 min, and the number of rotations per 5 min was calculated based on this, and the 5 min with the largest number of rotations was found from it, and the number of rotations within this 5 min was defined as the peak number of rotations. The first 5 minutes when the number of rotations increased to 20% of the peak number of rotations was the starting point of the rotation response time, and the first 5 minutes when the number of rotations dropped to 20% of the peak number of rotations was the terminating point of the rotation response time. The time between the starting point and the terminating point was regarded as the rotation response time.

The test results are represented as "mean ± standard deviation". The data was statistically analyzed by the SPSS16.0 software package. One-way ANOVA, paired sample T test and independent sample T test were used for comparison, with P < 0.05 showing a statistical difference.

6. Results and conclusions

The results of the first stage are shown in Tables 25 and 26.

Table 25 Total number of rotations after single administration of acetate of Compound 22

| Test drug | Dose of test drug (mg/kg) | Total number of rotations (60 min) |
|---|---|---|
| Acetate of compound 22 | 0 | 10.4±7.8 |
| | 1 | 7.7±5.6 |
| | 3 | 17.1±18.7 |
| | 10 | 11.6±13.9 |
| | 30 | 30.7±22.5* |
| | 100 | 35.2±18.8 |
| Note: Compared with the vehicle group (acetate of compound 22, 0 mg/kg), *P < 0.05. | | |

Table 26 Total number of rotations (mean ± standard deviation) based on combined use of acetate of Compound 22 and L-DOPA

| Test drug | Dose of test drug (mg/kg) | Rotation-induced drug and dose | Total number of rotations (60 min) |
|---|---|---|---|
| Acetate of compound 22 | 0 | L-DOPA (5 mg/kg)/ Benserazide (1.25 mg/kg) | 21.2±16.2 |
| | 1 | | 41.4±80.8 |
| | 3 | | 36.6±46.3 |
| | 10 | | 109.4±205.2 |
| | 30 | | 285.2±283.8* |
| | 100 | | 379.3±326.4* |
| Note: L-DOPA (5 mg/kg)/Benserazide (1.25 mg/kg) was administered by intraperitoneal injection at the 1st hour after the acetate of Compound 22 was orally administered. Compared with the vehicle group (acetate of compound 22, 0 mg/kg), *P < 0.05. | | | |

The test in the first stage was intended to evaluate the pharmaceutical effect of the acetate of Compound 22 as the test drug at different doses (0, 1, 3, 10, 30 and 100 mg/kg) on 6-OHDA-induced contralateral rotation in rats.

The results show that compared with the administration of vehicle (the acetate of Compound 22, 0 mg/kg), the number of rotations of the animal increased at a high dose after different doses of acetate of Compound 22 as a test drug were administered separately. The test drug can significantly increase the number of contralateral rotations at the dose of 30 mg/kg.

Although the acetate of Compound 22 when used alone did not arouse animal rotation to a high degree at all doses, and the pharmaceutical effect of L-DOPA/Benserazide alone was not obvious at a low dose, the pharmaceutical effect achieved by the combined use of the acetate of Compound 22 and L-DOPA was significant when the L-DOPA/Benserazide was kept at the same low dose. The acetate of Compound 22 as the test drug at 5 doses could increase the number of contralateral rotations induced by the L-DOPA/Benserazide, showing a dose-dependent relationship, wherein at the doses of 30 mg/kg and 100 mg/kg, the acetate of Compound 22 could significantly increase the number of contralateral rotations of the animals.

The results of the second stage are shown in Table 27.

Table 27 Rotational reaction time of animals in each test group (mean ± standard deviation, min)

| Group | Rotational reaction time - Day 0 | Rotational reaction time - Day 7 | Rotational reaction time - Day 14 | Rotational reaction time - Day 21 | Rotational reaction time - Day 22 |
|---|---|---|---|---|---|
| Group 2: vehicle + L-DOPA (20-25 mg/kg)/Benserazide (5-6.25 mg/kg) | 139.4±17.6 | 121.1±9.9# | 109.3±18.1# | 113.6±11.8# | 119.3±17.4# |
| Group 3: Acetate of Compound 22 (7.5 mg/kg, Day 22) + L-DOPA (20-25 mg/kg)/Benserazide (5-6.25 mg/kg) | 129.0±9.1 | 110.0±27.1 | 109.5±11.7 | 111.5±14.0 | 133.5±30.0 |
| Group 4: Acetate of Compound 22 (15 mg/kg, Day 22) + L-DOPA (20-25 mg/kg)/Benserazide (5-6.25 mg/kg) | 128.9±11.7 | 113.3±13.0 | 111.1±17.8 | 97.5±42.7 | 146.3±37.0& |
| Group 5: Istradefylline (10 mg/kg, Day 22) + L-DOPA (20-25 mg/kg)/Benserazide (5-6.25 mg/kg) | 116.1±34.4 | 126.7±10.6 | 116.3±13.8 | 114.4±15.9 | 150.6±37.8& |
| Group 6: Acetate of Compound 22 (30 mg/kg, Day 22) + L-DOPA (20-25 mg/kg)/Benserazide (5-6.25 mg/kg) | 135.0±13.1 | 121.0±13.3 | 110.6±21.9 | 104.4±23.0 | 153.9±22.7& |
| Group 7: Acetate of Compound 22 (5 or 10 mg/kg) + L-DOPA (20-25 mg/kg)/Benserazide (5-6.25 mg/kg) | 150.5±11.9 | 145.6±3.0* | 133.9±8.9*# | 128.3±13.5* # | - |
| Group 8: Istradefylline | 161.5±14.2 | 125.0±23.5 | - | - | - |
| (10 mg/kg) + L-DOPA (20-25 mg/kg)/Benserazide (5-6.25 mg/kg) | | | | | |

The results show that after the administration of high-dose L-DOPA (25 mg/kg, BID)/Baenserazide (6.25 mg/kg, BID) to 6-OHDA Parkinson's disease model of rats, the rats show signs of resistance to the L-DOPA on the 7th day, which is manifested by the significantly shortened rotation response time under the induction of L-DOPA, and this condition maintains until the 22nd day, i.e., the end of the test.

On Day 22, after the single administration of acetate of Compound 22 (7.5, 15 and 30 mg/kg) at different doses was administrated to the rats of 6-OHDA Parkinson's disease model with drug resistance, compared with Day 0 and Day 21, the test drug can restore or increase the rotation response time of the animal to varying extents, and the test drug can significantly increase the rotation response time at the doses of 15 mg/k and 30 mg/k (compared with those on Day 21), with a dose dependence. This indicates that after the animals develop resistance to L-DOPA/Benserazide, the acetate of Compound 22 can effectively restore or improve the original pharmaceutical effect of L-DOPA in drug-resistant animals.

The test results also show that the acetate of Compound 22 can also effectively prevent or slow down the resistance in the rats of 6-OHDA Parkinson's disease model to the L-DOPA/Benserazide.

In addition, compared with the model control group, the body weight of each rat in each test group does not change significantly during the test period, indicating that the acetate of Compound 22 as the test drug has no significant effect on the body weight of the test animal.

The model test shows that when patients with Parkinson's disease develop drug resistance due to long-term use of levodopa, levodopa may restore its therapeutic efficacy for these patients in the off stage of the Parkinson's disease by the combined use with the acetate of Compound 22. The use in claims for the treatment of Parkinson's disease finds a support.

**Example 10 Pharmacodynamic evaluation of subcutaneous homotransplantation of present compounds in murine colon cancer MC38 cell line of C57BL/6 female mouse model**

1. Test principle

The MC38 mouse colon cancer cell model is a commonly used model for verifying a pharmaceutical effect on the colon cancer. It is mostly used to study the occurrence and metastasis direction of the colorectal cancer, and has become a common approach to the pharmaceutical effect verification for tumors. The anti-tumor effect of the subcutaneous homotransplantation of a test drug in a murine colon cancer MC38 cell line of C57BL/6 female mouse model was evaluated.

2. Test design

| Group | Number of animals | Dosage group | Dose (mg/kg) | Administra tion mode | Planned dosage period | Actual dosage period |
|---|---|---|---|---|---|---|
| 1 | 10 | Vehicle control | 0 | p.o. | QD × 14 or 28 days | QD × 25 days |
| 2 | 10 | Anti-PD-1 | 100μg/ mouse | i.p. | Days 7, 9, 11, and 13 | Days 7, 9, 11, and 13 |
| 3 | 10 | Compound 22 | 30 | p.o. | QD × 14 or 28 days | QD × 25 days |
| 4 | 10 | Compound 22 | 30 | p.o. | QD × 14 or 28 days | QD × 25 days |
| | | Anti-PD-1 | 100μg/ mouse | i.p. | Days 7, 9, 11, and 13 | Days 7, 9, 11, and 13 |
| 5 | 10 | Compound 22 | 100 | p.o. | QD × 14 or 28 days | QD × 25 days |

3. Experiment materials and reagents

Experimental animals: C57BL/6 mice, female, 7-9 weeks old (the age of mice at the time of tumor cell inoculation), weight: 16.4-20.1 g, purchased from Shanghai Lingchang Biotechnology Co., Ltd. (LC), production license number: SCXK (Shanghai) 2013-0018, and animal certificate number: 2013001837385. Rearing environment: SPF level.

Anti-PD-1 antibody: batch number: 695318A1B, packaging specification: 18 mg, provided by BioXcell, colorless solution, stored at 4°C.

Preparation of solvent: 16.8 g of hydroxypropyl-beta-cyclodextrin (Zibo Qianhui Biotechnology Co., Ltd., batch number: 160101) was weighed, 42 ml of sterile water was added thereto, and after the hydroxypropyl-beta-cyclodextrin was completely dissolved, the resultant was set aside for later use.

4. Experimental method

MC38 cells were cultured in a DMEM culture solution containing 10% fetal bovine serum. The MC38 cells in the exponential growth phase were collected and resuspended in PBS to a suitable concentration for subcutaneous tumor inoculation in mice.
Female mice were subcutaneously inoculated with $1 \times 10^6$ MC38 cells on the right side. The day of inoculation was defined as Day 0. On the second day (Day 1) of MC38 cell inoculation, the mice were administered randomly according to body weight. The solution of Compound 22 was prepared at 3 mg/mL and 10 mg/mL respectively for later use.
Formula for calculating tumor volume: tumor volume ($mm^3$)=$1/2\times(a \times b^2)$ (a representing a long diameter and b representing a short diameter);
Tumor proliferation rate, T/C%, was a percentage ratio of tumor volume or tumor weight between the treatment group and the control group at a certain time point. It was calculated with a formula was as follows:

> $T/C\ \% = T_{TV} / C_{TV} \times 100\%$ ($T_{TV}$ (TTV: the average TV of the treatment group at a specific time point; $C_{TV}$: the average TV of the vehicle control group at a specific time point);
> Or $T/C\ \% = T_{TW} / C_{TW} \times 100\%$ ($T_{TW}$ (TTW: average tumor weight in the treatment group at the end of the experiment; $C_{TW}$: average tumor weight in the vehicle control group at the end of the experiment). The tumor inhibition rate, TGI (%), was calculated with a formula as follows: $TGI\% = (1-T/C) \times 100\%$. (T and C were the relative tumor volume (TV) or tumor weight (TW) at a specific time point in the treatment group and the control group, respectively).

All experimental results were represented as the average tumor volume ± SEM (mean standard error). Statistical analysis between different groups were conducted to select the optimal drug treatment point (usually after the last dosage). A comparison was conducted with the independent sample T test method to determine if there is a significant difference in tumor volume between the treatment group ad the control group. All data were analyzed with SPSS 18.0. $P < 0.05$ indicated a significant difference.

5. Experimental results and discussions

Table 28 Pharmaceutical effect of present compounds on mouse colon cancer model

| Experiment group | | Day 22 | | | |
|---|---|---|---|---|---|
| | | Tumor volume ( $\bar{x} \pm S$) | TGI (%) | T/C (%) | P Value (compared with the control group) |
| Group 1 | vehicle control group | 1839.95±166.98 | / | / | / |
| Group 2 | Anti-PD-1(100 μg/ animal) | 440.32±137.06 | 76 | 24 | <0.001 |
| Group 3 | Compound 22 (30 mg/kg) | 1205.60±259.65 | 34 | 66 | 0.055 |
| Group 4 | Anti-PD-1 (100 μg/ animal)<br>Compound 22 (30 mg/kg) | 268.89±57.10 | 85 | 15 | <0.001 |
| Group 5 | Compound 22 (100 mg/kg) | 951.28±116.30 | 48 | 52 | <0.001 |

The test results show that in the MC38 mouse colon cancer cell model, the test compound 22 shows a good tumor inhibition activity at the doses of 30 mg/kg and 100 mg/kg on Day 22, and the tumor inhibition rates (TGIs) are 34% and 48% respectively. The test compound 22 combined with the anti-PD-1 antibody also exhibits excellent anti-tumor activity in vivo, with the score of a tumor inhibition rate (TGI) of 85%, and no animal death or significant changes

in body weight are observed. The above experiment provides a support to the application of Compound 22, as a single agent or in combination with the anti-PD-1 antibody, to the corresponding indications of tumors.

**Example 11 Pharmacodynamic evaluation of present compounds in B16F10 murine melanoma model**

1. Test principle

The B16F10 murine melanoma model is a classic melanoma pharmaceutical effect evaluation model, which is made by subcutaneously inoculating the same B16F10 tumor cells into female C57BL/6 mice. The test compounds were administrated to evaluate their inhibitory effects on tumor growth in the model.

2. Experimental design

| Group | Numbe r of cases | Treatment | Dose (mg/kg)* | Dosage volume (mL/kg) | Route of administratio n | Administrati on frequency | Treatment course |
|---|---|---|---|---|---|---|---|
| 1 | 12 | Solvent control | - | 10 | p.o. | QD | 22 days |
| 2 | 12 | Anti PD-1 | 10 mg/kg | 5 | i.p. | 2/WK | 5 times |
| 3 | 12 | Compound 22 | 50 mg/kg | 10 | p.o. | QD | 25 days |
| 4 | 12 | Compound 22 | 50 mg/kg | 10 | p.o. | QD | 25 days |
| | | Anti PD-1 | 10 mg/kg | 5 | i.p. | 2/WK | 7 times |

3. Experiment materials and reagents

Experimental animals: species: rodent; strain: C57BL/6 mice; grade: SPF; age: 6-8 weeks; gender: female; weight: 18-22 g; laboratory animal provider: Charles River Laboratories in Beijing; production license number: SCXK (Beijing) 2016-0006; quality certificate number: 1100111911039475;

Anti PD-1 antibody: provider: Pharmaron (Beijing) New Pharmaceutical Technology Co., Ltd.; batch No.: 717919M1;

Vehicle: 40% hydroxypropyl-β-cyclodextrin (SIGMA, batch No.: Z08D9Y76902; weighing 120 g of HP-beta-CD and dissolving it with distilled water, and finally fixing the volume to 300 ml with a beaker to obtain a 40% hydroxypropyl-β-cyclodextrin solution).

4. Experimental method

B16F10 tumor cells were cultured in a DMEM medium containing inactivated 10% fetal bovine serum, 100 U/ml penicillin, 100 μg/ml streptomycin and 2 mM glutamine in an incubator at 37°C in the presence of 5% $CO_2$; and after the cells grew all over, the tumor cells were divided and passaged in respective flasks every 3 to 4 days. The tumor cells in the logarithmic growth phase were used for in vivo inoculation of tumors.

The B16F10 tumor cells resuspended in a serum-free DMEM culture solution were inoculated subcutaneously at $1\times10^5$/100 μl to the right ribs of experimental animals. In the second group, when the tumor grew to 75 $mm^3$, 12 animals with a uniform tumor volume were selected and administered according to the experimental design.

31.99 mg (purity: 93.79%) of Compound 22 were weighed out and added into 6 ml of 40% HP-beta-CD solution; and the resulting mixture was mixed well by vortexing and sonication, preparing a drug with the concentration of 5 mg/ml for later use.

0.418 ml of 7.18 mg/ml anti PD-1 was pipetted with a pipette and added into 1.082 ml of PH7.0 PBS solution; and the resulting mixture was gently inverted and mixed well, preparing a solution with the concentration of 2 mg/ml for later use.

The first day of tumor inoculation was D0, the first, third, and fourth groups were dosed on the second day of

tumor inoculation, and the second group was dosed when the tumor volume reached 75 $mm^3$.

Tumor volume: the tumor volume was measured 3 times a week with a vernier caliper, and the long and short diameters of the tumor were measured. The volume was calculated with a formula as follows:

$$\text{volume} = 0.5 \times \text{long diameter} \times \text{short diameter}^2.$$

Tumor growth inhibition rate (TGI, %):

$$\text{Tumor growth inhibition rate (TGI, \%)}=(1\text{-}T/C)\times 100.$$

$$TGI = 100\text{-}T/C \times 100,$$

T/C(%)=(average tumor volume of treatment groups on the current day-average tumor volume of treatment groups on the initial day D0)/( average tumor volume of the control group on the current day-average tumor volume of the control group on the initial day D0) × 100.

Survival data was analyzed with Graphpad Prism 8.0.

5. Experimental results and discussions

Table 29 Pharmaceutical effect of present compounds on tumor inhibition in mouse melanoma model

| Group | Treatment | Tumor volume ($mm^3$, Day 18) [a] | Tumor volume proliferated (%) ($mm^3$, Day 18) [a] | Tumor inhibition rate (%) [c] | *P* value [b] |
|---|---|---|---|---|---|
| 1 | Solvent control | 2,265 ± 288 | 3,032 ± 404 | - | - |
| 2 | Anti PD-1 10 mg/kg | 2,071 ± 199 | 2,822 ± 253 | 8.6 | 0.586 |
| 3 | Compound 22 (50 mg/kg) | 1,764 ± 314 | 2,425 ± 415 | 22.1 | 0.173 |
| 4 | Compound 22 (50 mg/kg) + Anti PD-1 10 mg/kg | 1,176 ± 199 | 1,750 ± 277 | 48.1 | 0.004 |
| [a] mean ± standard error; [b] vs. solvent control group. | | | | | |

As shown from the test results, in the B16F10 murine melanoma model, the treatment results on Day 18 after tumor inoculation show that the combined treatment group of Compound 22 and anti PD-1 (with an average tumor volume of 1176 $mm^3$) exhibits a significant anti-tumor effect (TGI = 48.1%, P = 0.004, vs. solvent control group); the treatment group of Compound 22 alone exhibits a greater anti-tumor trend (with an average tumor volume of 1764 $mm^3$, TGI = 22.1%, P = 0.173, vs. solvent control group); and the treatment group with anti-PD-1 alone (with an average tumor volume of 2071 $mm^3$, TGI=8.6%, P=0.586, vs. solvent control group) exhibits no anti-tumor effect, which is due to the fact that the model per se is an anti-PD-1 drug resistance model. No animal death or significant changes in body weight were observed seen in the experiment. The above experiment provides a support to the application of Compound 22, as a single agent or in combination with the anti-PD-1 antibody, to the corresponding indications of tumors.

**Example 12 Pharmacodynamic evaluation of present compounds in A20 murine lymphoma (B lymphocytes) homotransplantation model**

1. Experimental principle

A20 murine lymphoma (B lymphocyte) homotransplantation model is a classic pharmaceutical effect evaluation model for B lymphocyte cancer. It was constructed by subcutaneously inoculating homogeneous A20 tumor cells into the right ribs of female BALB/c mice by the same species. The test compounds were administrated to evaluate their inhibitory effects on tumor growth in the model.

2. Experimental design

| Group | Numbe r of cases | Treatment | Dose (mg/kg) | Dosage volume (mL/kg) | Route of administratio n | Administrati on frequency | Treatment course |
|---|---|---|---|---|---|---|---|
| 1 | 12 | Solvent control | - | 10 | p.o. | QD | 24 days |
| 2 | 12 | Compound 22 | 50 mg/kg | 10 | p.o. | QD | 24 days |

3. Experiment materials and reagents

Experimental animals: species: rodent; strain: BALB/c mice; grade: SPF; age: 6-8 weeks; gender: female; weight: 18-21g; laboratory animal provider: Charles River Laboratories in Beijing; production license number: SCXK (Beijing) 2016-0006; quality certificate number: 1100111911036383; Vehicle: 40% hydroxypropyl-β-cyclodextrin (SIGMA, batch No.: Z08D9Y76902; weighing 120 g of HP-beta-CD and dissolving it with distilled water, and finally fixing the volume to 300 ml with a beaker to obtain a 40% hydroxypropyl-β-cyclodextrin solution).

4. Experimental method

A20 tumor cells were cultured in an RPMI 1640 medium containing inactivated 10% fetal bovine serum, 100 U/ml penicillin, 100 μg/ml streptomycin and 0.05 mM 2-mercaptoethanol in an incubator at 37°C in the presence of 5% $CO_2$; and after the cells grew all over, the tumor cells were divided and passaged in respective flasks every 3 to 4 days. The tumor cells in the logarithmic growth phase were used for in vivo inoculation of tumors.

The A20 tumor cells resuspended in a serum-free RPMI 1640 culture solution were inoculated subcutaneously at $3 \times 10^5$/100 μl to the right ribs of experimental animals. A total of 30 animals were inoculated. On the first day after tumor cell inoculation, 24 animals with a uniform body weight were selected in terms of body weight, and were grouped for dosage, with 12 animals in each group. The specific dosage regimen was carried out according to the experimental design.

15.99mg (purity: 93.79%) of Compound 22 were weighed out and added into 3 ml of 40% HP-beta-CD solution; and the resulting mixture was mixed well by vortexing and sonication, preparing a 5 mg/mL solution of Compound 22 for later use.

Tumor volume: the tumor volume was measured 3 times a week with a vernier caliper, and the long and short diameters of the tumor were measured. The volume was calculated with a formula as follows:

$$\text{volume} = 0.5 \times \text{long diameter} \times \text{short diameter}^2.$$

Tumor growth inhibition rate (TGI, %):

$$\text{Tumor growth inhibition rate (TGI, \%)}=(1 - T/C)\times 100,$$

$$TGI = 100 - T/C \times 100;$$

T/C(%)=(average tumor volume of treatment groups on the current day-average tumor volume of treatment groups on the initial day D0)/( average tumor volume of the control group on the current day-average tumor volume of the control group on the initial day D0) × 100.

Statistical analysis of tumor volume was carried out between groups by the SPSS T-test.

5. Experimental results and conclusions

Table 30 Pharmaceutical effect of present compounds on tumor inhibition in mouse lymphoma model

| Group | Days after tumor inoculation | 6 | 8 | 10 | 13 | 15 | 17 | 20 | 22 | 24 |
|---|---|---|---|---|---|---|---|---|---|---|
| 1 | | - | - | - | - | - | - | - | - | - |
| 2 | TGI (%) | 11.9 | 8.4 | 7.0 | 16.5 | 29.4 | 32.6 | 28.5 | 36.4 | 35.6 |
| | P | 0.021* | 0.329 | 0.314 | 0.121 | 0.026* | 0.038* | 0.142 | 0.083 | 0.115 |

The test results show that the treatment group of Compound 22 (50 mg/kg) exhibits a significant anti-tumor trend, and a significant anti-tumor effect on Days 2, 15 and 17 after tumor inoculation (P < 0.05), with TGIs of 29.4% and 32.6% respectively. In addition, during the dosage, the experimental animals in each group were in good general conditions including activity, eating, etc.; no obvious clinical abnormalities were observed; and the overall body weight of the animals increased after treatment. The above experiment provides a support to the application of Compound 22, as a single agent, to the corresponding indications of tumors.

**Example 13 Pharmacodynamic evaluation of present compounds in CT26 murine colon cancer homotransplantation model**

1. Experimental principle

The CT26 murine colon cancer homotransplantation model is a classic pharmaceutical effect evaluation model for colon cancer. It is constructed by subcutaneously inoculating CT26 to BALB/c mice. The test compounds were administrated to evaluate their inhibitory effects on tumor growth in the model.

2. Experimental design

| Group | Number of animals | Dosage group | Dose (mg/kg) | Administra tion mode | Planned dosage period | Actual dosage period |
|---|---|---|---|---|---|---|
| 1 | 10 | Vehicle control | 0 | p.o. | QD × 14 or 28 days | QD × 25 days |
| 6 | 10 | Compound 22 | 100 | p.o. | QD × 14 or 28 days | QD × 25 days |

3. Experiment materials and reagents

Experimental animals: BALB/c mice, female, 7-8 weeks old (the age of mice at the time of tumor cell inoculation), weight: 17.4-23.5 g, 160 mice (100 mice plus another 60 surplus mice). They were purchased from Shanghai Lingchang Biotechnology Co., Ltd. (LC), with production license number: SCXK (Shanghai) 2013-0018, and animal certificate number: 2013001837385. Rearing environment: SPF level. ;
Solvent: 40% hydroxypropyl-β-cyclodextrin (Zibo Qianhui Biotechnology Co., Ltd., batch number: 160101; weighing 16.8 g of hydroxypropyl-β-cyclodextrin, adding 42 ml of sterile water thereto, and after the hydroxypropyl-beta-cyclodextrin was completely dissolved, setting the resultant aside for later use.).

4. Experimental method

CT26 cells were cultured in an RPMI1640 culture solution containing 10% fetal bovine serum. The CT26 cells in the exponential growth phase were collected and resuspended in PBS to a suitable concentration for subcutaneous tumor inoculation in mice.

Female mice were subcutaneously inoculated with $5 \times 10^5$ CT26 cells on the right side. The day of inoculation was defined as Day 0. On the second day (Day 1) of MC38 cell inoculation, the mice were administered randomly according to body weight.

64.35 mg of Compound 22 (60 mg free base) were weighed and added with 6.0 ml of solvent; the resulting mixture was vortexed and sonicated to obtain 6.0 ml of suspension, thereby preparing a 10 mg/mL solution of Compound 22 for later use.

Tumor proliferation rate, T/C%, was a percentage ratio of tumor volume or tumor weight between the treatment group and the control group at a certain time point. It was calculated with a formula was as follows:

T/C % = $T_{TV}$ / $C_{TV}$ $\times$ 100% ($T_{TV}$ (TTV: the average TV of the treatment group at a specific time point; $C_{TV}$: the average TV of the vehicle control group at a specific time point);

Or T/C % = $T_{TW}$ / $C_{TW}$ $\times$ 100% ($T_{TW}$ (TTW: average tumor weight in the treatment group at the end of the experiment; $C_{TW}$: average tumor weight in the vehicle control group at the end of the experiment). The tumor inhibition rate, TGI (%), was calculated with a formula as follows: TGI% = (1-T/C) $\times$ 100%. (T and C were the relative tumor volume (TV) or tumor weight (TW) at a specific time point in the treatment group and the control group, respectively).

All data were analyzed with SPSS 18.0.

5. Experimental results and conclusions

Table 31 Pharmaceutical effect of present compounds on tumor inhibition in mouse colon cancer model

| Experiment group | | Day 26 | | | |
|---|---|---|---|---|---|
| | | Tumor volume ($\bar{x}$ ±S) | TGI (%) | T/C (%) | P Value (compared with the control group) |
| Group 1 | vehicle control group | 2441.88±334.10 | -- | -- | -- |
| Group 2 | Compound 22 (100 mg/kg) | 1988.56±325.70 | 19 | 81 | 0.344 |

The test results show that the treatment group of Compound 22 (50 mg/kg) exhibits an anti-tumor effect on Day 26, with the TGI of 19%. In addition, during the dosage, the experimental animals in each group were in good general conditions including activity, eating, etc.; no obvious clinical abnormalities were observed. The above experiment provides a support to the application of Compound 22, as a single agent, to the corresponding indications of tumors.

**Example 14 Pharmacokinetics of present compounds in ICR mice**

**14.1 Pharmacokinetics of Compounds 27, 40, 22 and 41 in different modes of administration**

1. Experimental Information and Design

| Test compound | Route of administration | Vehicle | Administration dose/volume/ concent ration | Blood collection point |
|---|---|---|---|---|
| Compound 27 | IV (intravenous injection) | 10%DMAC/15%Sol utol HS 15/75%PBS | 1 mg/kg<br>5 mL/kg<br>0.2 mg/mL | 0.08, 0.25, 0.5, 1, 2, 4, 8, 24h |
| | PO (oral administration) | 10%DMAC/15%Sol utol HS 15/75%PBS | 5 mg/kg<br>10 mL/kg<br>0.5 mg/mL | 0.25, 0.5, 1, 2, 4, 8, 24h |
| Compound 40 | IV | 10%DMAC/15%Sol utol HS 15/75%PBS | 1 mg/kg<br>5 mL/kg<br>0.2 mg/mL | 0.08, 0.25, 0.5, 1, 2, 4, 8, 24h |
| | PO | 10%DMAC/15%Sol utol HS 15/75%PBS | 5 mg/kg<br>10 mL/kg<br>0.5 mg/mL | 0.25, 0.5, 1, 2, 4, 8, 24h |
| Compound 22 | IV | 10%DMAC/15%Sol utol HS 15/75%PBS (pH=7.4) | 1 mg/kg<br>5 mL/kg<br>0.2 mg/mL | 0.08, 0.25, 0.5, 1, 2, 4, 8, 24h |
| | PO | 10%DMAC/15%Sol | 5 mg/kg | 0.08, 0.25, 0.5, 1, 2, 4, |
| | | utol HS 15/75%PBS (pH=7.4) | 10 mL/kg<br>0.5 mg/mL | 8, 24h |
| Compound 41 | IV | 10%DMAC/15%Sol utol HS 15/75%PBS (pH=7.4) | 1 mg/kg<br>5 mL/kg<br>0.2 mg/mL | 0.08,0.25,0.5,1,2,4, 8, 24h |
| | PO | 10%DMAC/15%Sol utol HS 15/75%PBS (pH=7.4) | 5 mg/kg<br>10 mL/kg<br>0.5 mg/mL | 0.08,0.25,0.5,1,2,4, 8, 24h |
| Compound 27 | PO | 10%DMAC/15%Sol utol HS 15/75%PBS | 30 mg/kg<br>10 mL/kg<br>3 mg/mL | 0.25, 0.5, 1, 2, 4, 6, 8, 24h |
| Compound 22 | PO | 10%DMAC/15%Sol utol HS 15/75%PBS | 30 mg/kg<br>10 mL/kg<br>3 mg/mL | 0.25, 0.5, 1, 2, 4, 6, 8, 24h |
| Compound 40 | PO | 10%DMAC/15%Sol utol HS 15/75%PBS | 30 mg/kg<br>10 mL/kg<br>3 mg/mL | 0.25, 0.5, 1, 2, 4, 6, 8, 24h |

2. Calculation software

WinNonlin® 6.4 was used as the calculation software for PK parameters.

3. Results and conclusions

Table 32 Pharmacokinetic results of present compounds in ICR mice

| Test compound | Administration mode | Administration dose (mg/kg) | $T_{1/2}$ | $C_{max}$ | $AUC_{last}$ | $Cl_{_pred}$ | $Cl_{_F_pred}$ | F (%) |
|---|---|---|---|---|---|---|---|---|
| | | | h | ng/mL | hr*ng/mL | L/h/kg | L/h/kg | |
| Compound 27 | IV | 1 | 0.67 | 993.2 | 494.7 | 2.03 | / | / |
| | PO | 5 | 1.19 | 1551.7 | 3098.8 | / | 1.65 | 125.29 |
| Compound 40 | IV | 1 | 2.49 | 1878.6 | 1537.9 | 0.65 | / | / |
| | PO | 5 | 2.36 | 2134.7 | 5535.7 | / | 0.90 | 71.99 |
| Compound 22 | IV | 1 | 0.94 | 1689.9 | 924.6 | 1.09 | / | / |
| | PO | 5 | 2.74 | 3511.4 | 3962.8 | / | 1.26 | 85.72 |
| Compound 41 | IV | 1 | 0.28 | 2232.2 | 1108.5 | 0.91 | / | / |
| | PO | 5 | 1.85 | 1300.1 | 1801.8 | / | 2.92 | 32.51 |
| Compound 27 | PO (single) | 30 | 1.12 | 14530.5 | 33609.5 | / | 0.92 | / |
| | PO (after 7 consecutive days of administration) | 30 | 1.86 | 17341.0 | 41741.7 | / | 0.72 | / |
| Compou | PO (single) | 30 | 2.22 | 9654.7 | 17955.8 | / | 1.67 | / |
| nd 22 | PO (after 7 consecutive days of administration) | 30 | 3.04 | 4801.5 | 15383.9 | / | 1.95 | / |
| Compound 40 | PO (single) | 30 | 1.65 | 14361.0 | 24414.1 | / | 1.23 | / |
| | PO (after 7 consecutive days of administration) | 30 | 2.79 | 2326.2 | 10621.5 | / | 2.82 | / |

The experimental results show that the compounds in the list have excellent pharmacokinetic performance in the ICR mice. Compounds 27, 40, 22 and 41 exhibit higher in vivo exposure and higher bioavailability after oral administration. Compounds 27, 22 and 40 exhibit higher in vivo exposure after oral administration, and significant drug accumulation is not observed after consecutive oral administration of Compounds 27, 22 and 40 at the dose of 30 mg/kg.

**14.2 Pharmacokinetics of Compound 40 and benzene sulfonate thereof in ICR mice**

1. Experimental information

| | |
|---|---|
| Species: | ICR mice |
| Test compound: | Benzene sulfonate of Compound 40 |
| Route of administration: | PO (oral administration) |
| Vehicle blank control: | 40%Hp-β-CD |
| Solvent properties: | White suspension |
| Administration dose: | 100 mg/kg |
| Dosage volume: | 100 mL/kg |
| Dosage concentration: | 10 mg/mL |
| LLOQ: | 1 ng/mL |
| PK parameter calculation software | WinNonlin® 7.0 |

2. Experimental design

| Group | Test compound | Administration dose | Blood collection point |
|---|---|---|---|
| Group 1 | Compound 40 | 100 mg/kg | 0.25, 0.5, 1, 2, 4, 8, 24h |
| Group 2 | Benzene sulfonate of Compound 40 | 100 mg/kg (based on free base) | 0.25, 0.5, 1, 2, 4, 8, 24h |

3. Experimental results and conclusions

Table 33 Pharmacokinetic results of present compounds in ICR mice

| Group | T1/2, h | $T_{max}$ (h) | $C_{max}$ (ng/mL) | $AUC_{last}$ (h*ng/mL) | $AUC_{INF_pred}$ (h*ng/mL) | $Cl_{_F_pred}$ (L/h/kg) |
|---|---|---|---|---|---|---|
| Group 1 | 4.27 | 0.50 | 2897.3 | 11936.0 | 12129.3 | 0.82 |
| Group 2 | 2.29 | 0.50 | 36006.0 | 138326.1 | 138424.0 | 0.07 |

The data in Table 33 show that the test compound exhibits excellent pharmacokinetic parameters in the ICR mice. The in vivo exposures of Compound 40 and benzene sulfonate thereof are higher.

**Example 15 Pharmacokinetics of present compounds in SD rats**

**15.1 Pharmacokinetics of Compounds 27, 16, 14 and 22 in SD rats**

1. Experimental Information and Design

| Test compound | Route of administration | Vehicle | Administration dose/ volume/concent ration | Blood collection point |
|---|---|---|---|---|
| Compound 27 | IV | 10%DMAC/15%Sol utol HS 15/75%PBS | 1 mg/kg<br>5 mL/kg<br>0.2 mg/mL | 0.08, 0.25, 0.5, 1, 2, 4, 8, 24h |
| | PO | 10%DMAC/15%Sol utol HS 15/75%PBS | 5 mg/kg<br>10 mL/kg<br>0.5 mg/mL | 0.25, 0.5, 1, 2, 4, 8, 24h |
| Compound 16 | IV | 10%DMAC/15%Sol utol HS 15/75%PBS | 1 mg/kg<br>5 mL/kg<br>0.2 mg/mL | 0.08, 0.25, 0.5, 1, 2, 4, 8, 24h |
| | PO | 10%DMAC/15%Sol utol HS 15/75%PBS | 10 mg/kg<br>10 mL/kg<br>1 mg/mL | 0.08, 0.25, 0.5, 1, 2, 4, 8, 24h |
| Compound 14 | IV | 10%DMAC/15%Sol utol HS 15/75%PBS | 1 mg/kg<br>5 mL/kg<br>0.2 mg/mL | 0.08, 0.25, 0.5, 1, 2, 4, 8, 24h |
| | PO | 10%DMAC/15%Sol utol HS 15/75%PBS | 10 mg/kg<br>10 mL/kg<br>1 mg/mL | 0.08,0.25,0.5,1,2,4, 8, 24h |

(continued)

| Test compound | Route of administration | Vehicle | Administration dose/ volume/concent ration | Blood collection point |
|---|---|---|---|---|
| Compound 22 | IV | 10%DMAC/15%Sol utol HS 15/75%PBS(pH=7.4) | 1 mg/kg<br>5 mL/kg<br>0.2 mg/mL | 0.08, 0.25, 0.5, 1, 2, 4, 8, 24h |
| | PO | 10%DMAC/15%Sol utol HS 15/75%PBS(pH=7.4) | 3 mg/kg<br>10 mL/kg<br>0.3 mg/mL | 0.08, 0.25, 0.5, 1, 2, 4, 8, 24h |

2. Calculation software

WinNonlin® 6.4 was used as the calculation software for PK parameters.

3. Results and conclusions

Table 34 Pharmacokinetic results of present compounds in SD rats

| Test compound | Administratio n mode | Administratio n dose | $T_{1/2}$ | $C_{max}$ | $AUC_{last}$ | $Cl_{_pred}$ | $Cl_{_F_pred}$ | F (%) |
|---|---|---|---|---|---|---|---|---|
| | | | h | ng/mL | hr*ng/mL | L/h/kg | L/h/kg | |
| Compound 27 | IV | 1 | 0.51 | 1341.0 | 476.6 | 2.16 | / | / |
| | PO | 5 | 4.36 | 318.1 | 484.0 | / | 8.82 | 33.85 |
| Compound 16 | IV | 1 | 0.61 | 986.3 | 535.6 | 1.89 | / | / |
| | PO | 10 | 4.78 | 258.1 | 758.3 | / | 13.98 | / |
| Compound 14 | IV | 1 | 0.44 | 1680.6 | 678.0 | 1.48 | / | / |
| | PO | 10 | 3.05 | 573.7 | 1213.6 | / | 8.66 | 17.90 |
| Compound 22 | IV | 1 | 1.00 | 2347.5 | 1768.5 | 0.57 | / | / |
| | PO | 3 | 3.30 | 580.2 | 2095.1 | / | 1.52 | 39.49 |

The experimental results show that the compounds in the list have excellent pharmacokinetic performance in SD rats. The $T_{1/2}$ of intravenous injection is about 0.5-1 h, and the $T_{1/2}$ of oral administration is about 3-5 h, exhibiting higher in vivo exposure and higher bioavailability after oral administration.

**15.2 Table 34 Pharmacokinetics of test compounds in SD rats**

1. Test design

Administration dose:

| | |
|---|---|
| Compound name | Compounds 40, 27 and 47 |
| Vehicle | Orally administrated vehicle: 40% PEG400/60%PBS |
| Administration dose | Compound 40: IV, 1 mg/kg; PO, 10 mg/kg<br>Compound 27: IV, 1 mg/kg; PO, 10 mg/kg<br>Compound 40: IV, 1.64 mg/kg; PO, 2.36 mg/kg<br>Compound 47: PO, 3 mg/kg |

Administration dose: the compounds in this group were all administered in a mixed way, with 6 compounds in each group for mixed PO administration.

| Test compound | Route of administration | Vehicle | Administration dose | Blood collection point |
|---|---|---|---|---|
| Compound 17 | PO | 40% PEG400/60% PBS in water | 2 mg/kg | 0.25, 0.5, 1, 2, 4, 8 |
| Compound 18 | PO | 40% PEG400/60% PBS in water | 2 mg/kg | 0.25, 0.5, 1, 2, 4, 8 |
| Compound 21 | PO | 40% PEG400/60% PBS in water | 2 mg/kg | 0.25, 0.5, 1, 2, 4, 8 |
| Compound 23 | PO | 40% PEG400/60% PBS in water | 2 mg/kg | 0.25, 0.5, 1, 2, 4, 8 |
| Compound 25 | PO | 40% PEG400/60% PBS in water | 2 mg/kg | 0.25, 0.5, 1, 2, 4, 8 |
| Compound 28 | PO | 40% PEG400/60% PBS in water | 2 mg/kg | 0.25, 0.5, 1, 2, 4, 8 |
| Compound 29 | PO | 40% PEG400/60% PBS in water | 2 mg/kg | 0.25, 0.5, 1, 2, 4, 8 |
| Compound 35 | PO | 40% PEG400/60% PBS in water | 2 mg/kg | 0.25, 0.5, 1, 2, 4, 8 |
| Compound 37 | PO | 40% PEG400/60% PBS in water | 2 mg/kg | 0.25, 0.5, 1, 2, 4, 8 |
| Compound 48 | PO | 40% PEG400/60% PBS in water | 2 mg/kg | 0.25, 0.5, 1, 2, 4, 8 |
| Compound 44 | PO | 40% PEG400/60% PBS in water | 2 mg/kg | 0.25, 0.5, 1, 2, 4, 8 |
| Compound 40 | PO | 40% PEG400/60% PBS in water | 2 mg/kg | 0.25, 0.5, 1, 2, 4, 8 |
| Compound 41 | PO | 40% PEG400/60% PBS in water | 2 mg/kg | 0.25, 0.5, 1, 2, 4, 8 |
| Compound 42 | PO | 40% PEG400/60% PBS in water | 2 mg/kg | 0.25, 0.5, 1, 2, 4, 8 |
| Compound 43 | PO | 40% PEG400/60% PBS in water | 2 mg/kg | 0.25, 0.5, 1, 2, 4, 8 |
| Compound 44 | PO | 40% PEG400/60% PBS in water | 2 mg/kg | 0.25, 0.5, 1, 2, 4, 8 |
| Compound 45 | PO | 40% PEG400/60% PBS in water | 2 mg/kg | 0.25, 0.5, 1, 2, 4, 8 |
| Compound 46 | PO | 40% PEG400/60% PBS in water | 2 mg/kg | 0.25, 0.5, 1, 2, 4, 8 |
| Compound 47 | PO | 40% PEG400/60% PBS in water | 2 mg/kg | 0.25, 0.5, 1, 2, 4, 8 |
| Compound 48 | PO | 40% PEG400/60% PBS in water | 2 mg/kg | 0.25, 0.5, 1, 2, 4, 8 |
| Compound 49 | PO | 40% PEG400/60% PBS in water | 2 mg/kg | 0.25, 0.5, 1, 2, 4, 8 |

(continued)

| Test compound | Route of administration | Vehicle | Administration dose | Blood collection point |
|---|---|---|---|---|
| Compound 50 | PO | 40% PEG400/60% PBS in water | 2 mg/kg | 0.25, 0.5, 1, 2, 4, 8 |
| Compound 51 | PO | 40% PEG400/60% PBS in water | 2 mg/kg | 0.25,0.5,1,2,4,8 |
| Compound 52 | PO | 40% PEG400/60% PBS in water | 2 mg/kg | 0.25, 0.5, 1, 2, 4, 8 |

Detection model and detection method

| Species/strains | Rat/Sprague Dawley | |
|---|---|---|
| Number and gender of animals in each group | 3, male | |
| Internal standard | Verapamil | |
| Analysis method | Protein precipitation, using the LC-MS/MS method | |
| Time point for pharmacokineti cs | IV: 0.08, 0.25, 0.50, 1.00, 2.00, 4.00, 8.00, 24.00h<br>PO: 0.08, 0.25, 0.50, 1.00, 2.00, 4.00, 8.00, 24.00h | PO: 0.25, 0.50, 1.00, 2.00, 4.00, 8.00h |

2. Test Materials and equipment

1) Main reagents:

| Reagent name | Source | Batch No. |
|---|---|---|
| Formic acid | Aladdin | F1627037 |
| Acetonitrile | Merck KGaA Co. | JA050830 |
| PEG400 | Macklin Chemical Reagent Co., Ltd. | C10083707 |
| Verapamil | Aladdin | K1629079 |

2) Main instruments

| Instrument name | Model | Manufacturer | Instrument No. |
|---|---|---|---|
| Multi-functional vortex mixer | Vortex4 | IKA Germany | FX03025 |
| Ultrasonic cleaner | S22H | Zealway, Xiamen, CN | FX03020 |
| LC/MS (Compounds 40, 27, 40 and 47) | Qtrap 5500 | AB Sciex | FX03001 |
| LC/MS (Compounds 44 and 48) | TQS | Waters | - |

3. Analysis method

1) Liquid-phase conditions

Column: ACQUITY UPLC BEH C18 (2.1 mm × 50 mm, 1.7 μm)
Flow rate: 0.4 mL/min for Compounds 40, 27, 40 and 47, Column temperature: 40°C, Injection volume: 5 μL; 0.3 mL/min for Compounds 44 and 48, Column temperature: 35°C, Injection volume: 1 μL. Compounds 40, 27, 40 and 47: Mobile phase A: water (0.1% formic acid), Mobile phase B: acetonitrile (0.075% formic acid);
Compounds 44 and 48: Mobile phase A: water (0.1% formic acid), Mobile phase B: acetonitrile (0.1% formic acid).
Gradient elution. The elution procedure can be found in the table below.

| Time (min) | | Phase A (%) | Phase B (%) |
|---|---|---|---|
| 0.00 | 0.00 | 90 | 10 |
| 0.50 | 0.50 | 90 | 10 |
| 1.50 | 1.50 | 10 | 90 |
| 3.00 | 2.5 | 10 | 90 |
| 3.10 | 2.51 | 90 | 10 |
| 4.00 | 3.5 | 90 | 10 |

2) MS conditions

Electrospray ion source (Turbo spray), positive ion detection mode, and multiple reaction monitoring (MRM) scan mode selected for mass spectrometry analysis. The ion source parameters for mass spectrometry and the detection parameters of compounds are shown in the table below.

| CUR/kPa Curtain gas | GAS1/kPa Atomized gas | GAS2/kPa Auxiliary gas | TEM/°C Ion source temperature | IS/V EFI voltage | CAD/V Collision gas |
|---|---|---|---|---|---|
| 25 | 50 | 55 | 450 | 5500 | 8 |
| Capillary voltage (kv) | Cone voltage (V) | Desolvent temperature (°C) | Desolvent (L/hr) | Cone (L/hr) | Collision gas flow (mL/Min) |
| 3.50 | 11 | 500 | 550 | 150 | 0.15 |

4. Test Method

Blood was collected based on the blood collection points in the test protocol. 50 μL of plasma sample was added with acetonitrile (containing 5 ng/mL Verapamil) being 3 folds of the volume thereof; the resulting mixture was vortexed for 30 s, and then centrifuged for 15 min at 15,000 rpm at 4°C; and 10μ L of supernatant was taken for LC-MS/MS analysis.

The compounds were prepared based on the vehicle in the test protocol.

For each compound, 3 male SD rats weighing 200-220 g were fasted for one night before dosage, but had access to water.

Orally intragastric administration: before intragastric administration and at 0.083 h, 0.25 h, 0.5 h, 1 h, 2 h, 4 h, 8 h and 24 h after administration (for Compounds 40, 27, 40 and 47), or at 0.25 h, 0.5 h, 1 h, 4 h, 6 h and 8 h after administration (for Compounds 44 and 48), orbital blood was collected and put in a test tube that was treated with heparin sodium, and after centrifugation, the supernatant plasma was collected for LC-MS/MS analysis.

The plasma samples were processed according to the method for "sample treatment" and then analyzed by LC-MS/MS, accompanied with the standard curve and quality control samples. At a time point when the upper limit of quantification was exceeded, the samples were diluted 10 or 100 folds for analysis. All measured data were collected and processed by Analyst 1.6.3 software, and the data were calculated and processed with Microsoft Excel. With DAS 3.2.8 software, the pharmacokinetic parameters were calculated by the statistical moment method. The pharmacokinetic

parameters mainly include $T_{max}$, $t_{1/2}$, $C_{max}$, $AUC_{(0-t)}$ , etc.

5. Test results and conclusions

Table 35 Pharmacokinetic results of present compounds in SD rats

| Test compound | Adminis tration mode | Administratio n dose (mg/kg) | T1/2 | Cmax | AUClast | Cl_pred | Cl_F_ pred | F (%) |
|---|---|---|---|---|---|---|---|---|
| | | | h | ng/mL | hr*ng/mL | mL/h/kg | mL/h/kg | |
| Compound 40 | IV | 1 | 0.78 | 1281.99 | 1406.02 | 710.04 | / | / |
| | PO | 10 | 2.44 | 2439.21 | 6604.17 | / | 1375.37 | 46.97 |
| Compound 27 | IV | 1 | 0.47 | 139.17 | 83.82 | 11331.4 | / | / |
| | PO | 10 | 2.42 | 325.40 | 1068.7 | / | 8344.15 | 127.5 |
| Compound 40 | IV | 1.64 | 0.53 | 1314.36 | 1006.5 | 1622.87 | / | / |
| | PO | 2.36 | 0.23 | 303.44 | 1685.73 | / | 1395.24 | 116.3 |
| Compound 47 | PO | 3 | 2.25 | 537.58 | 1496.3 | / | 1389.74 | / |

Table 36 Pharmacokinetic results of present compounds in SD rats

| Test compound | Administration mode | Administration dose (mg/kg) | $AUC_{last}$ |
|---|---|---|---|
| | | | hr*ng/mL |
| Compound 17 | PO | 2 | 730 |
| Compound 18 | PO | 2 | 441 |
| Compound 21 | PO | 2 | 603 |
| Compound 23 | PO | 2 | 325 |
| Compound 25 | PO | 2 | 142 |
| Compound 28 | PO | 2 | 757 |
| Compound 29 | PO | 2 | 160 |
| Compound 35 | PO | 2 | 1604 |
| Compound 37 | PO | 2 | 1833 |
| Compound 48 | PO | 2 | 596.99 |
| Compound 44 | PO | 2 | 340.61 |
| Compound 40 | PO | 2 | 2363.07 |
| Compound 41 | PO | 2 | 1891.16 |
| Compound 42 | PO | 2 | 817.59 |
| Compound 43 | PO | 2 | 314.98 |
| Compound 45 | PO | 2 | 717.52 |
| Compound 46 | PO | 2 | 739.16 |
| Compound 47 | PO | 2 | 779.45 |
| Compound 51 | PO | 2 | 420.09 |
| Compound 52 | PO | 2 | 387.63 |

The experimental results in Table 35 and Table 36 show that the compounds in the list have excellent pharmacokinetic performance in SD rats, with higher $AUC_{last}$ values, exhibiting higher in vivo exposure and higher bioavailability after oral administration. The excellent pharmacokinetic properties in rats for the compounds in the list can provide a support to the further clinical experiment on human beings.

**15.3 Pharmacokinetics of acetate of Compound 22 in SD Rats with single and repeated administration**

1. Experimental design

| | |
|---|---|
| Compound name | Acetate of compound 22 |
| Batch No. | A05753-043A7 |
| Content | 97.8% |
| Vehicle | Intravenous vehicle: 10% 10%DMAC/15% Solutol HS 15/75% PBS (pH=7.4) Oral vehicle: acetic acid-sodium acetate (pH = 4.0) buffer containing 0.5% MC |
| Administration dose | Single administration: 2.0 mg/kg for intravenous injection; 21 mg/kg, 63 mg/kg, and 189 mg/kg for oral administration Repeated administration (7 days): 63 mg/kg for oral administration |
| Species/strains | Rat/Sprague Dawley |
| Number and gender of animals in each group | 3 males/3 females |
| Analytical matrix (anticoagulant) | Plasma [Potassium (K2) EDTA] |
| Internal standard | Verapamil |
| Analysis method | Protein precipitation, using the verified LC-MS/MS method |
| Lower limit of quantification | 2.00 ng/mL |
| Breadth of standard curve | 2.00 - 1000 ng/mL |
| Time point for pharmacokinetics | Single administration, 2.0 mg/kg intravenous injection group (Group 1): before administration, and 5, 15, 30 minutes and 1, 2, 4, 8 and 24 hours after administration Single administration, 21-189 mg/kg oral administration group (Groups 2-4): before administration, and 15, 30 minutes and 1, 2, 4, 6, 8 and 24 hours after administration Repeated administration, 63 mg/kg oral administration group (Group 5): Day 1: before administration, and 15, 30 minutes and 1, 2, 4, 6, 8 and 24 hours after administration<br>Days 4 and 5: 24 hours after administration<br>Day 7: before administration, and 15, 30 minutes and 1, 2, 4, 6, 8 and 24 hours after administration |
| Pharmacokinetic analysis | Non-compartmental model |
| GLP/Non-GLP | Non-GLP |

Administration information is shown below:

| Group | Test compound | Administrati on dose (mg/kg) | Dosage volume (mL/kg) | Concentration (mg/mL) | Route of administrati on | Number of animals (MALE/ FEMA LE) |
|---|---|---|---|---|---|---|
| 1 | ACETATE OF COMPOUND 22 [A] | 2.0 | 5.0 | 0.4 | Intravenous injection | 3/3 |
| 2 | ACETATE OF COMPOUND 22 [B] | 21 | 10 | 2.1 | Oral administrati on | 3/3 |
| 3 | ACETATE OF COMPOUND 22 [B] | 63 | 10 | 6.3 | Oral administrati on | 3/3 |
| 4 | Acetate of compound 22 [b] | 189 | 10 | 18.9 | Oral administrati on | 3/3 |
| 5 | Acetate of compound 22 [b] | 63 | 10 | 6.3 | Oral administrati on | 3/3 |
| a: The vehicle was 10% DMAC/15% Solutol HS 15/75% PBS (pH=7.4).<br>b: The solvent was 0.5%acetic acid-sodium acetate (pH=4.0) buffer containing 0.5% MC. | | | | | | |

2. Experiment materials

Animal information:

| | |
|---|---|
| Species/strain/grade | Sprague-Dawley (SD) rats [Crl: CD (SD)] (SPF/VAF) |
| Source | Charles River Laboratories in Beijing |
| Age at first dose | 7-9 weeks |
| Number of animals | 15 females and 15 males plus 5 females and 5 males as spare |
| Animal transfer time | August 13, 2019 |
| Animal quality certificate No. | 1100111911037824 (male)<br>1100111911037825 (female) |
| Experimental animal production license | SCXK (Beijing) 2016-0006 |
| Body weight at first dose | Male: 273.3-330.2 g, female: 210.7-250.4 g |

Experimental reagents:

| Designation | Source | Batch No. |
|---|---|---|
| DMAC | SIGMA | BCBT8964 |
| Solutol HS 15 | BASF SE | 69889088QO |
| PBS | GIBCO | 1948177 |
| Glacial acetic acid | HOheywell | SZBF2670V |
| Sodium acetate trihydrate | SIGMA | BC13W4910 |
| Methylcellulose | SIGMA | SLCB1319 |
| Deionized water | Pharmaron Laboratory | 08142019 |

3. Experimental method

1) Preparation of formulations

Preparation of formulation for intravenous injection: 7.13 mg of the acetate of Compound 22 was accurately weighed and put into a container; 1.5 mL of DMAC was added to the container; the resulting mixture was vortexed and mixed until complete dissolution; 2.25 mL of Solutol HS 15 was added to the mixture and vortexed; then, 11.25 mL of PBS was added to fix the volume to a final volume (15 mL); and the mixture was vortexed for 1 min to obtain a clear solution (pH=3.96) with a final concentration of 0.4 mg/mL.

Preparation of formulation for oral administration groups (Groups 2-5): in the second group, 69.91 mg of the acetate of Compound 22 was accurately weighed and put into a container; 20 mL of acetic acid-sodium acetate (pH=4.0) buffer containing 0.5% MC was added to the container; and the resulting mixture was stirred for 66 minutes and then sonicated for 30 minutes to fix the volume to a final volume (28 mL), thereby obtaining a clear solution (PH=3.99) with a final concentration of 2.1 mg/mL. In the third group, 209.7 mg of the acetate of Compound 22 was accurately weighed and put into a container; 20 mL of acetic acid-sodium acetate (pH=4.0) buffer containing 0.5% MC was added to the container; and the resulting mixture was stirred for 66 minutes and then sonicated for 30 minutes to a final volume (28 mL), thereby obtaining a clear solution (pH=4.15) with a final concentration of 6.3 mg/mL. In the fourth group, 629.2 mg of the acetate of Compound 22 was accurately weighed and put into a container; 20 mL of acetic acid-sodium acetate (pH=4.0) buffer containing 0.5% MC was added to the container; and the resulting mixture was stirred for 66 minutes and then sonicated for 30 minutes to a final volume (28 mL), thereby obtaining a clear solution (pH=4.35) with a final concentration of 18.9 mg/mL. In the fifth group, 1348.3 mg of the acetate of Compound 22 was accurately weighed and put into a container; 150 mL of acetic acid-sodium acetate (pH=4.0) buffer containing 0.5% MC was added to the container; and the resulting mixture was stirred for 66 minutes and then sonicated for 30 minutes to a final volume (180 mL), thereby obtaining a clear solution (pH=4.15) with a final concentration of 6.3 mg/mL. The formulations to be administrated were freshly prepared under yellow light prior to daily administration. The formulation for Group 5 was prepared before administration on Day 1, and then, divided into daily doses and stored at 5±3°C. After taking out the formulations on the day of administration, the formulations should be let stand at room temperature for at least 30 minutes before administration.

2) Dosing

The administration was carried out following the experimental design protocol.

3) Sample processing

After blood collection, the blood collection tube containing the anticoagulant was turned upside down several times to mix well, and placed on wet ice before centrifugation. Within 60 minutes after blood collection, the samples were centrifuged at 2000 g for 10 minutes at 2-8°C to separate red blood cells, thereby obtaining plasma samples. The plasma samples were transferred to cryovials and stored at -75 ± 15 °C until analysis.

Blood samples were collected from SD rats at room temperature before administration and within 24 hours after administration. All collected samples were placed on wet ice before centrifugation and stored at -75 ± 15 °C until analysis.

Sample processing method:

| Sample | Blank plasma (μL) | Plasma sample (μL) | Standard curve sample and quality control sample (μL) | Internal standard working solution (μL) | 0.1% formic acid in acetonitrile (μL) |
|---|---|---|---|---|---|
| Double blank sample | 25 | NA | NA | NA | 400 |
| Zero sample | 25 | NA | NA | 400 | NA |
| Standard curve sample | NA | NA | 25 | 400 | NA |
| and quality control sample | | | | | |

(continued)

| Sample | Blank plasma (μL) | Plasma sample (μL) | Standard curve sample and quality control sample (μL) | Internal standard working solution (μL) | 0.1% formic acid in acetonitrile (μL) |
|---|---|---|---|---|---|
| Pharmacokinetic sample | NA | 25 | NA | 400 | NA |
| NA: Not applicable<br>(1). The required volume was transferred to a 1.1 mL plastic tube according to the table above.<br>(2). The sample was mixed well with a vortexer, shook for 10 minutes, and centrifuged for 10 minutes at 4000 rpm and 4°C.<br>(3). After centrifugation, 20 μL of supernatant was transferred to a 1.1 mL plastic tube containing 140 μL of diluent and vortexed to mix well, and 60 μL of the mixed sample was transferred to a 1.1 mL plastic tube containing 100 μL of diluent and vortexed to mix well.<br>(4). 160 μL of the sample was transferred to a 96-well plate.<br>(5). 5.0 μL of the sample was injected into the LC-MS/MS. | | | | | |

4) Analysis methods and conditions

| Liquid-phase conditions: | | | | |
|---|---|---|---|---|
| Instrument | SHMADZU LC; Racker Changer II Autosampler | | | |
| Column | YMC-Triart C18-(2.1 mm × 50 mm, 5 μm) | | | |
| Column temperature | 4 °C | | | |
| Analysis time | 4.50 min | | | |
| Mobile phase | A: 0.1% acetic acid in deionized water | | | |
| | B: 0.1% acetic acid in acetonitrile | | | |
| Injection volume | 5.0 μL | | | |
| Holding time | Acetate of Compound 22 0.9 min | | | |
| | Verapamil 1.3 min | | | |
| Elution gradient | Time (minute) | A (%) | B (%) | Flow rate (mL/min) |
| | 0 | 70.0 | 30.0 | 0.600 |
| | 0.10 | 70.0 | 30.0 | 0.600 |
| | 0.50 | 70.0 | 30.0 | 0.600 |
| | 1.20 | 50.0 | 50.0 | 0.600 |
| | 1.60 | 10.0 | 90.0 | 0.600 |
| | 2.00 | 10.0 | 90.0 | 0.600 |
| | 2.50 | 70.0 | 30.0 | 0.600 |
| | 3.00 | 10.0 | 90.0 | 0.600 |
| | 3.50 | 10.0 | 90.0 | 0.600 |
| | 4.00 | 70.0 | 30.0 | 0.600 |
| | 4.50 | 70.0 | 30.0 | 0.600 |
| Autosampler temperature | 4 °C | | | |

(continued)

| MS conditions: | |
|---|---|
| Instrument | Applied biosystems/AB Sciex Triple Quad 4500 |
| Ion source | ESI |
| Ion mode | Positive |
| Detection mode | MRM |
| Voltage | 5000V |
| Temperature | 550 °C |

Mass spectrometry parameters

| Compound | Transition (*m*/*z*) | Dwell Time (*ms*) | DP *(volts)* | CE *(volts)* | EP *(volts)* | CXP *(volts)* |
|---|---|---|---|---|---|---|
| Acetate of compound 22 | 393.2→146.0 | 100 | 100 | 43 | 12 | 8 |
| Verapamil | 455.2→165.1 | 100 | 100 | 42 | 12 | 15 |
| Peak integration method: | Peak area | | | | | |

The concentration of the acetate of Compound 22 in the plasma of SD rats was determined by liquid chromatography-mass spectrometry, with verapamil as the internal standard compound. The mass spectrometer was Applied Biosystems/AB Sciex Triple Quad 4500. By scanning in the electrospray ion source positive ion mode, the mass-to-charge ratios of parent ions to daughter ions in the acetate of Compound 22 and in the verapamil were 393.2→146.0 and 455.2→165.0, respectively.

The pharmacokinetic parameters of the acetate of Compound 22 were calculated by the software Phoenix™ WinNonlin® (version 8.1) using the non-compartmental model. Pharmacokinetic data was calculated using the linear logarithmic trapezoidal method, with a weight of 1/(Y*Y). Samples with the sample concentration below the lower limit of quantification were excluded from the calculation of pharmacokinetic parameters. The mean value of each parameter was calculated using Microsoft Excel version 2010, and the mean value was determined from the pharmacokinetic parameters of each animal. The mean time to peak ($T_{max}$) was represented as a median.

4. Experimental results and conclusions

Table 37 Main pharmacokinetic parameters in vivo after administration of acetate of Compound 22 to SD rats (mean ± standard deviation)

| Group | Admin istratio n dose | Gen der | $C_0$ | $T_{1/2}$ | $T_{max}^a$ | $C_{max}$ | $AUC_{0-t}$ | CL | Vss | F |
|---|---|---|---|---|---|---|---|---|---|---|
| | (mg/kg ) | | (ng /mL) | (hr) | (hr) | (ng /mL) | (hr*ng/mL) | (mL/min/kg ) | (L/kg) | (%) |
| 1 (IV) | 2.0 | Mal e | 7055±699 | 1.43 (-) | NA | NA | 4873±601 | 6.56* | 0.445* | NA |
| | | Fe mal e | 8142±820 | 4.70±3.22 | NA | NA | 5853±149 | 5.66±0.166 | 0.465±0.122 | NA |
| | | Ove rall | 7598±905 | 3.39±2.90 | NA | NA | 5363±664 | 6.02±0.668 | 0.457±0.088 | NA |
| 2 (PO) | 21 | Mal e | NA | 3.21±0.304 | 0.5 | 16633±4100 | 42043±10546 | NA | NA | 82.2±20.6 |
| | | Fe mal e | NA | 3.73±0.771 | 0.5 | 20100±3859 | 43486±4146 | NA | NA | 70.8±6.75 |
| | | Ove rall | NA | 3.47±0.596 | 0.5 | 18367±4036 | 42765±7210 | NA | NA | 76.5±15.1 |
| 3 (PO) | 63 | Mal e | NA | 3.10±0.251 | 1.0 | 24200±2339 | 87302±3045 | NA | NA | 56.9±1.98 |
| | | Fe mal e | NA | 2.91±0.949 | 1.0 | 30533±9708 | 136518±40447 | NA | NA | 74.1±21.9 |
| | | Ove rall | NA | 3.01±0.630 | 1.0 | 27367±7205 | 111910±37212 | NA | NA | 65.5±16.8 |
| 4 (PO) | 189 | Mal e | NA | 3.68±1.22 | 4.0 | 44133±4706 | 234576±24311 | NA | NA | 50.9±5.28 |
| | | Fe mal e | NA | 7.63 (-) | 1.0 | 59567±12116 | 248087±55584 | NA | NA | 44.9±10.1 |
| | | Ove rall | NA | 5.26±3.05 | 2.0 | 51850±11791 | 241332±39077 | NA | NA | 47.9±7.91 |
| 5 (PO) Day 1 | 63 | Mal e | NA | 4.26±2.04 | 1.0 | 19857±9080 | 79661±31526 | NA | NA | 51.9±20.5 |
| | | Fe mal e | NA | 2.40±0.523 | 2.0 | 37667±2376 | 141976±23059 | NA | NA | 77.0±12.5 |

(continued)

| Group | Admin istratio n dose | Gen der | Co | $T_{1/2}$ | $T_{max}$[a] | $C_{max}$ | $AUC_{0-t}$ | CL | Vss | F |
|---|---|---|---|---|---|---|---|---|---|---|
| | (mg/kg ) | | (ng /mL) | (hr) | (hr) | (ng /mL) | (hr*ng/mL) | (mL/min/kg ) | (L/kg) | (%) |
| | | Ove rall | NA | 3.33±1.68 | 2.0 | 28762±1141 9 | 110818±4213 3 | NA | NA | 64.5±20.5 |
| 5 (PO) Day 7 | 63 | Mal e | NA | 3.69±0.34 2 | 1.0 | 30967±5754 | 98141±14699 | NA | NA | NA |
| | | Fe mal e | NA | 2.73±0.35 9 | 1.0 | 45567±1553 | 156575±3691 3 | NA | NA | NA |
| | | Ove rall | NA | 3.21±0.61 5 | 1.0 | 38267±8841 | 127358±4069 2 | NA | NA | NA |

NA: Not applicable.
a: $T_{max}$ mean is the median.
*: The standard deviation calculation was not performed when the number of individuals was smaller than 3.

The results are shown in Table 36. After the intravenous injection of 2.0 mg/kg acetate of Compound 22, the acetate of Compound 22 has the drug exposure AUCo-t of 5363 hr*ng/mL in the SD rats, the half-life $T_{1/2}$ is 3.39 hr, and the clearance rate (CL) is 6.02 mL/min/kg, which is much smaller than the hepatic blood flow (55.2 mL/min/kg). The acetate of Compound 22 is a low-clearance drug. The steady-state apparent volume of distribution ($V_{ss}$) is 0.457 L/kg, which is close to the total fluid volume (0.668 L/kg), indicating that the drug is widely distributed in the SD rats.

After the oral administration of 3 dose levels of the acetate of Compound 22 (21 mg/kg, 63 mg/kg and 189 mg/kg) to SD rats, the drug exposures ($AUC_{0-t}$) in the SD rats are 42765 hr* ng/mL, 111910 hr*ng/mL and 241332 hr*ng/mL, respectively, and the maximum plasma concentrations ($C_{max}$) are 18367 ng/mL, 27367 ng/mL and 51850 ng/mL, respectively. When the dose is increased from 21 mg/kg to 189 mg/kg, the increase ratios of AUCo-t and $C_{max}$ are lower than the dose ratio.

After the oral administration of the acetate of Compound 22 at 21 mg/kg, 63 mg/kg, and 189 mg/kg to SD rats, the average bioavailability of the acetate of Compound 22 is 76.5%, 65.5%, and 47.9%, respectively.

After the administration of the acetate of Compound 22 at 63 mg/kg daily for 7 consecutive days, the AUCo-t values on Day 1 and Day 7 are 110818 hr*ng/mL and 127358 hr*ng/mL, respectively, and the exposure ratio between Day 7 and Day 1 is less than 2, suggesting no significant accumulation of the acetate of Compound 22 in the SD rats after the repeated administration of the acetate of Compound 22 at 63 mg/kg for 7 consecutive days.

The experimental results show that the acetate of Compound 22 has excellent pharmacokinetic properties in rats, and is featured with low clearance and wide distribution; its in vivo exposure and maximum plasma concentration increase with increasing dose, showing high oral bioavailability; and no significant accumulation is observed in vivo after 7 days of administration.

**15.4 Pharmacokinetics of Compounds 22, 27 and 40 in SD rats after oral administration for 7 consecutive days**

1. Experimental design

12 male SD rats (about 7-9 weeks old on the day of administration, 283.0-298.1 g) were purchased from SPF (Beijing) Biotechnology Co., Ltd. They were randomly divided into 3 groups, with 4 rats in each group, and received orally administration once daily for 7 consecutive days. Compound 27 was administrated to Group 1 at 30 mg/kg, Compound 22 was administrated to Group 2 at 30 mg/kg, and Compound 40 was administrated to Group 3 at 30 mg/kg. All animals had free access to water and diet during dosing.

| Group | Test compound | Administration dose (mg/kg) | Dosage volume (mL/kg) | Concentration (mg/mL) | Administration mode | Number of animals (Mal)e |
|---|---|---|---|---|---|---|
| 1 | Compound 27 | 30.0 | 10.0 | 3.0 | Oral administration | 4 |
| 2 | Compound 22 | 30.0 | 10.0 | 3.0 | Oral administration | 4 |
| 3 | Compound 40 | 30.0 | 10.0 | 3.0 | Oral administration | 4 |

2. Experiment materials

1) Animal information

| | |
|---|---|
| Species/strain/grade: | Sprague-Dawley (SD) rats [Crl: CD (SD)] (SPF/VAF) |
| Source: | SPF (Beijing) Biotechnology Co., Ltd. |
| Experimental animal production license: | SCXK (Beijing) 2016-0002 |
| Animal quality certificate No.: | 11401500037777 |
| Age at first dose: | about 7-9 weeks |
| Weight at first dose: | male: 283.0-298.1 g |

2) Vehicle information

| Designation | Source | Batch No. |
|---|---|---|
| PEG400 | Sigma | BCBV8368 |
| PBS | Gibco | 1951153 |

3) Test compound information

| Name: | Compound 27 | Compound 22 | Compound 40 |
|---|---|---|---|
| Purity : | 98.43% | 99.00% | 97.76% |
| Expiration date: | Undetected | Undetected | Undetected |
| Storage condition: | store at room temperature in the dark | store at room temperature in the dark | store at room temperature in the dark |
| Traits: | White powder | White powder | White powder |
| Correction factor: | 1.02 | 1.01 | 1.02 |
| Correction factor = FW/MW*1/purity | | | |

3. Experimental method

1) Steps for preparing formulations:

0.3366 g of Compound 27, 0.3333 g of Compound 22 and 0.3366 g of Compound 40 were weighed out respectively, and dissolved in 44 mL of PEG400 with stirring and ultrasonication; 50 mL of PBS was added to the resulting mixture, stirred and sonicated for complete dissolution; PBS was added to fix the volume to the final volume of 110 mL, thereby obtaining the target formulation solution with the final concentration of 3.0 mg/mL. The prepared formulation solution was divided into 7 portions, each of 15 mL, and stored in a refrigerator at 4 °C. Before administration, the formulation was held at room temperature, until its temperature returned to room temperature; the formulation was stirred at room temperature for at least 10 minutes; and the stirring continued during the administration. The entire process for preparing the formulation was operated under yellow light, and during transferring, the prepared formulation was wrapped with tin foil to protect it from light.

2) Pharmacokinetic sampling:

| Sampling method: | tail vein and orbital vein puncture |
|---|---|
| Sampling time points: | Day 1: 15, 30 minutes and 1, 2, 4, 6, 8 and 24 hours after administration<br>Day 7: before administration, and 15, 30 minutes and 1, 2, 4, 6, 8 and 24 hours after administration |
| Sampling volume: | about 0.2 mL per rat per time point |

(continued)

| | |
|---|---|
| Sample processing and storage: | 1) After blood collection, the blood collection tube containing the anticoagulant was turned upside down several times to mix well, and placed on wet ice.<br>2) Within 60 minutes after blood collection, the samples were centrifuged at 2000 g for 10 minutes at 2-8°C to separate red blood cells, thereby obtaining plasma samples.<br>3) The plasma samples were transferred to cryovials and stored at -75 ± 15°C until analysis. |

3) Sample analysis

The concentrations of Compounds 27, 22 and 40 in the plasma of SD rats were determined by liquid chromatography-mass spectrometry, with verapamil as the internal standard compound. With the mass spectrometer scanning in the electrospray ion source positive ion mode, the mass-to-charge ratios of parent ions to daughter ions in Compounds 27, 40 and 22 and tolbutamide were 395.2→178.2, 404.2→158.9, 393.4→146.2 and 455.2→165.0, respectively.

The pharmacokinetic parameters of Compounds 27, 40 and 22 were calculated by the software Phoenix™ WinNonlin Version 6.1 using the non-compartmental model. Pharmacokinetic data was calculated using the linear logarithmic trapezoidal method, with a weight of 1/Y*Y. Samples with the sample concentration below the lower limit of quantification were excluded from the calculation of pharmacokinetic parameters. The mean value of each parameter was calculated using Microsoft Excel version 2010, and the mean value was determined from the pharmacokinetic parameters of each animal. The mean time to peak ($T_{max}$) was represented as a median.

4. Analysis method

1) LC/MS biological analysis method for Compound 27

Information of standard curve and quality control sample:

| | |
|---|---|
| Stock solution: | Compound 27: 1.00 mg/mL in DMSO |
| Internal standard stock solution: | Verapamil: 1.00 mg/mL in DMSO |
| Internal standard concentration: | 25 ng/mL Tolbutamide in acetonitrile |
| Plasma volume: | 25 μL |
| Volume of internal standard working solution: | 200 μL |
| Breadth of standard curve: | 2-200 ng/mL |
| Standard-curve solution: | Double blank, 0, 2.00, 5.00, 10.0, 20.0, 50.0, 80.0, 100 and 200 ng/mL |
| Regression mode: | Linear (1/Concentration $^2$) |
| Quality control sample concentration in plasma: | 6.00 ng/mL (LQC)<br>40.0 ng/mL (MQC)<br>160 ng/mL (HQC) |

Liquid-phase conditions:

| | |
|---|---|
| Instrument: | SHIMADZU (LC-20AD, Serial No.: L20104923601AE; LC-20AD, Serial No: L20104923599AE); CTC Analytics HTC PAL autosampler (MXY013-02A, Serial No. 260299) |
| Column: | Phenomenex Gemini NX-C18 (50×2.0 mm, 3.0 μm) |
| Column temperature: | Ambient |
| Analysis time | 4.00 min |

(continued)

| | | | | |
|---|---|---|---|---|
| Mobile phase | A: 0.1% formic acid in deionized water | | | |
| | B: 0.1% formic acid in acetonitrile | | | |
| Injection volume | 10 μL | | | |
| Holding time | Compound 27 | 1.83 min | | |
| | Verapamil | 1.90 min | | |
| Elution gradient | Time (min) | A (%) | B (%) | Flow rate (mL/min) |
| | 0 | 95.0 | 5.00 | 0.600 |
| | 0.50 | 95.0 | 5.00 | 0.600 |
| | 1.50 | 5.00 | 95.0 | 0.600 |
| | 2.80 | 5.00 | 95.0 | 0.600 |
| | 3.00 | 95.0 | 5.00 | 0.600 |
| | 4.00 | 95.0 | 5.00 | 0.600 |
| Autosampler temperature | 5±3 °C | | | |

MS conditions:

| | | |
|---|---|---|
| Instrument | Applied Biosystems MDS SCIEX API4000 LC / MS / MS 仪器 (序列号 25540110831) | |
| Ion source | ESI | |
| Ion mode | Positive ions | |
| Detection mode | MRM | |
| ESI voltage | 5500 | |
| Temperature | 550 °C | |
| Mass spectrometry parameters | Compound | Transition (*m*/*z*) |
| | Compound 27 | 395.2→178.2 |
| | Verapamil | 455.2→165.0 |
| Peak integration method: | Peak area | |

2) LC/MS biological analysis method for Compound 40

Information of standard curve and quality control sample:

| | |
|---|---|
| Stock solution: | Compound 40: 1.00 mg/mL in DMSO |
| Internal standard stock solution: | Verapamil: 1.00 mg/mL in DMSO |
| Internal standard concentration: | 25 ng/mL Tolbutamide in acetonitrile |
| Plasma volume: | 25 μL |
| Volume of internal standard working solution: | 200 μL |
| Breadth of standard curve: | 2-200 ng/mL |

(continued)

| | |
|---|---|
| Standard-curve solution: | Double blank, 0, 2.00, 5.00, 10.0, 20.0, 50.0, 80.0, 100 and 200 ng/mL |
| Regression mode: | Linear (1/Concentration $^2$) |
| Quality control sample concentration in plasma: | 6.00 ng/mL (LQC) |
| | 40.0 ng/mL (MQC) |
| | 160 ng/mL (HQC) |

Liquid-phase conditions:

| | | | | |
|---|---|---|---|---|
| Instrument: | SHIMADZU (LC-20AD, Serial No.: L20104923601AE; LC-20AD, Serial No.: L20104923599AE); CTC Analytics HTC PAL autosampler (MXY013-02A, Serial No. 260299) | | | |
| Column: | Phenomenex Gemini NX-C18 (50×2.0 mm, 3.0 μm) | | | |
| Column temperature: | Ambient | | | |
| Analysis time | 4.00 min | | | |
| Mobile phase | A: 0.1% formic acid in deionized water | | | |
| | B: 0.1% formic acid in acetonitrile | | | |
| Injection volume | 10 μL | | | |
| Holding time | Compound 40 2.07 min | | | |
| | Verapamil 1.94 min | | | |
| Elution gradient | Time (min) | A (%) | B (%) | Flow rate (mL/min) |
| | 0 | 95.0 | 5.00 | 0.600 |
| | 0.50 | 95.0 | 5.00 | 0.600 |
| | 1.50 | 5.00 | 95.0 | 0.600 |
| | 2.80 | 5.00 | 95.0 | 0.600 |
| | 3.00 | 95.0 | 5.00 | 0.600 |
| | 4.00 | 95.0 | 5.00 | 0.600 |
| Autosampler temperature | 5±3 °C | | | |

MS conditions

| | | | | | | | |
|---|---|---|---|---|---|---|---|
| Instrument | Applied Biosystems MDS SCIEX API4000 LC / MS / MS instruments (Serial NO. 25540110831) | | | | | | |
| Ion source | ESI | | | | | | |
| Ion mode | Positive ions | | | | | | |
| Detection mode | MRM | | | | | | |
| ESI voltage: | 5500 | | | | | | |
| Temperature | 550 °C | | | | | | |
| Mass spectrometry | Compoun d | Transition (m/z) | Dwell Time (ms) | DP (volts) | CE (volts) | EP (volts) | CXP (volts) |

(continued)

| | | | | | | | |
|---|---|---|---|---|---|---|---|
| parameters | Compoun d 40 | 404.2→158.9 | 150 | 70 | 35 | 10 | 8 |
| | Verapamil | 455.2→165.0 | 150 | 70 | 25 | 10 | 8 |
| Peak integration method: | Peak area | | | | | | |

3) LC/MS biological analysis method for Compound 22

Information of standard curve and quality control sample:

| | |
|---|---|
| Stock solution: | Compound 22: 1.00 mg/mL in DMSO |
| Internal standard stock solution: | Verapamil: 1.00 mg/mL in DMSO |
| Internal standard concentration: | 25 ng/mL Tolbutamide in acetonitrile |
| Plasma volume: | 25 μL |
| Volume of internal standard working solution: | 200 μL |
| Breadth of standard curve: | 2-200 ng/mL |
| Standard-curve solution: | Double blank, 0, 2.00, 5.00, 10.0, 20.0, 50.0, 80.0, 100 and 200 ng/mL |
| Regression mode: | Linear (1/Concentration $^2$) |
| Quality control sample concentration in plasma: | 6.00 ng/mL (LQC) |
| | 40.0 ng/mL (MQC) |
| | 160 ng/mL (HQC) |

Liquid-phase conditions:

| | | | | |
|---|---|---|---|---|
| Instrument: | SHIMADZU (LC-20AD, Serial No.: L20104923601AE; LC-20AD, Serial No.: L20104923599AE); CTC Analytics HTC PAL autosampler (MXY013-02A, Serial No. 260299) | | | |
| Column: | Phenomenex Gemini NX-C18 (50×2.0 mm, 3.0 μm) | | | |
| Column temperature: | Ambient | | | |
| Analysis time | 4.00 min | | | |
| Mobile phase | A: 0.1% formic acid in deionized water | | | |
| | B: 0.1% formic acid in acetonitrile | | | |
| Injection volume | 10 μL | | | |
| Holding time | Compound 22 2.02 min | | | |
| | Verapamil 1.90 min | | | |
| Elution gradient | Time (min) | A (%) | B (%) | Flow rate (mL/min) |
| | 0 | 95.0 | 5.00 | 0.600 |
| | 0.50 | 95.0 | 5.00 | 0.600 |

(continued)

| | | | | |
|---|---|---|---|---|
| | 1.50 | 5.00 | 95.0 | 0.600 |
| | 2.80 | 5.00 | 95.0 | 0.600 |
| | 3.00 | 95.0 | 5.00 | 0.600 |
| | 4.00 | 95.0 | 5.00 | 0.600 |
| Autosampler temperature | 5±3 °C | | | |

MS conditions:

| | | | | | | | |
|---|---|---|---|---|---|---|---|
| Instrument | Applied Biosystems MDS SCIEX API4000 LC / MS / MS instruments (Serial NO. 25540110831) | | | | | | |
| Ion source | ESI | | | | | | |
| Ion mode | Positive ions | | | | | | |
| Detection mode | MRM | | | | | | |
| ESI voltage: | 5500 | | | | | | |
| Temperature | 550 °C | | | | | | |
| Mass spectrometry parameters | Compound | Transition (*m*/*z*) | Dwell Time (ms) | DP (volts) | CE (volts) | EP (volts) | CXP (volts) |
| | Compound 22 | 393.4→146.2 | 150 | 70 | 36 | 10 | 8 |
| | Verapamil | 455.2→165.0 | 150 | 70 | 25 | 10 | 8 |
| Peak integration method: | Peak area | | | | | | |

5. Experimental results and conclusions

Table 38 Main PK parameters (mean values) of Compounds 27, 22 and 40 after oral administration for 7 consecutive days

| Group | Test compound | Administration dose (mg/kg) | $T_{max}$[a] | $C_{max}$ | $AUC_{0-t}$ | $C_{min}$ | $C_{avg}$ | AUC_tau |
|---|---|---|---|---|---|---|---|---|
| | | | (hr) | (ng/mL) | (hr*ng/mL) | (ng/mL) | (ng/mL) | (hr*ng/mL) |
| 1 (Day 1) | Compound 27 | 30.0 | 2 | 7103 | 29550 | - | - | - |
| 1 (Day 7) | | | 1.5 | 4615 | 22194 | 9.23 | 925 | 22194 |
| 2 (Day 1) | Compound 22 | 30.0 | 4 | 1022 | 6630 | - | - | - |
| 2 (Day 7) | | | 1.5 | 947 | 6953 | 11.4 | 290 | 6953 |

(continued)

| Group | Test compound | Administration dose (mg/kg) | $T_{max}$[a] | $C_{max}$ | $AUC_{0-t}$ | $C_{min}$ | $C_{avg}$ | AUC_ tau |
|---|---|---|---|---|---|---|---|---|
| | | | (hr) | (ng/mL) | (hr*ng/ mL) | (ng/mL) | (ng/mL) | (hr*ng/ mL) |
| 3 (Day 1) | Compound 40 | 30.0 | 3 | 503 | 5154 | - | - | - |
| 3 (Day 7) | | | 4 | 581 | 6584 | 52.3 | 274 | 6584 |
| a: $T_{max}$ value is the median.<br>"-": Not applicable. | | | | | | | | |

Male SD rats were orally administered with Compound 27, Compound 22, and Compound 40 at 30 mg/kg once a day for 7 consecutive days, and the plasma drug concentrations were detected on Day 1 and Day 7 after administration.

After the male SD rats were orally administered with Compound 27, Compound 22, and Compound 40 at 30 mg/kg once a day for 7 consecutive days, the drug exposures AUCo-t on Day 1 were 29550 hr*ng/mL, 6630 hr*ng/mL and 5154 hr*ng/mL respectively; and their maximum plasma concentrations $C_{max}$ were 7103 ng/mL, 1022 ng/mL and 503 ng/mL, respectively. On the seventh day of continuous administration, the drug exposures AUCo-t of compound 27, compound 22 and compound 40 in the plasma of SD rats were 22194 hr*ng/mL, 6953 hr*ng/mL and 6584 hr*ng/mL, respectively; and the maximum plasma concentrations $C_{max}$ were 4615 ng/mL, 947 ng/mL and 581 ng/mL, respectively. The results show that after the oral administration of Compound 27, Compound 22, and Compound 40 at 30 mg/kg once a day for 7 consecutive days, Compound 27, Compound 22, and Compound 40 do not show significant accumulation in the SD rats (Table 38).

The data show that the test compounds exhibit excellent pharmacokinetic parameters in the SD mice, high in vivo exposure, and no significant accumulation after consecutive administration. The excellent pharmacokinetic properties in rats for the test compounds can provide a support to the further clinical experiment on human beings.

**Example 16 Pharmacokinetic evaluation of present compounds in female BALB/c mice**

1. Experimental design

| Group | Number of animals | Dosage group | Dose (mg/kg) | Administration mode | Planned dosage period | Actual dosage period |
|---|---|---|---|---|---|---|
| 1 | 12 | Compound 22 | 100 | p.o. | QD × 1 time | QD × 1 time |
| 2 | 12 | Compound 22 | 100 | p.o. | QD × 1 time | QD × 1 time |

2. Experiment materials

1) Test animals

BALB/c mice, female, 7-9 weeks old (the age of mice at the start of the experiment), weight: 19.2-22.1 g, 51 mice (34 mice plus another 10 surplus mice). They were purchased from Shanghai Lingchang Biotechnology Co., Ltd. (LC), with animal certificate number: 2013001835211. Rearing environment: SPF level.

2) Test article

Test article: Compound 22: batch No.: F, package specification: 38.0 mg/vial+5045.1 mg/vial, purity: 98.96%, moisture: 5.38%, provided by Vimgreen Pharma, Zhejiang, China, powder, sealed at RT.

3. Experimental method

1) Random grouping

Before administration began, all animals were weighed and randomly grouped according to their body weight by StudyDirector™ (Version No. 3.1.399.19, supplier: Studylog System, Inc., S. San Francisco, CA, USA).

2) Preparation of test article and vehicle

| Test article/control article | Dose (mg/kg) | Preparation | Concentration (mg/ml) | Preparation frequency | Store after preparation |
|---|---|---|---|---|---|
| Vehicle 1 (40% PEG400/60% Water) for preparing drugs for G1 | -- | 1.92 ml of PEG400 was added with 2.88 ml of sterilized water, and mix well for later use. | -- | Prepare for immediate use | Discard after use |
| Vehicle 2 (40% (2-hydroxypropyl)-β-cyclodextrin) for preparing drugs for G2 | -- | 2.0g of (2-hydroxypropyl)-β-cyclodextrin was weighed and added with 5.0 ml of sterilized water, and after complete dissolution, the resulting mixture was set aside for later use. | -- | Prepare for immediate use | After use Discard |
| Compound 22 G1 | 100 | 38.45 mg of Compound 22 (36 mg of free base) were weighed and added with 3.6 ml of vehicle 1; and the resulting mixture was vortexed and sonicated to obtain 3.6 ml of suspension. | 10 | Prepare for immediate use | After use Discard |
| Compound 22 G2 | 100 | 38.45 mg of Compound 22 (36 mg of free base) were weighed and added with 3.6 ml of vehicle 2; and the resulting mixture was vortexed and sonicated to obtain 3.6 ml of suspension. | 10 | Prepare for immediate use | After use Discard |

3) Experimental observation and data acquisition

The experimental protocols for animal experiments throughout this experiment were reviewed and approved by the CrownBio IACUC. During the experiment, the animal experiments were operated according to the requirements of AAALAC. After grouping, routine monitoring covered the effects of treatment on the normal behaviors of animals, including, the activity, food and water intake, eyes, clothing hair and other abnormalities of the experimental animals. Clinical symptoms observed during the experiment were recorded in the original data. In the experiment, animal body weight data was acquired by StudyDirector™ (Version No.: 3.1.399.19, Supplier: Studylog System, Inc.) software; after measurements on the balance, the original data was directly imported into the software; and any changes in the data would be recorded in this software. All processes such as dosing and weighing were carried out in a biosafety cabinet or a clean bench.

4) Experimental end point

After single administration, plasma was collected from each group at 0.25 h, 0.5 h, 1 h, 2 h, 4 h, 6 h, 8 h and 24 h, with 3 mice at each time point, and the experiment was terminated after 24h. 10 remaining mice were used to collect blank plasma.
Experimental results and discussions

Table 39 Pharmacokinetic data of Compound 22 in different vehicles

| Group | Administration dose (mg/kg) | T1/2 (hr) | Tmax (hr) | Cmax (ng /mL) | AUC0-t (hr*ng/mL ) | AUC0-∞ (ng·hr/mL) | Last time point of AUClast (hr) |
|---|---|---|---|---|---|---|---|
| 1 | 100 | 3.04 | 1.0 | 2078 | 15945 | 16072 | 24 |
| 2 | 100 | 2.66 | 0.5 | 5577 | 28353 | 28487 | 24 |

After single administration to the mice in G1 and G2, plasma was collected from each group at 0.25 h, 0.5 h, 1 h, 2 h, 4 h, 6 h, 8 h and 24 h, respectively, to detect the PK response of Compound 22 in the mice. The results show that the concentrations of Compound 22 in the plasma of the mice in G2 at different time points are significantly higher than those in the plasma of the mice in G1. 40% (2-hydroxypropyl)-β-cyclodextrin as the vehicle of Compound 22 presents a good effect. Meanwhile, under the dose condition of 100 mg/kg, the mice have higher drug exposure and show excellent pharmacokinetic performance.

## Claims

1. Use of a compound of formula I in the preparation of a drug for treating diseases,

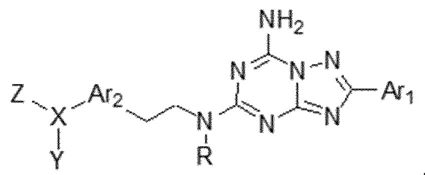

, formula I

wherein:

R is hydrogen or methyl;
$Ar_1$ is furanyl optionally with a substituent, phenyl optionally with a substituent, or pyridyl optionally with a substituent; any aromatic ring optionally with a substituent is substituted by halogen or oxo;
$Ar_2$ is phenyl optionally with a substituent, pyridyl optionally with a substituent, or pyrimidinyl optionally with a substituent; any aromatic ring optionally with a substituent is substituted by halogen, hydroxyl, cyano, or methoxy;
X is oxygen or nitrogen; and
Y and Z are each independently hydrogen, $C_{1-3}$ alkyl optionally with a substituent, $C_{1-5}$ cycloalkyl optionally with a substituent, heterocycloalkyl optionally with a substituent, heterocycloalkylalkyl optionally with a substituent, aryl optionally with a substituent, $C_{1-3}$ alkylcarbonyl optionally with a substituent, $C_{1-5}$ cycloalkylcarbonyl optionally with a substituent, heterocycloalkylcarbonyl optionally with a substituent, heterocycloalkylalkylcarbonyl optionally with a substituent,
arylcarbonyl optionally with a substituent, or heteroarylcarbonyl optionally with a substituent; any said group optionally with a substituent is substituted by halogen, hydroxy, methoxy, ethoxy, trifluoromethoxy, trifluoroethoxy, alkylamino, cycloalkylamino, heterocyclyl, aryl, heteroaryl, or
$C_{1-3}$ alkyl polyoxyethylene; or, Y and Z are linked to form heterocycloalkyl optionally with a substituent and having 5 to 7 ring atoms; any said ring optionally with a substituent is substituted by halogen, oxo, methoxy, ethoxy, trifluoromethoxy, or trifluoroethoxy; or Y or Z is not present, or a pharmaceutically acceptable solvate or salt thereof.

2. The use according to claim 1, wherein R is hydrogen; $Ar_1$ is 2-furanyl; $Ar_2$ is phenyl optionally with a substituent; any said phenyl optionally with a substituent is substituted by halogen; X is oxygen or nitrogen; Y and Z are each independently hydrogen, $C_{2-3}$ alkyl optionally with a substituent, heterocycloalkyl optionally with a substituent, or heterocycloalkylalkyl optionally with a substituent; any said group optionally substituted is substituted by methoxy, ethoxy, trifluoromethoxy, trifluoroethoxy, or $C_{1-2}$ alkyl polyoxyethylene; or Y and Z are linked to form a morpholinyl ring; or Y or Z is not present, or a pharmaceutically acceptable solvate or salt thereof.

3. The use according to any one of claims 1-2, wherein R is hydrogen; $Ar_1$ is 2-furanyl; $Ar_2$ is phenyl; X is nitrogen;

and Y and Z are each independently hydrogen, ethyl optionally with a substituent, or oxetanyl optionally with a substituent; any said group optionally with a substituent is substituted by methoxy, ethoxy, trifluoromethoxy or trifluoroethoxy; or, Y and Z are linked to form a morpholinyl ring, or a pharmaceutically acceptable solvate or salt thereof.

**4.** The use according to any one of claims 1-3, wherein said compound is selected from the group consisting of:

N-(4-(2-((7-amino-2-(furan-2-yl)-[1,2,4]triazolo[1,5-a][1,3,5]triazin-5-yl)amino)ethyl)phenyl)-3-morpholine propanamide;
N-(4-(2-((7-amino-2-(furan-2-yl)-[1,2,4]triazolo[1,5-a][1,3,5]triazin-5-yl)amino)ethyl)phenyl)-2-morpholine propanamide;
N-(4-(2-((7-amino-2-(furan-2-yl)-[1,2,4]triazolo[1,5-a][1,3,5]triazin-5-yl)amino)ethyl)phenyl)pivalamide;
N-(4-(2-((7-amino-2-(furan-2-yl)-[1,2,4]triazolo[1,5-a][1,3,5]triazin-5-yl)amino)ethyl)phenyl)-3-hydroxy-2,2-dimethylpropionamide;
N-(4-(2-((7-amino-2-(furan-2-yl)-[1,2,4]triazolo[1,5-a][1,3,5]triazin-5-yl)amino)ethyl)phenyl)-2-hydroxy-2-methylpropionamide;
N-(4-(2-((7-amino-2-(furan-2-yl)-[1,2,4]triazolo[1,5-a][1,3,5]triazin-5-yl)amino)ethyl)phenyl)-4-methyltetrahydro-2H-pyran-4-carboxamide;
2-(furan-2-yl)-N5-(2-(pyrimidin-4-yl)ethyl)-[1,2,4]triazolo[1,5-a][1,3,5]triazine-5,7-diamine;
2-(furan-2-yl)-N5-(2-(pyridin-3-yl)ethyl)-[1,2,4]triazolo[1,5-a][1,3,5]triazine-5,7-diamine;
2-(furan-2-yl)-N5-(4-(2-(pyrrolidin-1-yl)ethoxy)phenethyl)-[1,2,4]triazolo[1,5-a][1,3,5]-triazine-5,7-diamine;
N5-(4-(2-(azetidine-1-yl)ethoxy)phenethyl)-2-(furan-2-yl)-[1,2,4]triazolo[1,5-a][1,3,5]triazine-5,7-diamine;
2-(furan-2-yl)-N5-(4-(2-(4-methylpiperazin-1-yl)ethoxy)phenethyl)-[1,2,4]triazolo[1,5-a][1,3,5]triazine-5,7-diamine;
2-(furan-2-yl)-N5-(4-(3-(4-methylpiperazin-1-yl)propoxy)phenethyl)-[1,2,4]triazolo[1,5-a][1,3,5]triazine-5,7-diamine;
N5-(4-(2-(3,3-difluoropyrrolidin-1-yl)ethoxy)phenethyl)-2-(furan-2-yl)-[1,2,4]triazolo[1,5-a][1,3,5]triazine-5,7-diamine;
(R)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine;
(S)-N5-(4-(2-(3-fluoropyrrolidin-1-yl)ethoxy)phenethyl)-2-(furan-2-yl)-[1,2,4]triazolo[1,5-a][1,3,5]triazine-5,7-diamine;
2-(furan-2-yl)-N5-(4-((2-morpholinoethyl)amino)phenethyl)-[1,2,4]triazolo[1,5-a][1,3,5]triazine-5,7-diamine;
2-(furan-2-yl)-N5-(4-((tetrahydro-2H-pyran-4-yl)amino)phenethyl)-[1,2,4]triazolo[1,5-a][1,3,5]triazine-5,7-diamine;
2-(furan-2-yl)-N5-(4-((tetrahydrofuran-3-yl)amino)phenethyl)-[1,2,4]triazolo[1,5-a][1,3,5]triazine-5,7-diamine;
2-(furan-2-yl)-N5-(4-(4-methylpiperazin-1-yl)phenethyl)-[1,2,4]triazolo[1,5-a][1,3,5]triazine-5,7-diamine;
N5-(4-(bis(2-methoxyethyl)amino)phenethyl)-2-(furan-2-yl)-[1,2,4]triazolo[1,5-a][1,3,5]triazine-5,7-diamine;
2-(furan-2-yl)-N5-(4-(oxetane-3-ylamino)phenethyl)-[1,2,4]triazolo[1,5-a][1,3,5]triazine-5,7-diamine;
2-(furan-2-yl)-N5-(4-(oxetan-3-ylmethyl)phenethyl)-[1,2,4]triazolo[1,5-a][1,3,5]triazine-5,7-diamine;
2-(furan-2-yl)-N5-(4-((1-methoxypropane-2-yl)amino)phenethyl)-[1,2,4]triazolo[1,5-a][1,3,5]triazine-5,7-diamine;
2-(furan-2-yl)-N5-(4-((2-methoxyethyl)(methyl)amino)phenethyl)-[1,2,4]triazolo[1,5-a][1,3,5]triazine-5,7-diamine;
N5-(4-(ethyl(2-methoxyethyl)amino)phenethyl)-2-(furan-2-yl)-[1,2,4]triazolo[1,5-a][1,3,5]triazine-5,7-diamine;
2-(furan-2-yl)-N5-(4-((2-methoxyethyl)amino)phenethyl)-[1,2,4]triazolo[1,5-a][1,3,5]triazine-5,7-diamine;
2-(furan-2-yl)-N5-(4-((2-(2-methoxyethoxy)ethyl)amino)phenethyl)-[1,2,4]triazolo[1,5-a][1,3,5]triazine-5,7-diamine;
2-(furan-2-yl)-N5-(4-((2-(2-(2-methoxyethoxy)ethoxy)ethyl)amino)phenethyl)-[1,2,4]triazolo[1,5-a][1,3,5]triazine-5,7-diamine;
N5-(4-((2-ethoxyethyl)amino)phenethyl)-2-(furan-2-yl)-[1,2,4]triazolo[1,5-a][1,3,5]triazine-5,7-diamine;
2-(furan-2-yl)-N5-(4-((2-(2,2,2-trifluoroethoxy)ethyl)amino)phenethyl)-[1,2,4]triazolo[1,5-a][1,3,5]triazine-5,7-diamine;
2-(furan-2-yl)-N5-(4-((3-methoxypropyl)amino)phenethyl)-[1,2,4]triazolo[1,5-a][1,3,5]triazine-5,7-diamine;
2-((4-(2-((7-amino-2-(furan-2-yl)-[1,2,4]triazolo[1,5-a][1,3,5]triazine-5-yl)amino)ethyl)-phenyl)amino)acetonitrile;
N5-(4-(1,3-dimethoxypropane-2-ylamino)phenethyl)-2-(furan-2-yl)-[1,2,4]triazolo[1,5-a][1,3,5]triazine-5,7-diamine;

2-(3-fluorophenyl)-N5-(4-((2-methoxyethyl)amino)phenethyl)-[1,2,4]triazolo[1,5-a][1,3,5]triazine-5,7-diamine; and
2-(3-fluorophenyl)-N5-(4-(oxetane-3-ylamino)phenethyl)-[1,2,4]triazolo[1,5-a][1,3,5]triazine-5,7-diamine;
or a pharmaceutically acceptable solvate or salt thereof.

**5.** Use of a compound of formula II in the preparation of a drug for treating diseases,

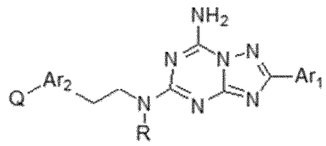
, formula II

wherein,

R is hydrogen or methyl;
$Ar_1$ is furanyl optionally with a substituent, phenyl optionally with a substituent, or pyridyl optionally with a substituent; any aromatic ring optionally with a substituent is substituted by halogen or oxo;
$Ar_2$ is phenyl optionally with a substituent, pyridyl optionally with a substituent, or pyrimidinyl optionally with a substituent; any aromatic ring optionally with a substituent is substituted by halogen, hydroxyl, cyano, or methoxy; and
Q is a 5-6 membered aromatic ring optionally substituted by X, an aminocarbonyl group optionally substituted by Y and Z on nitrogen, an aminosulfonyl group optionally substituted by Y and Z on nitrogen, a nitro group, or a cyano group; X is halogen or optionally substituted $C_{1-3}$ alkyl; any said optionally substituted alkyl group is substituted by halogen, cyano, methoxy , ethoxy, trifluoromethoxy, trifluoroethoxy, aryl or heteroaryl; Y and Z are each independently hydrogen or optionally substituted $C_{1-3}$ alkyl; any said optionally substituted alkyl group is substituted by halogen, hydroxy, methyl, alkylamino, or cycloalkylamino; or, Y and Z are linked to form an optionally substituted ring having 5 to 7 ring atoms; any said optionally substituted ring is substituted by halogen, methyl, ethyl, trifluoromethyl or trifluoroethyl,
or a pharmaceutically acceptable solvate or salt thereof.

**6.** The use according to claim 5, wherein

R is hydrogen;
$Ar_1$ is 2-furanyl;
$Ar_2$ is phenyl or pyridyl; and
Q is nitro, cyano, or a 5-6 membered aromatic ring optionally substituted by X; X is optionally substituted $C_{1-3}$ alkyl; any said optionally substituted alkyl is halogen, cyano, methoxy, aryl, or heteroaryl,
or a pharmaceutically acceptable solvate or salt thereof.

**7.** The use according to any one of claims 5-6, wherein

R is hydrogen;
$Ar_1$ is 2-furanyl;
$Ar_2$ is phenyl; and
Q is a tetrazole ring optionally substituted by X; X is an optionally substituted $C_{1-3}$ alkyl; any said optionally substituted alkyl is optionally substituted by halogen, cyano, or methoxy,
or a pharmaceutically acceptable solvate or salt thereof.

**8.** The use according to any one of claims 5-7, wherein said compound is selected from the group consisting of:

4-(2-(7-amino-2-(furan-2-yl)-[1,2,4]triazolo[1,5-a][1,3,5]triazine-5-ylamino)ethyl)-N,N-dimethylbenzamide;
N5-(4-(1-methyl-1H-tetrazol-5-yl)phenethyl)-2-(furan-2-yl)-[1,2,4]triazolo[1,5-a][1,3,5]triazine-5,7-diamine;
N5-(4-(2-methyl-2H-tetrazol-5-yl)phenethyl)-2-(furan-2-yl)-[1,2,4]triazolo[1,5-a][1,3,5]triazine-5,7-diamine;
N5-(4-(2-ethyl-2H-tetrazol-5-yl)phenethyl)-2-(furan-2-yl)-[1,2,4]triazolo[1,5-a][1,3,5]triazine-5,7-diamine;
N5-(4-(2-isopropyl-2H-tetrazol-5-yl)phenethyl)-2-(furan-2-yl)-[1,2,4]triazolo[1,5-a][1,3,5]triazine-5,7-diamine;
N5-(4-(2-propyl-2H-tetrazol-5-yl)phenethyl)-2-(furan-2-yl)-[1,2,4]triazolo[1,5-a][1,3,5]triazine-5,7-diamine;
2-(5-(4-(2-(7-amino-2-(furan-2-yl)-[1,2,4]triazolo[1,5-a][1,3,5]triazine-5-ylamino)ethyl)-phenyl)-2H-tetrazol-2-yl)acetonitrile;
4-(2-(7-amino-2-(furan-2-yl)-[1,2,4]triazolo[1,5-a][1,3,5]triazine-5-ylamino)ethyl)-benzonitrile;
N5-(4-(2-(2,2,2-trifluoroethyl)-2H-tetrazol-5-yl)phenethyl)-2-(furan-2-yl)-[1,2,4]triazolo[1,5-a][1,3,5]triazine-5,7-diamine;
N5-(4-(2-(2-methoxyethyl)-2H-tetrazol-5-yl)phenethyl)-2-(furan-2-yl)-[1,2,4]triazolo[1,5-a][1,3,5]triazine-5,7-diamine;
2-(furan-2-yl)-N5-(4-(pyridin-2-yl)phenethyl)-[1,2,4]triazolo[1,5-a][1,3,5]triazine-5,7-diamine;
2-(furan-2-yl)-N5-(4-(pyridin-3-yl)phenethyl)-[1,2,4]triazolo[1,5-a][1,3,5]triazine-5,7-diamine;
2-(furan-2-yl)-N5-(4-(pyridin-4-yl)phenethyl)-[1,2,4]triazolo[1,5-a][1,3,5]triazine-5,7-diamine;
2-(furan-2-yl)-N5-(4-(pyrimidin-2-yl)phenethyl)-[1,2,4]triazolo[1,5-a][1,3,5]triazine-5,7-diamine;
2-(furan-2-yl)-N5-(4-(1-methyl-1H-1,2,4-triazol-3-yl)phenethyl)-[1,2,4]triazolo[1,5-a][1,3,5]triazine-5,7-diamine;
N5-(4-(2H-tetrazol-5-yl)phenethyl)-2-(furan-2-yl)-[1,2,4]triazolo[1,5-a][1,3,5]triazine-5,7-diamine;
2-(furan-2-yl)-N-(4-(methylsulfonyl)phenethyl)-[1,2,4]triazolo[1,5-a][1,3,5]triazine-5-amine;
2-(3-fluorophenyl)-N5-(4-(2-methyl-2H-tetrazol-5-yl)phenethyl)-[1,2,4]triazolo[1,5-a][1,3,5]triazine-5,7-diamine;
N5-(4-(2-methyl-2H-tetrazol-5-yl)phenethyl)-2-phenyl-[1,2,4]triazolo[1,5-a][1,3,5]triazine-5,7-diamine; and
2-(furan-2-yl)-N5-(2-(6-(2-methyl-2H-tetrazol-5-yl)pyridin-3-yl)ethyl)-[1,2,4]triazolo[1,5-a][1,3,5]triazine-5,7-diamine,
or a pharmaceutically acceptable solvate or salt thereof.

**9.** The use according to any one of claims 1-8, wherein said compound is selected from the group consisting of:

2-(furan-2-yl)-N5-(4-(oxetane-3-ylamino)phenethyl)-[1,2,4]triazolo[1,5-a][1,3,5]triazine-5,7-diamine;
2-(furan-2-yl)-N5-(4-((2-methoxyethyl)amino)phenethyl)-[1,2,4]triazolo[1,5-a][1,3,5]triazine-5,7-diamine;
N5-(4-(2-methyl-2H-tetrazol-5-yl)phenethyl)-2-(furan-2-yl)-[1,2,4]triazolo[1,5-a][1,3,5]triazine-5,7-diamine; and
N5-(4-(2-ethyl-2H-tetrazol-5-yl)phenethyl)-2-(furan-2-yl)-[1,2,4]triazolo[1,5-a][1,3,5]triazine-5,7-diamine;
and a pharmaceutically acceptable solvate or salt thereof.

**10.** The use according to any one of claims 1-9, wherein said pharmaceutically acceptable solvate or salt of said compound comprises acetate and/or benzenesulfonate.

**11.** The use according to any one of claims 1-10, wherein said diseases comprise Parkinson's disease.

**12.** The use according to any one of claims 1-11, wherein said diseases comprise tumors.

**13.** The use according to claim 12, wherein said tumors comprise solid tumors and non-solid tumors.

**14.** The use according to claim 13, wherein said solid tumors comprise a colon cancer and melanoma.

**15.** The use according to claim 13, wherein said non-solid tumors comprise lymphoma.

**16.** The use according to any one of claims 1-15, wherein said drug is prepared to adapt to oral administration and/or injection administration.

**17.** The use according to any one of claims 1-16, wherein said drug further comprises a pharmaceutically acceptable carrier.

**18.** The use according to any one of claims 1-17, wherein said pharmaceutically acceptable carrier comprises cyclodextrin.

**19.** A method for treating diseases, comprising the step of administering said compound of any one of claims 1-18 to a subject in need thereof.

**20.** The method according to claim 19, wherein said diseases comprise Parkinson's disease.

**21.** The method according to claim 20, wherein said subject exhibits early signs of the Parkinson's disease.

**22.** The method according to any one of claims 20-21, wherein said subject has previously received or not received a

drug for treating the Parkinson's disease.

**23.** The method according to according to any one of claims 20-22, wherein said subject receives a drug for treating the Parkinson's disease before, concurrently with or after administration of said compound.

**24.** The method according to claim 23, wherein said subject develops resistance to a drug for treating the Parkinson's disease.

**25.** The method according to any one of claims 20-24, wherein said drug for treating the Parkinson's disease comprises dopamine.

**26.** The method according to any one of claims 20-25, wherein said treating comprises reducing the rate of progression of the Parkinson's disease in said subject, and/or delaying the clinical progression of the Parkinson's disease in said subject.

**27.** The method according to any one of claims 20-26, wherein said rate of progression of the Parkinson's disease in said subject is assessed by UPDRS.

**28.** The method according to claim 19, wherein said diseases comprise tumors.

**29.** The method according to claim 28, wherein said tumors comprise solid tumors and non-solid tumors.

**30.** The method according to claim 29, wherein said solid tumors comprise a colon cancer and melanoma.

**31.** The method according to claim 30, wherein said non-solid tumors comprise lymphoma.

**32.** The method according to any one of claims 19-31, comprising administrating said compound orally and/or by injection.

**33.** The method according to any one of claims 19-32, wherein said compound is administered at a frequency of once a day or twice a day.

**34.** The method according to any one of claims 19-33, wherein said compound is administered at a dose of from about 0.001 mg/kg to about 500 mg/kg.

**35.** The method according to any one of claims 19-34, wherein said compound is administered at a dose of from about 1 mg/kg to about 100 mg/kg.

**36.** The method according to any one of claims 28-35, wherein said subject receives an immunotherapy for treating tumors before, concurrently with, or after administration of said compound.

**37.** The method according to claim 36, wherein said immunotherapy for treating the tumors comprises an immune checkpoint inhibitor.

**38.** The method according to claim 37, wherein said immunotherapy for treating the tumors comprises a PD-1 antibody.

**39.** A pharmaceutical combination or kit, comprising (1) said compound of any one of claims 1-10, and (2) a drug for treating Parkinson's disease.

**40.** The pharmaceutical combination or kit according to claim 39, wherein said drug for treating Parkinson's disease comprises dopamine.

**41.** A pharmaceutical combination or kit, comprising (1) said compound of any one of claims 1-10, and (2) an immunotherapy for treating a tumor.

**42.** The pharmaceutical combination or kit according to claim 41, wherein said immunotherapy for treating the tumors comprises an immune checkpoint inhibitor.

**43.** The pharmaceutical combination or kit according to claim 42, wherein said immunotherapy for treating the tumors

comprises a PD-1 antibody.

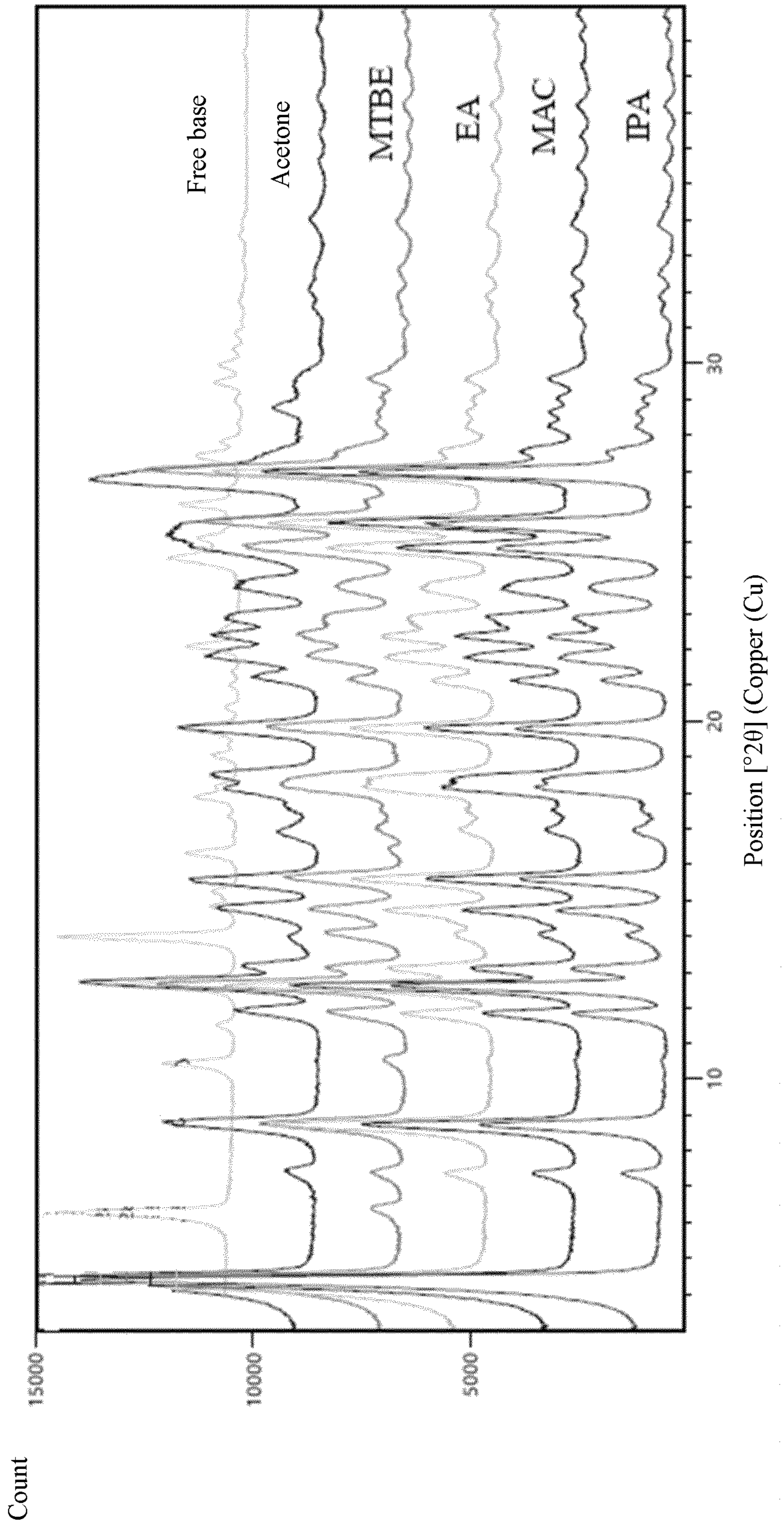
Free base
Acetone
MTBE
EA
MAC
IPA
15000
10000
5000
10
20
30
Position [°2θ] (Copper (Cu))
Count

**FIG. 1**

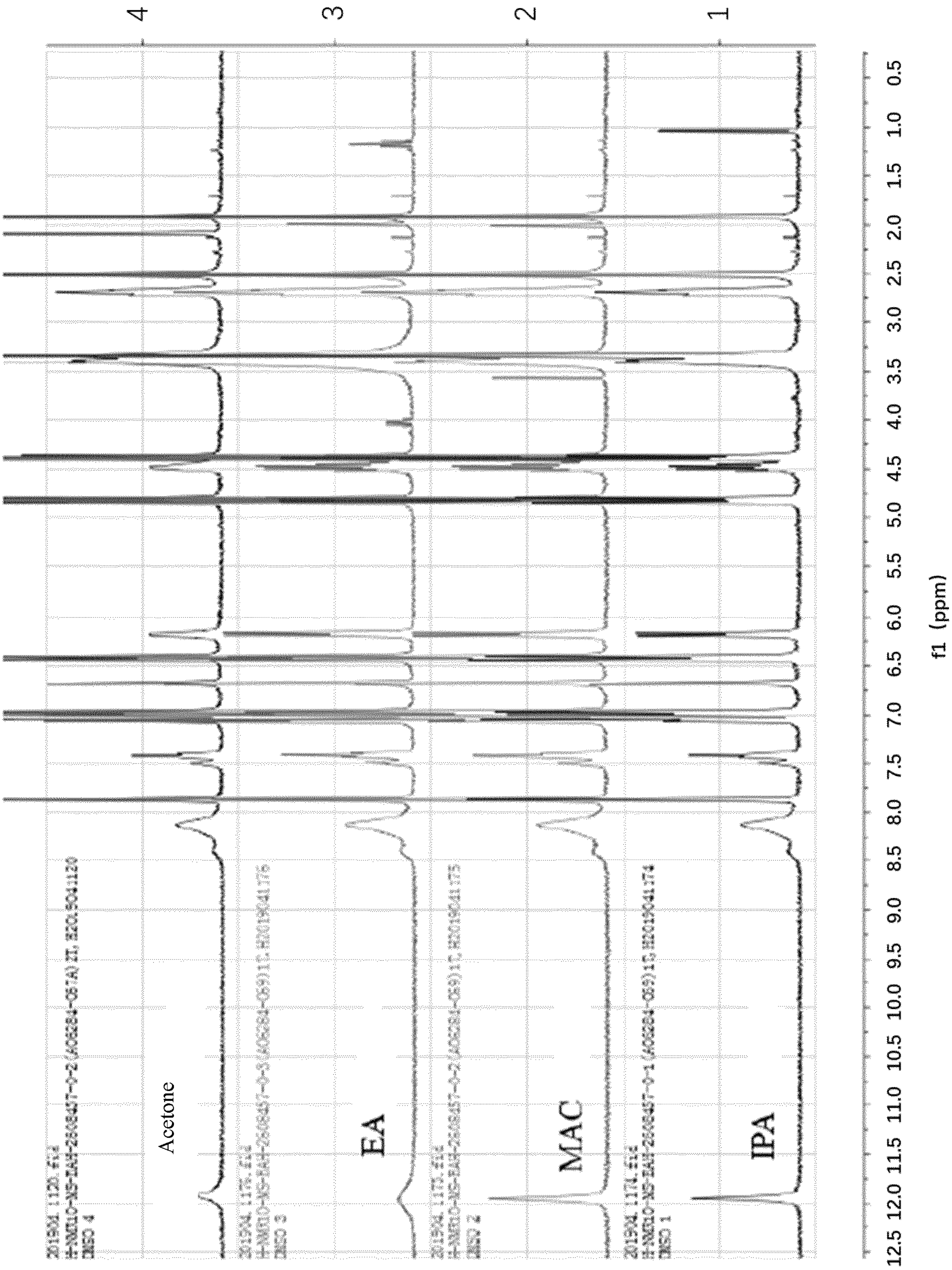


FIG. 2

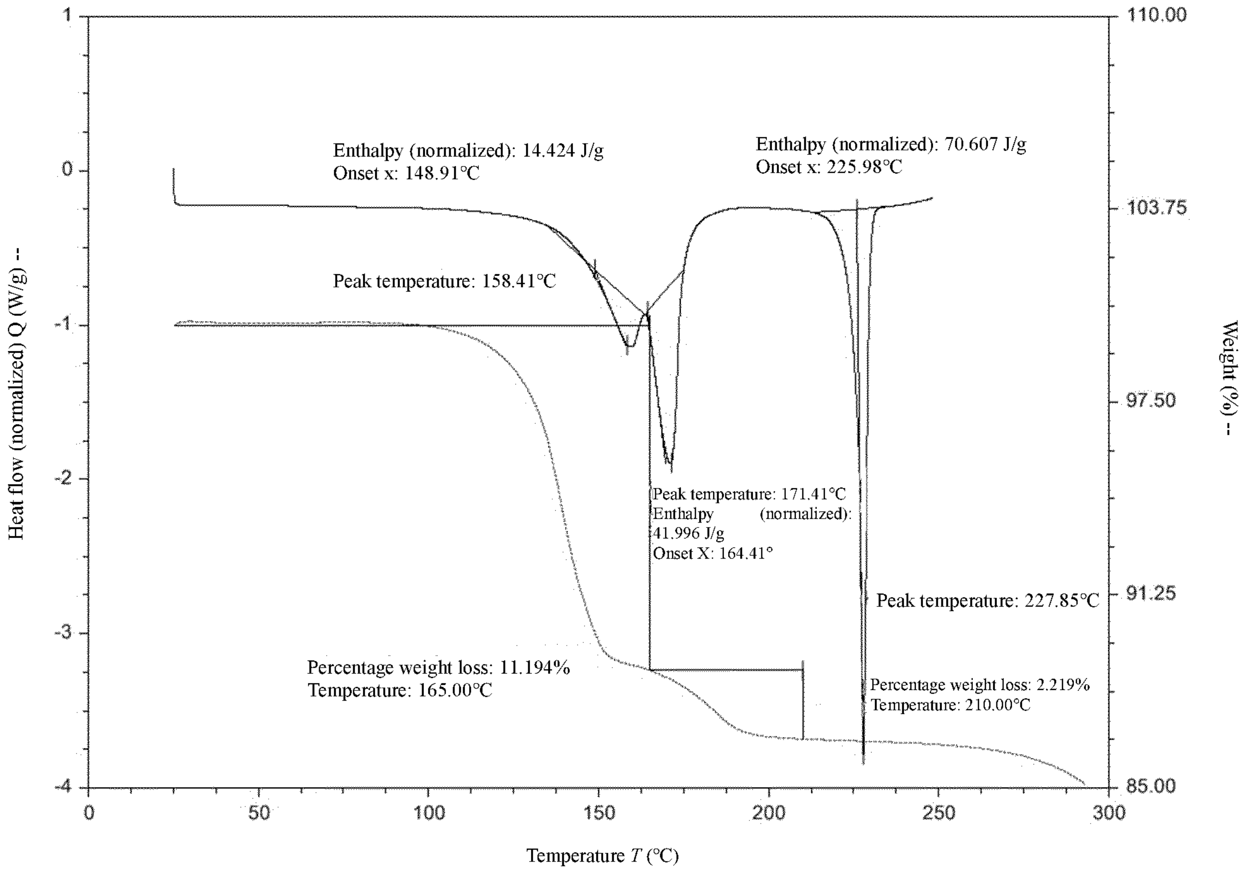

1
0
-1
-2
-3
-4
110.00
103.75
97.50
91.25
85.00
Heat flow (normalized) Q (W/g) --
Weight (%) --
Enthalpy (normalized): 14.424 J/g
Onset x: 148.91°C
Peak temperature: 158.41°C
Enthalpy (normalized): 70.607 J/g
Onset x: 225.98°C
Peak temperature: 171.41°C
Enthalpy (normalized): 41.996 J/g
Onset X: 164.41°
Peak temperature: 227.85°C
Percentage weight loss: 11.194%
Temperature: 165.00°C
Percentage weight loss: 2.219%
Temperature: 210.00°C
0
50
100
150
200
250
300
Temperature T (°C)


**FIG. 3**

# INTERNATIONAL SEARCH REPORT

International application No.
**PCT/CN2020/139690**

## A. CLASSIFICATION OF SUBJECT MATTER

C07D 487/04(2006.01)i; A61K 31/53(2006.01)i; A61P 35/00(2006.01)i; A61P 25/16(2006.01)i

According to International Patent Classification (IPC) or to both national classification and IPC

## B. FIELDS SEARCHED

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; DWPI; SIPOABS; CJFD; CNKI; CNTXT; USTXT; WOTXT; EPTXT; STN-REGISTRY; STN-CAPLUS; PATENTICS; 超星读秀; ISI-Web of Science: 浙江春禾医药科技有限公司, 孙三兴, 陈正树, 叶进启, 赵龙, 胡崇波, 杨勇, 管峰, 潘姝花, 胡宁, 潘婷婷, 宋国伟, 候方杰, 三唑, 三嗪, 乙胺, 腺苷, 癌, 肿瘤, 帕金森, 老年痴呆, +triazol+, +triazin+, +ethylamin+, adenosine, cancer, tumour, tumor, parkinson

## C. DOCUMENTS CONSIDERED TO BE RELEVANT

| Category* | Citation of document, with indication, where appropriate, of the relevant passages | Relevant to claim No. |
|---|---|---|
| X | SIHVER, W., et al. "Binding of Tritiated and Radioiodinated ZM241, 385 to Brain A2A Adenosine Receptors" *Nuclear Medicine and Biology,* Vol. 31, No. 2, 29 February 2004 (2004-02-29), ISSN: 0969-8051, page 173, column 1, paragraph 1 and 2, page 174, figure 1, and page 175, column 2, paragraph 2 | 1-11, 16-27, 32-35, 39, 40 |
| X | JORG, M., et al. "Novel Adenosine A2A Receptor Ligands: A Synthetic, Functional and Computational Investigation of Selected Literature Adenosine A2A Receptor Antagonists for Extending into Extracellular Space" *Bioorganic & Medicinal Chemistry Letters,* Vol. 23, No. 11, 02 April 2013 (2013-04-02), ISSN: 0960-894X, description, page 3427, column 1, line 1 to page 3428, column 1, line 1, and page 3432, table 1 | 1-11, 16-27, 32-35, 39, 40 |
| X | CN 1056879 A (IMPERIAL CHEMICAL INDUSTRIES PLC) 11 December 1991 (1991-12-11) description, page 22, lines 22-25, embodiments 71, 81, 161, 162, 164, 167 and 172, page 28, lines 18-26, and claim 1 | 1, 2, 10, 16, 17, 19, 32 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 February 2021** | **12 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/139690**

| C. DOCUMENTS CONSIDERED TO BE RELEVANT | | |
|---|---|---|
| Category* | Citation of document, with indication, where appropriate, of the relevant passages | Relevant to claim No. |
| X | US 5380714 A (ICI PLC) 10 January 1995 (1995-01-10)<br>description, column 6, pages 46-54 and column 10, lines 9-30, and claim 1 | 5, 10, 16, 17, 19, 32 |
| PX | CN 111051309 A (ZHEJIANG VIMGREEN PHARMACEUTICALS LTD.) 21 April 2020 (2020-04-21)<br>description, paragraphs [0060]-[0062], [0071] and [0072], and embodiment | 1-43 |
| PX | CN 111164084 A (ZHEJIANG VIMGREEN PHARMACEUTICALS LTD.) 15 May 2020 (2020-05-15)<br>description, paragraphs [0050]-[0053], [0062] and [0063], and embodiment | 1-43 |
| Y | LEONE, R. D., et al. "Targeting Adenosine for Cancer Immunotherapy"<br>*Journal for ImmunoTherapy of Cancer,* Vol. 6, No. 1, 18 June 2018 (2018-06-18), ISSN: 2051-1426,<br>the abstract, and page 3, column 1, paragraph 2 | 11-18, 20-43 |
| Y | JORG, M., et al. "Novel Adenosine A2A Receptor Ligands: A Synthetic, Functional and Computational Investigation of Selected Literature Adenosine A2A Receptor Antagonists for Extending into Extracellular Space"<br>*Bioorganic & Medicinal Chemistry Letters,* Vol. 23, No. 11, 02 April 2013 (2013-04-02), ISSN: 0960-894X,<br>description, page 3427, column 1, line 1 to page 3428, column 1, line 1, and page 3432, table 1 | 12-15, 28-31, 36-38, 41-43 |
| Y | SIHVER, W., et al. "Binding of Tritiated and Radioiodinated ZM241, 385 to Brain A2A Adenosine Receptors"<br>*Nuclear Medicine and Biology,* Vol. 31, No. 2, 29 February 2004 (2004-02-29), ISSN: 0969-8051,<br>page 173, column 1, paragraphs 1 and 2, page 174, figure 1, and page 175, column 2, paragraph 2 | 12-15, 28-31, 36-38, 41-43 |
| Y | CN 1056879 A (IMPERIAL CHEMICAL INDUSTRIES PLC) 11 December 1991 (1991-12-11)<br>description, page 22, lines 22-25, embodiments 71, 81, 161, 162, 164, 167 and 172, page 28, lines 18-26, and claim 1 | 1-43 |
| Y | US 5380714 A (ICI PLC) 10 January 1995 (1995-01-10)<br>description, column 6, pages 46-54 and column 10, lines 9-30, and claim 1 | 1-43 |

Form PCT/ISA/210 (second sheet) (January 2015)

**INTERNATIONAL SEARCH REPORT**

International application No.
**PCT/CN2020/139690**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-38**
because they relate to subject matter not required to be searched by this Authority, namely:

[1] Claims 19-38 set forth a method of treating diseases, which do not warrant an international search according to the criteria set out in PCT Rule 39.1(iv).

[2] A search on claims 19-38 is made on the basis of the following modification reasonably expected: the method of treating a disease in claims 19-38 is modified as a corresponding use in the preparation of a drug for the treatment of a disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/139690**

| Patent document cited in search report | | | Publication date (day/month/year) | Patent family member(s) | | | Publication date (day/month/year) |
|---|---|---|---|---|---|---|---|
| CN | 1056879 | A | 11 December 1991 | IL | 98316 | A | 26 May 1995 |
| | | | | EP | 0459702 | A1 | 04 December 1991 |
| | | | | GB | 2244487 | A | 04 December 1991 |
| | | | | PT | 97776 | A | 31 March 1992 |
| | | | | AU | 642494 | B2 | 21 October 1993 |
| | | | | US | 5270311 | A | 14 December 1993 |
| | | | | TW | 238306 | B | 11 January 1995 |
| | | | | JP | 2504879 | B2 | 05 June 1996 |
| | | | | FI | 912563 | A | 30 November 1991 |
| | | | | NO | 178401 | B | 11 December 1995 |
| | | | | ZA | 9104094 | A | 26 February 1992 |
| | | | | CS | 9101595 | A3 | 15 January 1992 |
| | | | | CA | 2043424 | A1 | 30 November 1991 |
| | | | | NO | 912051 | A | 02 December 1991 |
| | | | | JPH | 0597855 | A | 20 April 1993 |
| | | | | GB | 2244487 | B | 02 February 1994 |
| | | | | HUT | 61311 | A | 30 December 1992 |
| | | | | RO | 109542 | B1 | 30 March 1995 |
| | | | | AU | 7734891 | A | 05 December 1991 |
| | | | | NZ | 238288 | A | 27 July 1993 |
| US | 5380714 | A | 10 January 1995 | JPH | 05222045 | A | 31 August 1993 |
| | | | | EP | 0544443 | A1 | 02 June 1993 |
| | | | | CA | 2082462 | A1 | 26 May 1993 |
| CN | 111051309 | A | 21 April 2020 | HK | 40019786 | A0 | 16 October 2020 |
| | | | | WO | 2020002969 | A1 | 02 January 2020 |
| CN | 111164084 | A | 15 May 2020 | HK | 40019785 | A0 | 16 October 2020 |
| | | | | WO | 2020002968 | A1 | 02 January 2020 |
| | | | | CA | 3103068 | A1 | 02 January 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0231499 A


### Non-patent literature cited in the description


- **HOEHN MM ; YAHR MD.** Parkinsonism: onset, progression and mortality. *Neurology,* 1967, vol. 17, 427-42
- **PERKIN TRANS.** *J. Chem. Soc.,* 1995, vol. 1, 801-808
- *Structural Chemistry.,* 2013, vol. 24, 1241-1251
- **BECKER.** *J Neurol,* 2002, vol. 249 (3), III-40
- **PRUNIER C et al.** *Neuroimage,* July 2003, vol. 19 (3), 810-6
- **BECKER.** *J Neurol,* 2002, vol. 249 (3), 111-40
- **STERN.** *Annals of Neurol,* 2004, vol. 56, 169
- Unified Scoring Scale for Parkinson's Disease (UPDRS)